# EUROPEAN PATENT SPECIFICATION

(11) **EP 1 578 933 B1**
(45) Date of publication and mention of the grant of the patent: **10.03.2010**
(21) Application number: 03765708.7
(22) Date of filing: 18.07.2003
(51) Int. Cl.: C12Q 1/68, C07H 21/02, C07H 21/04, C12N 15/85, C12N 15/86

(54) **COMPOSITIONS AND METHODS FOR SI-RNA INHIBITION OF ANGIOGENESIS**
ZUSAMMENSETZUNGEN UND VERFAHREN ZUR siRNA-INHIBITION DER ANGIOGENESE
COMPOSITIONS ET PROCEDE D'INHIBITION DE L'ANGIOGENESE PAR ARN-SI

(30) Priority: 24.07.2002 US 398417 P; 14.11.2002 US 294228
(43) Date of publication of application: 28.09.2005
(60) Divisional application: 10151221.8
(73) Proprietor: The Trustees of The University of Pennsylvania, Philadelphia, Pennsylvania 19104-6283 (US)
(72) Inventor: TOLENTINO, Michael, J.., Celebration, Florida 34747 (US); REICH, Samuel, Jotham, Bala Cynwyd, PA 19004 (US)
(74) Representative: Main, Malcolm Charles
(86) International application number: PCT/US2003/022444
(87) International publication number: WO 2004/009769

(56) References cited:
- WO-A-95/04142
- WO-A-02/096927
- REICH S J ET AL: "Small interfering RNA (siRNA) targeting VEGF effectively inhibits ocular neovascularization in a mouse model" MOLECULAR VISION, SN, ATLANTA,, US, vol. 9, no. 31, 30 May 2003 (2003-05-30), pages 210-216, XP002343182 ISSN: 1090-0535
- SHU X ET AL: "Sphingosine kinase mediates vascular endothelial growth factor-induced activation of Ras and mitogen-activated protein kinases" MOLECULAR AND CELLULAR BIOLOGY, AMERICAN SOCIETY FOR MICROBIOLOGY, WASHINGTON, US, vol. 22, no. 22, November 2002 (2002-11), pages 7758-7768, XP002999707 ISSN: 0270-7306
- HOUCK ET AL: 'The vascular endothelial growth factor family: identification of a fourth molecular species and characterization of alternative splicing of RNA' MOLECULAR ENDOCRINOLOGY vol. 12, 1991, pages 1806 - 1814, XP000613073
- ELBASHIR ET AL: 'RNA interference is mediated by 21- and 22-nucleotide RNAs' GENES AND DEVELOPMENT vol. 15, 2001, pages 188 - 200, XP002204651
- SHI ET AL: 'Inhibition of renal cell carcinoma angiogenesis and growth by antisense oligonucleotides targeting vascular endothelial growth factor' BRITISH JOURNAL OF CANCER vol. 87, 2002, pages 119 - 126, XP002995137
- BRANTL S.: 'Antisense-RNA regulation and RNA interference' BIOCHIMICA BIOPHYSICA ACTA vol. 1575, 2002, pages 15 - 25, XP004356720

## Description

### Cross Reference to Related Application

This application claims the benefit of U.S. provisional patent application serial no. 60/398,417, filed on July 24, 2002, and U.S. nonprovisional patent application serial no. 10/294,228, filed November 14, 2002.

### Reference to Government Grant

The invention described herein was supported in part by NIH/NEI grant no. R01-EY10820, EY-13410 and EY12156. The U.S. government has certain rights in this invention.

### Field of the Invention

This invention relates to the regulation of gene expression by small interfering RNA, in particular for treating diseases or conditions involving angiogenesis.

### Background of the Invention

Angiogenesis, defined as the growth of new capillary blood vessels or "neovascularization," plays a fundamental role in growth and development. In mature humans, the ability to initiate angiogenesis is present in all tissues, but is held under strict control. A key regulator of angiogenesis is vascular endothelial growth factor ("VEGF"), also called vascular permeability factor ("VPF"). VEGF exists in at least four different alternative splice forms in humans VEGF₁₂₁, VEGF₁₆₅, VEGF₁₈₉ and VEGF₂₀₆), all of which exert similar biological activities.

Angiogenesis is initiated when secreted VEGF binds to the Flt-1 and Flk-1/KDR receptors (also called VEGF receptor 1 and VEGF receptor 2), which are expressed on the surface of endothelial cells. Flt-1 and Flk-1/KDR are transmembrane protein tyrosine kinases, and binding of VEGF initiates a cell signal cascade resulting in the ultimate neovascularization in the surrounding tissue.

Aberrant angiogenesis, or the pathogenic growth of new blood vessels, is implicated in a number of conditions. Among these conditions are diabetic retinopathy, psoriasis, exudative or "wet" age-related macular degeneration ("ARMD"), rheumatoid arthritis and other inflammatory diseases, and most cancers. The diseased tissues or tumors associated with these conditions express abnormally high levels of VEGF, and show a high degree of vascularization or vascular permeability.

ARMD in particular is a clinically important angiogenic disease. This condition is characterized by choroidal neovascularization in one or both eyes in aging individuals, and is the major cause of blindness in industrialized countries.

A number of therapeutic strategies exist for inhibiting aberrant angiogenesis, which attempt to reduce the production or effect of VEGF. For example, anti-VEGF or anti-VEGF receptor antibodies (Kim ES et al. (2002), PNAS USA 99: 11399-11404), and soluble VEGF "traps" which compete with endothelial cell receptors for VEGF binding (Holash J et al. (2002), PNAS USA 99: 11393-11398) have been developed. Classical VEGF "antisense" or aptamer therapies directed against VEGF gene expression have also been proposed (U.S. published application 2001/0021772 of Uhlmann et al.). However, the anti-angiogenic agents used in these therapies can produce only a stoichiometric reduction in VEGF or VEGF receptor, and the agents are typically overwhelmed by the abnormally high production of VEGF by the diseased tissue. The results achieved with available anti-angiogenic therapies have therefore been unsatisfactory.

RNA interference (hereinafter "RNAi") is a method of post-transcriptional gene regulation that is conserved throughout many eukaryotic organisms. RNAi is induced by short (*i.e*., <30 nucleotide) double stranded RNA ("dsRNA") molecules which are present in the cell (Fire A et al. (1998), Nature 391: 806-811). These short dsRNA molecules, called "short interfering RNA" or "siRNA," cause the destruction of messenger RNAs ("mRNAs") which share sequence homology with the siRNA to within one nucleotide resolution (Elbashir SM et al. (2001), Genes Dev, 15: 188-200). It is believed that the siRNA and the targeted mRNA bind to an "RNA-induced silencing complex" or "RISC", which cleaves the targeted mRNA. The siRNA is apparently recycled much like a multiple-turnover enzyme, with 1 siRNA molecule capable of inducing cleavage of approximately 1000 mRNA molecules. siRNA-mediated RNAi degradation of an mRNA is therefore more effective than currently available technologies for inhibiting expression of a target gene.

Elbashir SM et al. (2001), *supra,* has shown that synthetic siRNA of 21 and 22 nucleotides in length, and which have short 3' overhangs, are able to induce RNAi of target mRNA in a Drosophila cell lysate. Cultured mammalian cells also exhibit RNAi degradation with synthetic siRNA (Elbashir SM et al. (2001) Nature, 411: 494-498), and RNAi degradation induced by synthetic siRNA has recently been shown in living mice (McCaffrey AP et al. (2002), Nature, 418: 38-39; Xia H et al. (2002), Nat. Biotech. 20: 1006-1010). The therapeutic potential of siRNA-induced RNAi degradation has been demonstrated in several recent *in vitro* studies, including the siRNA-directed inhibition of HIV-1 infection (Novina CD et al. (2002), Nat. Med. 8: 681-686) and reduction of neurotoxic polyglutamine disease protein expression (Xia H et al. (2002), *supra*).

What is needed, therefore, are agents which selectively inhibit expression of VEGF or VEGF receptors in catalytic or sub-stoichiometric amounts.

### Summary of the Invention

The present invention is directed to siRNAs having the sequence shown in SEQ ID NO 97, 98, 50, 51, 52, 56 which specifically target and cause RNAi-induced degradation of mRNA from VEGF, Flt-1 and Flk-1/KDR genes. The siRNA compounds and compositions of the invention are used to inhibit angiogenesis, in particular for the treatment of cancerous tumors, age-related macular degeneration, and other angiogenic diseases.

Thus, the invention provides an isolated siRNA which targets human VEGF mRNA, human Flt-1 mRNA, human Flk-1/KDR mRNA, or an alternative splice form, mutant or cognate thereof. The siRNA comprises a sense RNA strand and an antisense RNA strand which form an RNA duplex. The sense RNA strand comprises a nucleotide sequence identical to a target sequence of about 19 to about 25 contiguous nucleotides in the target mRNA.

The invention also provides recombinant plasmids and viral vectors which express the siRNA of the invention, as well as pharmaceutical compositions comprising the siRNA of the invention and a pharmaceutically acceptable carrier.

The invention further provides a method of inhibiting expression of human VEGF mRNA, human Flt-1 mRNA, human Flk-1/KDR mRNA, or an alternative splice form, mutant or cognate thereof, comprising administering to a subject an effective amount of the siRNA of the invention such that the target mRNA is degraded.

The invention further provides a method of inhibiting angiogenesis in a subject, comprising administering to a subject an effective amount of an siRNA targeted to human VEGF mRNA, human Flt-1 mRNA, human Flk-1/KDR mRNA, or an alternative splice form, mutant or cognate thereof.

The invention further provides a method of treating an angiogenic disease, comprising administering to a subject in need of such treatment an effective amount of an siRNA targeted to human VEGF mRNA, human Flt-1 mRNA, human Flk-1/KDR mRNA, or an alternative splice form, mutant or cognate thereof, such that angiogenesis associated with the angiogenic disease is inhibited.

### Brief Description of the Drawings

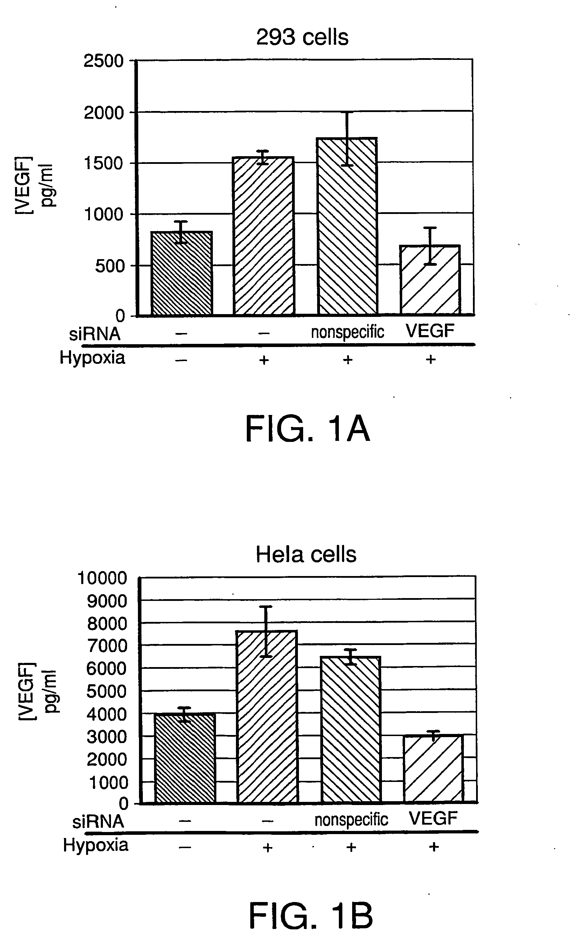

**FIGS. 1A** and **1B** are a histograms of VEGF concentration (in pg/ml) in hypoxic 293 and HeLa cells treated with no siRNA ("-"); nonspecific siRNA ("nonspecific"); or siRNA targeting human VEGF mRNA ("VEGF"). VEGF concentration (in pg/ml) in non-hypoxic 293 and HeLa cells is also shown. Each bar represents the average of four experiments, and the error is the standard deviation of the mean.

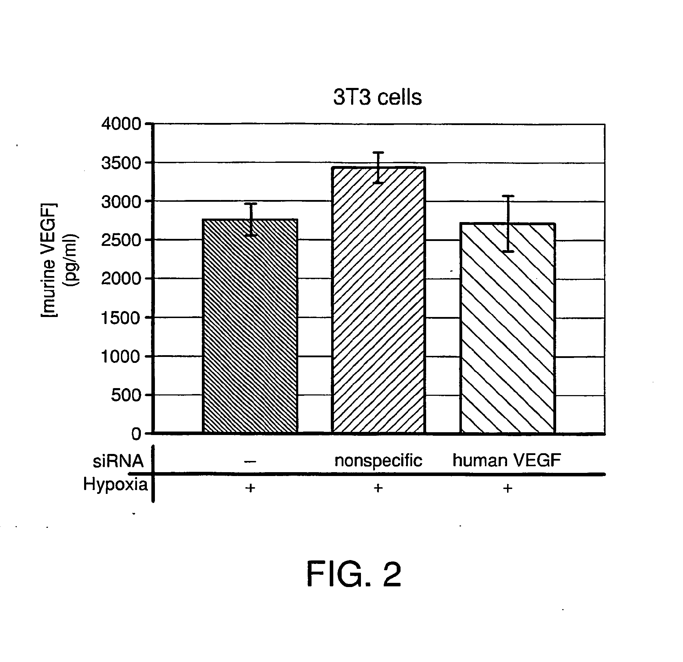

**FIG. 2** is a histogram of murine VEGF concentration (in pg/ml) in hypoxic NIH 3T3 cells treated with no siRNA ("-"); nonspecific siRNA ("nonspecific"); or siRNA targeting human VEGF mRNA ("VEGF"). Each bar represents the average of six experiments and the error is the standard deviation of the mean.

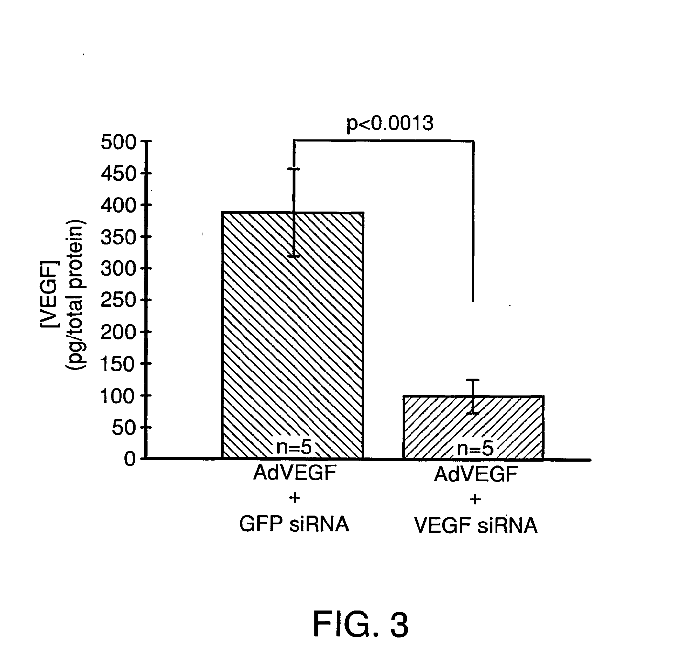

**FIG. 3** is a histogram of human VEGF concentration (pg/total protein) in retinas from mice injected with adenovirus expressing human VEGF ("AdVEGF") in the presence of either GFP siRNA (dark gray bar) or human VEGF siRNA (light grey bar). Each bar represent the average of 5 eyes and the error bars represent the standard error of the mean.

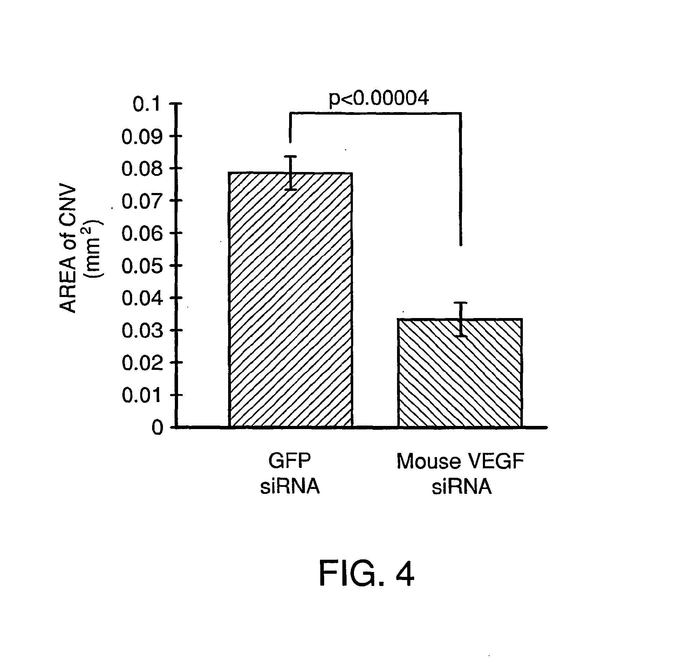

**FIG. 4** is a histogram showing the mean area (in mm²) of laser-induced CNV in control eyes given subretinal injections of GFP siRNA (N=9; "GFP siRNA"), and in eyes given subretinal injections of mouse VEGF siRNA (N=7; "Mouse VEGF siRNA"). The error bars represent the standard error of the mean.

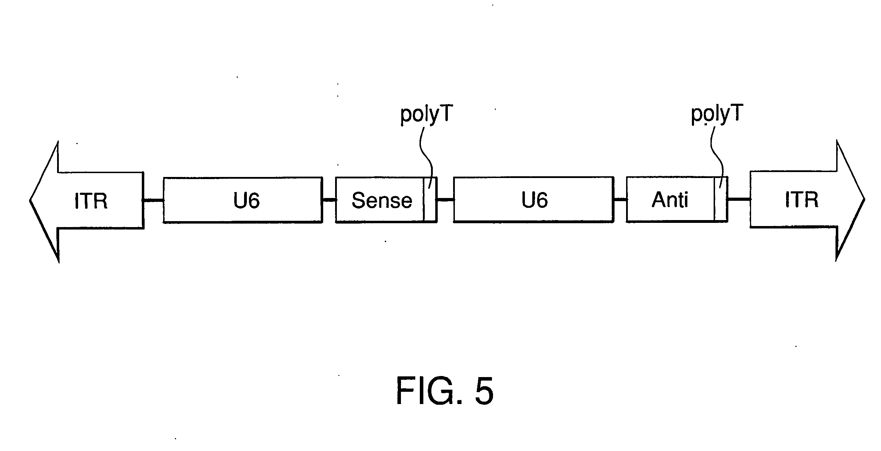

**FIG. 5** is a schematic representation of pAAVsiRNA, a cis-acting plasmid used to generate a recombinant AAV viral vector of the invention. "ITR": AAV inverted terminal repeats; "U6": U6 RNA promoters; "Sense": siRNA sense coding sequence; "Anti": siRNA antisense coding sequence; "PolyT": polythymidine termination signals.

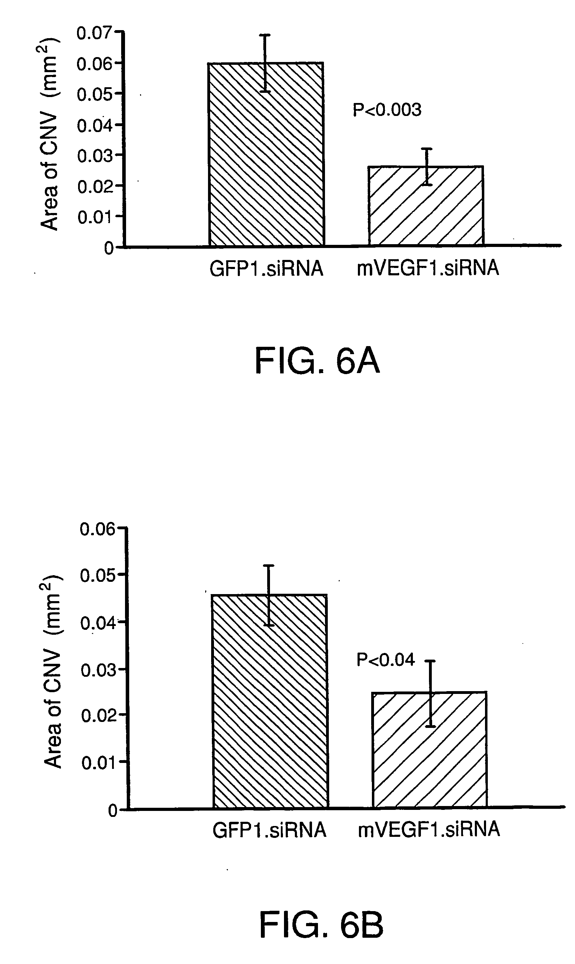

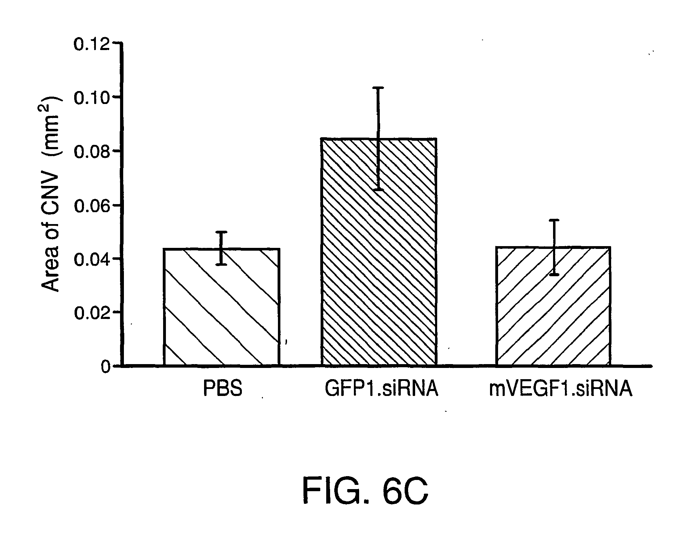

**Fig. 6** shows histograms of the mean area (in mm²) of laser-induced CNV in treatment in mouse eyes injected (A) subretinally or (B) intravitreally with a mouse anti-VEGF siRNA ("mVEGFI.siRNA") or control siRNA ("GFP1.siRNA"). The error bars represent the standard error of the mean. (**C**) is a histogram of the mean area (in mm²) of laser-induced CNV in mouse eyes injected intravitreally with: phosphate-buffered saline with no siRNA at 1 day post-laser induction ("PBS"; CNV area measured at 14 days post-laser induction); control siRNA at 14 days post-laser induction ("GFP1.siRNA"; CNV area measured at 21 days post-laser induction); or a mouse anti-VEGF siRNA at 14 days post-laser induction ("mVEGF1.siRNA"; CNV area measured at 21 days post-laser induction). The error bars represent the standard error of the mean.

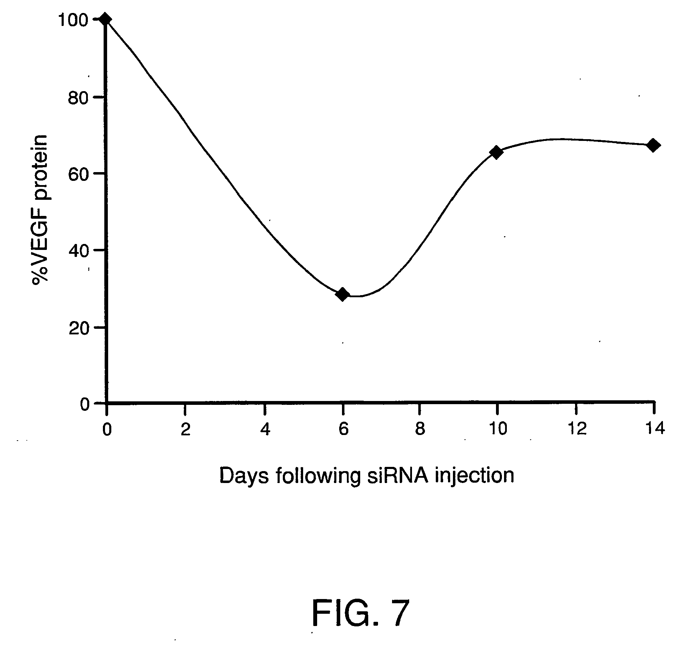

**Fig. 7** is a graph of the percent of VEGF ("%VEGF") protein in mouse eyes injected sub-retinally with human anti-VEGF siRNA ("Cand5") and control siRNA ("GFP1.siRNA") at 0 (n=2; pre-siRNA injection), 6 (n=3), 10 (n=3) and 14 (n=3) days post-injection. %VEGF = ([VEGF] in the Cand5 eye/[VEGF] in the GFP1.siRNA eye) *100.

### Detailed Description of the Invention

Unless otherwise indicated, all nucleic acid sequences herein are given in the 5' to 3' direction The invention specifically relates to SEQ ID NO. 97, 98, 50, 51 52, 56. All references to other SEQ ID Nos are illustrative and do not define the invention. Also, all deoxyribonucleotides in a nucleic acid sequence are represented by capital letters (*e.g*., deoxythymidine is "T"), and ribonucleotides in a nucleic acid sequence are represented by lower case letters (*e.g*., uridine is "u").

Compositions and methods comprising siRNA targeted to VEGF, Flt-1 or Flk-1/KDR mRNA are advantageously used to inhibit angiogenesis, in particular for the treatment of angiogenic disease. The siRNA of the invention are believed to cause the RNAi-mediated degradation of these mRNAs, so that the protein product of the VEGF, Flt-1 or Fllc-1/KDR genes is not produced or is produced in reduced amounts. Because VEGF binding to the Flt-1 or Flk-1/hDR receptors is required for initiating and maintaining angiogenesis, the siRNA-mediated degradation of VEGF, Flt-1 or Flk-1/KDR mRNA inhibits the angiogenic process.

The invention therefore provides isolated siRNA comprising short doublestranded RNA from about 17 nucleotides to about 29 nucleotides in length, preferably from about 19 to about 25 nucleotides in length, that are targeted to the target mRNA. The siRNA comprise a sense RNA strand and a complementary antisense RNA strand annealed together by standard Watson-Crick base-pairing interactions (hereinafter "base-paired"). As is described in more detail below, the sense strand comprises a nucleic acid sequence which is identical to a target sequence contained within the target mRNA.

The sense and antisense strands of the present siRNA can comprise two complementary, single-stranded RNA molecules or can comprise a single molecule in which two complementary portions are base-paired and are covalently linked by a single-stranded "hairpin" area. Without wishing to be bound by any theory, it is believed that the hairpin area of the latter type of siRNA molecule is cleaved intracellularly by the "Dicer" protein (or its equivalent) to form an siRNA of two individual base-paired RNA molecules (see Tuschl, T. (2002), *supra*).

As used herein, "isolated" means altered or removed from the natural state through human intervention. For example, an siRNA naturally present in a living animal is not "isolated," but a synthetic siRNA, or an siRNA partially or completely separated from the coexisting materials of its natural state is "isolated." An isolated siRNA can exist in substantially purified form, or can exist in a non-native environment such as, for example, a cell into which the siRNA has been delivered.

As used herein, "target mRNA" means human VEGF, Flt-1 or Flk-1/KDR mRNA, mutant or alternative splice forms of human VEGF, Flt-1 or Flk-1/KDR mRNA, or mRNA from cognate VEGF, Flt-1 or Flk-1/KDR genes.

As used herein, a gene or mRNA which is "cognate" to human VEGF, Flt-1 or Flk-1/KDR is a gene or mRNA from another mammalian species which is homologous to human VEGF, Flt-1 or Flk-1/KDR. For example, the cognate VEGF mRNA from the mouse is given in SEQ ID NO: 1.

Splice variants of human VEGF are known, including VEGF₁₂₁ (SEQ ID NO: 2), VEGF₁₆₅ (SEQ ID NO: 3), VEGF₁₈₉ (SEQ ID NO: 4) and VEGF₂₀₆ (SEQ ID NO: 5). The mRNA transcribed from the human VEGF, Flt-1 (SEQ ID NO: 6) or Flk-1/KDR (SEQ ID NO: 7) genes can be analyzed for further alternative splice forms using techniques well-known in the art. Such techniques include reverse transcription-polymerase chain reaction (RT-PCR), northern blotting and *in-situ* hybridization. Techniques for analyzing mRNA sequences are described, for example, in Busting SA *(2000), J. Mol. Endocrinol*. 25: 169-193, the entire disclosure of which is herein incorporated by reference. Representative techniques for identifying alternatively spliced mRNAs are also described below.

For example, databases that contain nucleotide sequences related to a given disease gene can be used to identify alternatively spliced mRNA. Such databases include GenBank, Embase, and the Cancer Genome Anatomy Project (CGAP) database. The CGAP database, for example, contains expressed sequence tags (ESTs) from various types of human cancers. An mRNA or gene sequence from the VEGF, Flt-1 or Flk-1/KDR genes can be used to query such a database to determine whether ESTs representing alternatively spliced mRNAs have been found for a these genes.

A technique called "RNAse protection" can also be used to identify alternatively spliced VEGF, Flt-1 or Flk-1/KDR mRNAs. RNAse protection involves translation of a gene sequence into synthetic RNA, which is hybridized to RNA derived from other cells; for example, cells from tissue at or near the site of neovascularization. The hybridized RNA is then incubated with enzymes that recognize RNA:RNA hybrid mismatches. Smaller than expected fragments indicate the presence of alternatively spliced mRNAs. The putative alternatively spliced mRNAs can be cloned and sequenced by methods well known to those skilled in the art.

RT-PCR can also be used to identify alternatively spliced VEGF, Flt-1 or Flk-1/KDR mRNAs. In RT-PCR, mRNA from the diseased tissue is converted into cDNA by the enzyme reverse transcriptase, using methods well-known to those of ordinary skill in the art. The entire coding sequence of the cDNA is then amplified via PCR using a forward primer located in the 3' untranslated region, and a reverse primer located in the 5' untranslated region. The amplified products can be analyzed for alternative splice forms, for example by comparing the size of the amplified products with the size of the expected product from normally spliced mRNA, *e.g*., by agarose gel electrophoresis. Any change in the size of the amplified product can indicate alternative splicing.

mRNA produced from mutant VEGF, Flt-1 or Flk-1/KDR genes can also be readily identified through the techniques described above for identifying alternative splice forms. As used herein, "mutant" VEGF, Flt-1 or Flk-1/KDR genes or mRNA include human VEGF, Flt-1 or Flk-1/KDR genes or mRNA which differ in sequence from the VEGF, Flt-1 or Flk-1/KDR sequences set forth herein. Thus, allelic forms of these genes, and the mRNA produced from them, are considered "mutants" for purposes of this invention.

It is understood that human VEGF, Flt-1 or Flk-1/KDR mRNA may contain target sequences in common with their respective alternative splice forms, cognates or mutants. A single siRNA comprising such a common targeting sequence can therefore induce RNAi-mediated degradation of different RNA types which contain the common targeting sequence.

The siRNA of the invention can comprise partially purified RNA, substantially pure RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA, including modifications that make the siRNA resistant to nuclease digestion.

One or both strands of the siRNA of the invention can also comprise a 3' overhang. As used herein, a "3' overhang" refers to at least one unpaired nucleotide extending from the 3'-end of a duplexed RNA strand.

Thus in one embodiment, the siRNA of the invention comprises at least one 3' overhang of from 1 to about 6 nucleotides (which includes ribonucleotides or deoxynucleotides) in length, preferably from 1 to about 5 nucleotides in length, more preferably from 1 to about 4 nucleotides in length, and particularly preferably from about 2 to about 4 nucleotides in length.

In the embodiment in which both strands of the siRNA molecule comprise a 3' overhang, the length of the overhangs can be the same or different for each strand. In a most preferred embodiment, the 3' overhang is present on both strands of the siRNA, and is 2 nucleotides in length. For example, each strand of the siRNA of the invention can comprise 3' overhangs of dithymidylic acid ("TT") or diuridylic acid ("uu").

In order to enhance the stability of the present siRNA, the 3' overhangs can be also stabilized against degradation. In one embodiment, the overhangs are stabilized by including purine nucleotides, such as adenosine or guanosine nucleotides. Alternatively, substitution of pyrimidine nucleotides by modified analogues, *e.g*., substitution of uridine nucleotides in the 3' overhangs with 2'-deoxythymidine, is tolerated and does not affect the efficiency of RNAi degradation. In particular, the absence of a 2' hydroxyl in the 2'-deoxythymidine significantly enhances the nuclease resistance of the 3'overhang in tissue culture medium.

In certain embodiments, the siRNA of the invention comprises the sequence AA(N19)TT or NA(N21), where N is any nucleotide. These siRNA comprise approximately 30-70% GC, and preferably comprise approximately 50% G/C. The sequence of the sense siRNA strand corresponds to (N19)TT or N21 (*i.e*., positions 3 to 23), respectively. In the latter case, the 3' end of the sense siRNA is converted to TT. The rationale for this sequence conversion is to generate a symmetric duplex with respect to the sequence composition of the sense and antisense strand 3' overhangs. The antisense RNA strand is then synthesized as the complement to positions 1 to 21 of the sense strand.

Because position 1 of the 23-nt sense strand in these embodiments is not recognized in a sequence-specific manner by the antisense strand, the 3'-most nucleotide residue of the antisense strand can be chosen deliberately. However, the penultimate nucleotide of the antisense strand (complementary to position 2 of the 23-nt sense strand in either embodiment) is generally complementary to the targeted sequence.

In another embodiment, the siRNA of the invention comprises the sequence NAR(N17)YNN, where R is a purine (*e.g*., A or G) and Y is a pyrimidine (*e.g*., C or U/T). The respective 21-nt sense and antisense RNA strands of this embodiment therefore generally begin with a purine nucleotide. Such siRNA can be expressed from pol III expression vectors without a change in targeting site, as expression of RNAs from pol III promoters is only believed to be efficient when the first transcribed nucleotide is a purine.

The siRNA of the invention can be targeted to any stretch of approximately 19-25 contiguous nucleotides in any of the target mRNA sequences (the "target sequence"). Techniques for selecting target sequences for siRNA are given, for example, in Tuschl T et al., "The siRNA User Guide," revised Oct. 11, 2002, the entire disclosure of which is herein incorporated by reference. "The siRNA User Guide" is available on the world wide web at a website maintained by Dr. Thomas Tuschl, Department of Cellular Biochemistry, AG 105, Max-Planck-Institute for Biophysical Chemistry, 37077 Göttingen, Germany, and can be found by accessing the website of the Max Planck Institute and searching with the keyword "siRNA." Thus, the sense strand of the present siRNA comprises a nucleotide sequence identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (*i.e*., in the 3' direction) from the start codon. The target sequence can, however, be located in the 5' or 3' untranslated regions, or in the region nearby the start codon (see, *e.g*., the target sequences of SEQ ID NOS: 73 and 74 in Table 1 below, which are within 100 nt of the 5'-end of the VEGF₁₂₁ cDNA

For example, a suitable target sequence in the VEGF₁₂₁ cDNA sequence is:
TCATCACGAAGTGGTGAAG (SEQ ID NO: 8)

Thus, an siRNA of the invention targeting this sequence, and which has 3' uu overhangs on each strand (overhangs shown in bold), is:
5'-ucaucacgaaguggugaaguu-3' (SEQ ID NO: 9)
3'-uuaguagugcuucaccacuuc-5' (SEQ ID NO: 10)

An siRNA of the invention targeting this same sequence, but having 3' TT overhangs on each strand (overhangs shown in bold) is:
5'-ucaucacgaaguggugaagTT-3'(SEQ ID NO: 11)
3'-TTaguagugcuucaccacuuc-5' (SEQ ID NO: 12)

Other VEGF₁₂₁ target sequences from which siRNA of the invention can be derived are given in Table 1. It is understood that all VEGF₁₂₁ target sequences listed in Table 1 are within that portion of the VEGF₁₂₁ alternative splice form which is common to all human VEGF alternative splice forms. Thus, the VEGF₁₂₁ target sequences in Table 1 can also target VEGF₁₆₅, VEGF₁₈₉ and VEGF₂₀₆ mRNA. Target sequences which target a specific VEGF isoform can also be readily identified. For example, a target sequence which targets VEGF₁₆₅ mRNA but not VEGF₁₂₁ mRNA is AACGTACTTGCAGATGTGACA (SEQ ID NO: 13). Exemplary target sequences for human Flt-1 are given in SEQ ID NOS: 91 - 504, and exemplary target sequences for human Flk-1/KDR are given in SEQ ID NOS: 505 - 864.

**Table 1 - VEGF Target Sequences**

| **target sequence** | **SEQ ID NO:** | | **target sequence** | **SEQ ID NO:** |
|---|---|---|---|---|
| GTTCATGGATGTCTATCAG | 14 | | TCCCTGTGGGCCTTGCTCA | 30 |
| TCGAGACCCTGGTGGACAT | 15 | | GCATTTGTTTGTACAAGAT | 31 |
| TGACGAGGGCCTGGAGTGT | 16 | | GATCCGCAGACGTGTAAAT | 32 |
| TGACGAGGGCCTGGAGTGT | 17 | | ATGTTCCTGCA.AAAACACA | 33 |
| CATCACCATGCAGATTATG | 18 | | TGTTCCTGCAAAAACACAG | 34 |
| ACCTCACCAAGGCCAGCAC | 19 | | AAACACAGACTCGCGTTGC | 35 |
| GGCCAGCACATAGGAGAGA | 20 | | AACACAGACTCGCGTTGCA | 36 |
| CAAATGTGAATGCAGACCA | 21 | | ACACAGACTCGCGTTGCAA | 37 |
| ATGTGAATGCAGACCAAAG | 22 | | CACAGACTCGCGTTGCAAG | 38 |
| TGCAGACCAAAGAAAGATA | 23 | | GGCGAGGCAGCTTGAGTTA | 39 |
| AGAAAGATAGAGCAAGACA | 24 | | ACGAACGTACTTGCAGATG | 40 |
| GAAAGATAGAGCAAGACAA | 25 | | CGAACGTACTTGCAGATGT | 41 |
| GATAGAGCAAGACAAGAAA | 26 | | CGTACTTGCAGATGTGACA | 42 |
| GACAAGAAAATCCCTGTGG | 27 | | GTGGTCCCAGGCTGCACCC | 43 |
| GAAAATCCCTGTGGGCCTT | 28 | | GGAGGAGGGCAGAATCATC | 44 |
| AATCCCTGTGGGCCTTGCT | 29 | | GTGGTGAAGTTCATGGATG | 45 |
| AATCATCACGAAGTGGTGAAG | 46 | | AAGCATTTGTTTGTACAAGATCC | 62 |
| AAGTTCATGGATGTCTATCAG | 47 | | AAGATCCGCAGACGTGTAAATGT | 63 |
| AATCGAGACCCTGGTGGACAT | 48 | | AAATGTTCCTGCAAAAACACAGA | 64 |
| AATGACGAGGGCCTGGAGTGT | 49 | | AATGTTCCTGCAAAAACACAGAC | 65 |
| AACATCACCATGCAGATTATG | 50 | | AAAAACACAGACTCGCGTTGCAA | 66 |
| AAACCTCACCAAGGCCAGCAC | 51 | | AAAACACAGACTCGCGTTGCAAG | 67 |
| AAGGCCAGCACATAGGAGAGA | 52 | | AAACACAGACTCGCGTTGCAAGG | 68 |
| AACAAATGTGAATGCAGACCA | 53 | | AACACAGACTCGCGTTGCAAGGC | 69 |
| AAATGTGAATGCAGACCAAAG | 54 | | AAGGCGAGGCAGCTTGAGTTAAA | 70 |
| AATGCAGACCAAAGAAAGATA | 55 | | AAACGAACGTACTTGCAGATGTG | 71 |
| AAAGAAAGATAGAGCAAGACA | 56 | | AACGAACGTACTTGCAGATGTGA | 72 |
| AAGAAAGATAGAGCAAGACAA | 57 | | AAGTGGTCCCAGGCTGCACCCAT | 73 |
| AAGATAGAGCAAGACAAGAAAAT | 58 | | AAGGAGGAGGGCAGAATCATCAC | 74 |
| AAGACAAGAAAATCCCTGTGGGC | 59 | | AAGTGGTGAAGTTCATGGATGTC | 75 |
| AAGAAAATCCCTGTGGGCCTTGC | 60 | | AAAATCCCTGTGGGCCTTGCTCA | 76 |
| AATCCCTGTGGGCCTTGCTCAGA | 61 | | GGCAGAATCATCACGAAGTGG | 81 |
| CCTGGTGGACATCTTCCAGGA | 82 | | CACACACTCGCGTTGCAAGGC | 87 |
| GAGATCGAGTACATCTTCAAG | 83 | | TCACCATGCAGATTATGCGGA | 88 |
| TGGAGTGTGTGCCCACTGAGG | 84 | | TAGAGCAAGACAAGAA.AATCC | 89 |
| GAGCTTCCTACAGCACAACAA | 85 | | CCGCAGACGTGTAAATGTTCC | 90 |
| TTGCTCAGAGCGGAGAAAGCA | 86 | | | |

The siRNA of the invention can be obtained using a number of techniques known to those of skill in the art. For example, the siRNA can be chemically synthesized or recombinantly produced using methods known in the art, such as the Drosophila *in vitro* system described in U.S. published application 2002/0086356 of Tuschl et al., the entire disclosure of which is herein incorporated by reference.

Preferably, the siRNA of the invention are chemically synthesized using appropriately protected ribonucleoside phosphoramidites and a conventional DNA/RNA synthesizer. The siRNA can be synthesized as two separate, complementary RNA molecules, or as a single RNA molecule with two complementary regions. Commercial suppliers of synthetic RNA molecules or synthesis reagents include Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA) and Cruachem (Glasgow, UK).

Alternatively, siRNA can also be expressed from recombinant circular or linear DNA plasmids using any suitable promoter. Suitable promoters for expressing siRNA of the invention from a plasmid include, for example, the U6 or H1 RNA pol III promoter sequences and the cytomegalovirus promoter. Selection of other suitable promoters is within the skill in the art. The recombinant plasmids of the invention can also comprise inducible or regulatable promoters for expression of the siRNA in a particular tissue or in a particular intracellular environment.

The siRNA expressed from recombinant plasmids can either be isolated from cultured cell expression systems by standard techniques, or can be expressed intracellularly at or near the area of neovascularization *in vivo.* The use of recombinant plasmids to deliver siRNA of the invention to cells *in vivo* is discussed in more detail below.

siRNA of the invention can be expressed from a recombinant plasmid either as two separate, complementary RNA molecules, or as a single RNA molecule with two complementary regions.

Selection of plasmids suitable for expressing siRNA of the invention, methods for inserting nucleic acid sequences for expressing the siRNA into the plasmid, and methods of delivering the recombinant plasmid to the cells of interest are within the skill in the art. See, for example Tuschl, T. (2002), Nat. Biotechnol, 20: 446-448; Brummelkamp TR et al. (2002), Science 296: 550-553; Miyagishi M et al. (2002), Nat. Biotechnol. 20: 497-500; Paddison PJ et al. (2002), Genes Dev. 16: 948-958; Lee NS et al. (2002), Nat. Biotechnol. 20: 500-505; and Paul CP et al. (2002), Nat. Biotechnol. 20: 505-508, the entire disclosures of which are herein incorporated by reference.

A plasmid comprising nucleic acid sequences for expressing an siRNA of the invention is described in Example 7 below. That plasmid, called pAAVsiRNA, comprises a sense RNA strand coding sequence in operable connection with a polyT termination sequence under the control of a human U6 RNA promoter, and an antisense RNA strand coding sequence in operable connection with a polyT termination sequence under the control of a human U6 RNA promoter. The plasmid pAAVsiRNA is ultimately intended for use in producing an recombinant adeno-associated viral vector comprising the same nucleic acid sequences for expressing an siRNA of the invention.

As used herein, "in operable connection with a polyT termination sequence" means that the nucleic acid sequences encoding the sense or antisense strands are immediately adjacent to the polyT termination signal in the 5' direction. During transcription of the sense or antisense sequences from the plasmid, the polyT termination signals act to terminate transcription.

As used herein, "under the control" of a promoter means that the nucleic acid sequences encoding the sense or antisense strands are located 3' of the promoter, so that the promoter can initiate transcription of the sense or antisense coding sequences.

The siRNA of the invention can also be expressed from recombinant viral vectors intracellularly at or near the area of neovascularization *in vivo.* The recombinant viral vectors of the invention comprise sequences encoding the siRNA of the invention and any suitable promoter for expressing the siRNA sequences. Suitable promoters include, for example, the U6 or H1 RNA pol III promoter sequences and the cytomegalovirus promoter. Selection of other suitable promoters is within the skill in the art. The recombinant viral vectors of the invention can also comprise inducible or regulatable promoters for expression of the siRNA in a particular tissue or in a particular intracellular environment. The use of recombinant viral vectors to deliver siRNA of the invention to cells *in vivo* is discussed in more detail below.

siRNA of the invention can be expressed from a recombinant viral vector either as two separate, complementary RNA molecules, or as a single RNA molecule with two complementary regions.

Any viral vector capable of accepting the coding sequences for the siRNA molecule(s) to be expressed can be used, for example vectors derived from adenovirus (AV); adeno-associated virus (AAV); retroviruses (*e.g*, lentiviruses (LV), Rhabdoviruses, murine leukemia virus); herpes virus, and the like. The tropism of the viral vectors can also be modified by pseudotyping the vectors with envelope proteins or other surface antigens from other viruses. For example, an AAV vector of the invention can be pseudotyped with surface proteins from vesicular stomatitis virus (VSV), rabies, Ebola, Mokola, and the like.

Selection of recombinant viral vectors suitable for use in the invention, methods for inserting nucleic acid sequences for expressing the siRNA into the vector, and methods of delivering the viral vector to the cells of interest are within the skill in the art. See, for example, Domburg R (1995), Gene Therap. 2: 301-310; Eglitis MA (1988), Biotechniques 6: 608-614; Miller AD (1990), Hum Gene Therap. 1: 5-14; and Anderson WF (1998), Nature 392: 25-30, the entire disclosures of which are herein incorporated by reference.

Preferred viral vectors are those derived from AV and AAV. In a particularly preferred embodiment, the siRNA of the invention is expressed as two separate, complementary single-stranded RNA molecules from a recombinant AAV vector comprising, for example, either the U6 or H1 RNA promoters, or the cytomegalovirus (CMV) promoter.

A suitable AV vector for expressing the siRNA of the invention, a method for constructing the recombinant AV vector, and a method for delivering the vector into target cells, are described in Xia H et al. (2002), Nat. Biotech. 20: 1006-1010.

Suitable AAV vectors for expressing the siRNA of the invention, methods for constructing the recombinant AAV vector, and methods for delivering the vectors into target cells are described in Samulski R et al. (1987), J. Virol. 61: 3096-3101; Fisher KJ et al. (1996), J. Virol., 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641, the entire disclosures of which are herein incorporated by reference. An exemplary method for generating a recombinant AAV vector of the invention is described in Example 7 below.

The ability of an siRNA containing a given target sequence to cause RNAi-mediated degradation of the target mRNA can be evaluated using standard techniques for measuring the levels of RNA or protein in cells. For example, siRNA of the invention can be delivered to cultured cells, and the levels of target mRNA can be measured by Northern blot or dot blotting techniques, or by quantitative RT-PCR. Alternatively, the levels of VEGF, Flt-1 or Flk-1/KDR receptor protein in the cultured cells can be measured by ELISA or Western blot. A suitable cell culture system for measuring the effect of the present siRNA on target mRNA or protein levels is described in Example 1 bellow.

RNAi-mediated degradation of target mRNA by an siRNA containing a given target sequence can also be evaluated with animal models of neovascularization, such as the ROP or CNV mouse models. For example, areas of neovascularization in an ROP or CNV mouse can be measured before and after administration of an siRNA. A reduction in the areas of neovascularization in these models upon administration of the siRNA indicates the down-regulation of the target mRNA (see Example 6 below).

As discussed above, the siRNA of the invention target and cause the RNAi-mediated degradation of VEGF, Flt-1 or Flk-1/KDR mRNA, or alternative splice forms, mutants or cognates thereof. Degradation of the target mRNA by the present siRNA reduces the production of a functional gene product from the VEGF, Flt-1 or Flk-1/KDR genes. Thus, the invention provides a method of inhibiting expression of VEGF, Flt-1 or Flk-1/KDR in a subject, comprising administering an effective amount of an siRNA of the invention to the subject, such that the target mRNA is degraded. As the products of the VEGF, Flt-1 and Flk-1/KDR genes are required for initiating and maintaining angiogenesis, the invention also provides a method of inhibiting angiogenesis in a subject by the RNAi-mediated degradation of the target mRNA by the present siRNA.

As used herein, a "subject" includes a human being or non-human animal. Preferably, the subject is a human being.

As used herein, an "effective amount" of the siRNA is an amount sufficient to cause RNAi-mediated degradation of the target mRNA, or an amount sufficient to inhibit the progression of angiogenesis in a subject.

RNAi-mediated degradation of the target mRNA can be detected by measuring levels of the target mRNA or protein in the cells of a subject, using standard techniques for isolating and quantifying mRNA or protein as described above.

Inhibition of angiogenesis can be evaluated by directly measuring the progress of pathogenic or nonpathogenic angiogenesis in a subject; for example, by observing the size of a neovascularized area before and after treatment with the siRNA of the invention. An inhibition of angiogenesis is indicated if the size of the neovascularized area stays the same or is reduced. Techniques for observing and measuring the size of neovascularized areas in a subject are within the skill in the art; for example, areas of choroid neovascularization can be observed by ophthalmoscopy.

Inhibition of angiogenesis can also be inferred through observing a change or reversal in a pathogenic condition associated with the angiogenesis. For example, in ARMD, a slowing, halting or reversal of vision loss indicates an inhibition of angiogenesis in the choroid. For tumors, a slowing, halting or reversal of tumor growth, or a slowing or halting of tumor metastasis, indicates an inhibition of angiogenesis at or near the tumor site. Inhibition of non-pathogenic angiogenesis can also be inferred from, for example, fat loss or a reduction in cholesterol levels upon administration of the siRNA of the invention.

It is understood that the siRNA of the invention can degrade the target mRNA (and thus inhibit angiogenesis) in substoichiometric amounts. Without wishing to be bound by any theory, it is believed that the siRNA of the invention causes degradation of the target mRNA in a catalytic manner. Thus, compared to standard anti-angiogenic therapies, significantly less siRNA needs to be delivered at or near the site of neovascularization to have a therapeutic effect.

One skilled in the art can readily determine an effective amount of the siRNA of the invention to be administered to a given subject, by taking into account factors such as the size and weight of the subject; the extent of the neovascularization or disease penetration; the age, health and sex of the subject; the route of administration; and whether the administration is regional or systemic. Generally, an effective amount of the siRNA of the invention comprises an intercellular concentration at or near the neovascularization site of from about 1 nanomolar (nM) to about 100 nM, preferably from about 2 nM to about 50 nM, more preferably from about 2.5 nM to about 10 nM. It is contemplated that greater or lesser amounts of siRNA can be administered.

The present methods can be used to inhibit angiogenesis which is non-pathogenic; *i.e*., angiogenesis which results from normal processes in the subject. Examples of non-pathogenic angiogenesis include endometrial neovascularization, and processes involved in the production of fatty tissues or cholesterol. Thus, the invention provides a method for inhibiting non-pathogenic angiogenesis, *e.g*., for controlling weight or promoting fat loss, for reducing cholesterol levels, or as an abortifacient.

The present methods can also inhibit angiogenesis which is associated with an angiogenic disease; *i.e*., a disease in which pathogenicity is associated with inappropriate or uncontrolled angiogenesis. For example, most cancerous solid tumors generate an adequate blood supply for themselves by inducing angiogenesis in and around the tumor site. This tumor-induced angiogenesis is often required for tumor growth, and also allows metastatic cells to enter the bloodstream.

Other angiogenic diseases include diabetic retinopathy, age-related macular degeneration (ARMD), psoriasis, rheumatoid arthritis and other inflammatory diseases. These diseases are characterized by the destruction of normal tissue by newly formed blood vessels in the area of neovascularization. For example, in ARMD, the choroid is invaded and destroyed by capillaries. The angiogenesis-driven destruction of the choroid in ARMD eventually leads to partial or full blindness.

Preferably, an siRNA of the invention is used to inhibit the growth or metastasis of solid tumors associated with cancers; for example breast cancer, lung cancer, head and neck cancer, brain cancer, abdominal cancer, colon cancer, colorectal cancer, esophagus cancer, gastrointestinal cancer, glioma, liver cancer, tongue cancer, neuroblastoma, osteosarcoma, ovarian cancer, pancreatic cancer, prostate cancer, retinoblastoma, Wilm's tumor, multiple myeloma; skin cancer (*e.g*., melanoma), lymphomas and blood cancer.

More preferably, an siRNA of the invention is used to inhibit choroidal neovascularization in age-related macular degeneration.

For treating angiogenic diseases, the siRNA of the invention can administered to a subject in combination with a pharmaceutical agent which is different from the present siRNA. Alternatively, the siRNA of the invention can be administered to a subject in combination with another therapeutic method designed to treat the angiogenic disease. For example, the siRNA of the invention can be administered in combination with therapeutic methods currently employed for treating cancer or preventing tumor metastasis (*e.g*., radiation therapy, chemotherapy, and surgery). For treating tumors, the siRNA of the invention is preferably administered to a subject in combination with radiation therapy, or in combination with chemotherapeutic agents such as cisplatin, carboplatin, cyclophosphamide, 5-fluorouracil, adriamycin, daunorubicin or tamoxifen.

In the present methods, the present siRNA can be administered to the subject either as naked siRNA, in conjunction with a delivery reagent, or as a recombinant plasmid or viral vector which expresses the siRNA.

Suitable delivery reagents for administration in conjunction with the present siRNA include the Mirus Transit TKO lipophilic reagent; lipofectin; lipofectamine; cellfectin; or polycations (*e.g*., polylysine), or liposomes. A preferred delivery reagent is a liposome.

Liposomes can aid in the delivery of the siRNA to a particular tissue, such as retinal or tumor tissue, and can also increase the blood half-life of the siRNA. Liposomes suitable for use in the invention are formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example as described in Szoka et al. (1980), Ann. Rev. Biophys. Bioeng. 9: 467; and U.S. Pat. Nos. 4,235,871, 4,501,728, 4,837,028, and 5,019,369, the entire disclosures of which are herein incorporated by reference.

Preferably, the liposomes encapsulating the present siRNA comprises a ligand molecule that can target the liposome to a particular cell or tissue at or near the site of angiogenesis. Ligands which bind to receptors prevalent in tumor or vascular endothelial cells, such as monoclonal antibodies that bind to tumor antigens or endothelial cell surface antigens, are preferred.

Particularly preferably, the liposomes encapsulating the present siRNA are modified so as to avoid clearance by the mononuclear macrophage and reticuloendothelial systems, for example by having opsonization-inhibition moieties bound to the surface of the structure. In one embodiment, a liposome of the invention can comprise both opsonization-inhibition moieties and a ligand.

Opsonization-inhibiting moieties for use in preparing the liposomes of the invention are typically large hydrophilic polymers that are bound to the liposome membrane. As used herein, an opsonization inhibiting moiety is "bound" to a liposome membrane when it is chemically or physically attached to the membrane, *e.g*., by the intercalation of a lipid-soluble anchor into the membrane itself, or by binding directly to active groups of membrane lipids. These opsonization-inhibiting hydrophilic polymers form a protective surface layer which significantly decreases the uptake of the liposomes by the macrophage-monocyte system ("MMS") and reticuloendothelial system ("RES"); *e.g*., as described in U.S. Pat. No. 4,920,016, the entire disclosure of which is herein incorporated by reference. Liposomes modified with opsonization-inhibition moieties thus remain in the circulation much longer than unmodified liposomes. For this reason, such liposomes are sometimes called "stealth" liposomes.

Stealth liposomes are known to accumulate in tissues fed by porous or "leaky" microvasculature. Thus, target tissue characterized by such microvasculature defects, for example solid tumors, will efficiently accumulate these liposomes; see Gabizon, et al. (1988), P.N.A.S, USA, 18: 6949-53. In addition, the reduced uptake by the RES lowers the toxicity of stealth liposomes by preventing significant accumulation in the liver and spleen. Thus, liposomes of the invention that are modified with opsonization-inhibition moieties can deliver the present siRNA to tumor cells.

Opsonization inhibiting moieties suitable for modifying liposomes are preferably water-soluble polymers with a number-average molecular weight from about 500 to about 40,000 daltons, and more preferably from about 2,000 to about 20,000 daltons. Such polymers include polyethylene glycol (PEG) or polypropylene glycol (PPG) derivatives; *e.g*., methoxy PEG or PPG, and PEG or PPG stearate; synthetic polymers such as polyacrylamide or poly N-vinyl pyrrolidone; linear, branched, or dendrimeric polyamidoamines; polyacrylic acids; polyalcohols, e.g., polyvinylalcohol and polyxylitol to which carboxylic or amino groups are chemically linked, as well as gangliosides, such as ganglioside GM₁. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, e.g., galacturonic acid, glucuronic acid, mannuronic acid, hyaluronic acid, pectic acid, neuraminic acid, alginic acid, carrageenan; aminated polysaccharides or oligosaccharides (linear or branched); or carboxylated polysaccharides or oligosaccharides, e.g., reacted with derivatives of carbonic acids with resultant linking of carboxylic groups.

Preferably, the opsonization-inhibiting moiety is a PEG, PPG, or derivatives thereof. Liposomes modified with PEG or PEG-derivatives are sometimes called "PEGylated liposomes."

The opsonization inhibiting moiety can be bound to the liposome membrane by any one of numerous well-known techniques. For example, an N-hydroxysuccinimide ester of PEG can be bound to a phosphatidyl-ethanolamine lipid-soluble anchor, and then bound to a membrane. Similarly, a dextran polymer can be derivatized with a stearylamine lipid-soluble anchor via reductive amination using Na(CN)BH₃ and a solvent mixture such as tetrahydrofuran and water in a 30:12 ratio at 60 °C.

Recombinant plasmids which express siRNA of the invention are discussed above. Such recombinant plasmids can also be administered directly or in conjunction with a suitable delivery reagent, including the Mirus Transit LT1 lipophilic reagent; lipofectin; lipofectamine; cellfectin; polycations (*e.g*., polylysine) or liposomes. Recombinant viral vectors which express siRNA of the invention are also discussed above, and methods for delivering such vectors to an area of neovascularization in a patient are within the skill in the art.

The siRNA of the invention can be administered to the subject by any means suitable for delivering the siRNA to the cells of the tissue at or near the area of neovascularization. For example, the siRNA can be administered by gene gun, electroporation, or by other suitable parenteral or enteral administration routes.

Suitable enteral administration routes include oral, rectal, or intranasal delivery.

Suitable parenteral administration routes include intravascular administration (e.g. intravenous bolus injection, intravenous infusion, intra-arterial bolus injection, intra-arterial infusion and catheter instillation into the vasculature); peri- and intra-tissue administration (*e.g*., peri-tumoral and intra-tumoral injection, intra-retinal injection or subretinal injection); subcutaneous injection or deposition including subcutaneous infusion (such as by osmotic pumps); direct (*e.g*., topical) application to the area at or near the site of neovascularization, for example by a catheter or other placement device (*e.g*., a corneal pellet or a suppository, eye-dropper, or an implant comprising a porous, non-porous, or gelatinous material); and inhalation. Suitable placement devices include the ocular implants described in U.S. Pat. Nos. 5,902,598 and 6,375,972, and the biodegradable ocular implants described in U.S. Pat. No 6,331,313, the entire disclosures of which are herein incorporated by reference. Such ocular implants are available from Control Delivery Systems, Inc. (Watertown, MA) and Oculex Pharmaceuticals, Inc. (Sunnyvale, CA).

In a preferred embodiment, injections or infusions of the siRNA are given at or near the site of neovascularization. More preferably, the siRNA is administered topically to the eye, *e.g*. in liquid or gel form to the lower eye lid or conjunctival cul-de-sac, as is within the skill in the art (see, *e.g*., Acheampong AA et al, 2002, Drug Metabol. and Disposition 30: 421-429, the entire disclosure of which is herein incorporated by reference).

Typically, the siRNA of the invention is administered topically to the eye in amounts of from about 5 microliters to about 75 microliters, for example from about 7 microliters to about 50 microliters, preferably from about 10 microliters to about 30 microliters. It is understood that topical instillation in the eye of siRNA in volumes greater than 75 microliters can result in loss of siRNA from the eye through spillage and drainage. Thus, it is preferable to administer a high concentration of siRNA (*e.g*., 100-1000 nM) in as small a volume as possible.

A particularly preferred parenteral administration route is intraocular administration. It is understood that intraocular administration of the present siRNA can be accomplished by injection or direct (*e.g*., topical) administration to the eye, as long as the administration route allows the siRNA to enter the eye. In addition to the topical routes of administration to the eye described above, suitable intraocular routes of administration include intravitreal, intraretinal, subretinal, subtenon, peri- and retro-orbital, trans-corneal and trans-scleral administration. Such intraocular administration routes are within the skill in the art; see, *e.g*., and Acheampong AA et al, 2002, *supra;* and Bennett et al. (1996), Hum. Gene Ther. 7: 1763-1769 and Ambati J et al., 2002, Progress in Retinal and Eye Res. 21: 145-151, the entire disclosures of which are herein incorporated by reference.

The siRNA of the invention can be administered in a single dose or in multiple doses. Where the administration of the siRNA of the invention is by infusion, the infusion can be a single sustained dose or can be delivered by multiple infusions. Injection of the agent directly into the tissue is at or near the site of neovascularization preferred. Multiple injections of the agent into the tissue at or near the site of neovascularization are particularly preferred.

One skilled in the art can also readily determine an appropriate dosage regimen for administering the siRNA of the invention to a given subject. For example, the siRNA can be administered to the subject once, such as by a single injection or deposition at or near the neovascularization site. Alternatively, the siRNA can be administered to a subject multiple times daily or weekly. For example, the siRNA can be administered to a subject once weekly for a period of from about three to about twenty-eight weeks, more preferably from about seven to about ten weeks. In a preferred dosage regimen, the siRNA is injected at or near the site of neovascularization (*e.g*., intravitreally) once a week for seven weeks. It is understood that periodic administrations of the siRNA of the invention for an indefinite length of time may be necessary for subjects suffering from a chronic neovascularization disease, such as wet ARMD or diabetic retinopathy.

Where a dosage regimen comprises multiple administrations, it is understood that the effective amount of siRNA administered to the subject can comprise the total amount of siRNA administered over the entire dosage regimen.

The siRNA of the invention are preferably formulated as pharmaceutical compositions prior to administering to a subject, according to techniques known in the art. Pharmaceutical compositions of the present invention are characterized as being at least sterile and pyrogen-free. As used herein, "pharmaceutical formulations" include formulations for human and veterinary use. Methods for preparing pharmaceutical compositions of the invention are within the skill in the art, for example as described in Remington's Pharmaceutical Science, 17th ed., Mack Publishing Company, Easton, Pa. (1985), the entire disclosure of which is herein incorporated by reference.

The present pharmaceutical formulations comprise an siRNA of the invention (*e.g*., 0.1 to 90% by weight), or a physiologically acceptable salt thereof, mixed with a physiologically acceptable carrier medium. Preferred physiologically acceptable carrier media are water, buffered water, saline solutions (*e.g*., normal saline or balanced saline solutions such as Hank's or Earle's balanced salt solutions), 0.4% saline, 0.3% glycine, hyaluronic acid and the like.

Pharmaceutical compositions of the invention can also comprise conventional pharmaceutical excipients and/or additives. Suitable pharmaceutical excipients include stabilizers, antioxidants, osmolality adjusting agents, buffers, and pH adjusting agents. Suitable additives include physiologically biocompatible buffers (*e.g*., tromethamine hydrochloride), additions of chelants (such as, for example, DTPA or DTPA-bisamide) or calcium chelate complexes (as for example calcium DTPA, CaNaDTPA-bisamide), or, optionally, additions of calcium or sodium salts (for example, calcium chloride, calcium ascorbate, calcium gluconate or calcium lactate). Pharmaceutical compositions of the invention can be packaged for use in liquid form, or can be lyophilized.

For topical administration to the eye, conventional intraocular delivery reagents can be used. For example, pharmaceutical compositions of the invention for topical intraocular delivery can comprise saline solutions as described above, corneal penetration enhancers, insoluble particles, petrolatum or other gel-based ointments, polymers which undergo a viscosity increase upon instillation in the eye, or mucoadhesive polymers. Preferably, the intraocular delivery reagent increases corneal penetration, or prolongs preocular retention of the siRNA through viscosity effects or by establishing physicochemical interactions with the mucin layer covering the corneal epithelium.

Suitable insoluble particles for topical intraocular delivery include the calcium phosphate particles described in U.S. Pat. No. 6,355,271 of Bell et al., the entire disclosure of which is herein incorporated by reference. Suitable polymers which undergo a viscosity increase upon instillation in the eye include polyethylenepolyoxypropylene block copolymers such as poloxamer 407 (*e.g*., at a concentration of 25%), cellulose acetophthalate (*e.g*., at a concentration of 30%), or a low-acetyl gellan gum such as Gelrite® (available from CP Kelco, Wilmington, DE). Suitable mucoadhesive polymers include hydrocolloids with multiple hydrophilic functional groups such as carboxyl, hydroxyl, amide and/or sulfate groups; for example, hydroxypropylcellulose, polyacrylic acid, high-molecular weight polyethylene glycols (*e.g*., >200,000 number average molecular weight), dextrans, hyaluronic acid, polygalacturonic acid, and xylocan. Suitable corneal penetration enhancers include cyclodextrins, benzalkonium chloride, polyoxyethylene glycol lauryl ether (*e.g*., Brij® 35), polyoxyethylene glycol stearyl ether (*e.g*., Brij® 78), polyoxyethylene glycol oleyl ether (*e.g*., Brij® 98), ethylene diamine tetraacetic acid (EDTA), digitonin, sodium taurocholate, saponins and polyoxyethylated castor oil such as Cremaphor EL.

For solid compositions, conventional nontoxic solid carriers can be used; for example, pharmaceutical grades of mannitol, lactose, starch, magnesium stearate, sodium saccharin, talcum, cellulose, glucose, sucrose, magnesium carbonate, and the like.

For example, a solid pharmaceutical composition for oral administration can comprise any of the carriers and excipients listed above and 10-95%, preferably 25%-75%, of one or more siRNA of the invention. A pharmaceutical composition for aerosol (inhalational) administration can comprise 0.01-20% by weight, preferably 1%-10% by weight, of one or more siRNA of the invention encapsulated in a liposome as described above, and propellant. A carrier can also be included as desired; *e.g*., lecithin for intranasal delivery.

The invention will now be illustrated with the following non-limiting examples. The animal experiments described in Examples 4-6 and 8-9 were performed using the University of Pennsylvania institutional guidelines for the care and use of animals in research. The animal experiment described in Example 10 will be performed in accordance with the Standard Operating Procedures of Sierra Biomedical, 587 Dunn Circle, Sparks, NV, 89431.

### Example 1 - siRNA Transfection and Hypoxia Induction In Vitro

*siRNA Design -* A 19 nt sequence located 329 nt from the 5' end of human VEGF mRNA was chosen as a target sequence: AAACCTCACCAAGGCCAGCAC (SEQ ID NO: 51). To ensure that it was not contained in the mRNA from any other genes, this target sequence was entered into the BLAST search engine provided by NCBI. The use of the BLAST algorithm is described in Altschul et al. (1990), J. Mol. Biol. 215: 403-410 and Altschul et al. (1997), Nucleic Acids Res. 25: 3389-3402, the disclosures of which are herein incorporated by reference in their entirety. As no other mRNA was found which contained the target sequence, an siRNA duplex was synthesized to target this sequence (Dharmacon Research, Inc., Lafayette, CO).

The siRNA duplex had the following sense and antisense strands.
sense:
5'-accucaccaaggccagcac**TT**-3' (SEQ ID NO: 77).
antisense:
5'-gugcuggccuuggugaggu**TT**-3' (SEQ ID NO: 78).

Together, the siRNA sense and antisense strands formed a 19 nt doublestranded siRNA with TT 3' overhangs (shown in bold) on each strand. This siRNA was termed "Candidate 5" or "Cand5." Other siRNA which target human VEGF mRNA were designed and tested as described for Cand5.

An siRNA targeting the following sequence in green fluorescent protein (GFP) mRNA was used as a nonspecific control: GGCTACGTCCAGCGCACC (SEQ ID NO: 79). The siRNA was purchased from Dharmacon (Lafayette, CO).

*siRNA Transfection and Hypoxia Induction* In Vitro - Human cell lines (293; Hela and ARPE19) were separately seeded into 24-well plates in 250 microliters of complete DMEM medium one day prior to transfection, so that the cells were ∼50% confluent at the time of transfection. Cells were transfected with 2.5 nM Cand5 siRNA, and with either no siRNA or 2.5 nM non-specific siRNA (targeting GFP) as controls. Transfections were performed in all cell lines with the "Transit TKO Transfection" reagent, as recommended by the manufacturer (Mirus).

Twenty four hours after transfection, hypoxia was induced in the cells by the addition of desferoxamide mesylate to a final concentration of 130 micromolar in each well. Twenty four hours post-transfection, the cell culture medium was removed from all wells, and a human VEGF ELISA (R&D systems, Minneapolis, MN) was performed on the culture medium as described in the Quantikine human VEGF ELISA protocol available from the manufacturer, the entire disclosure of which is herein incorporated by reference.

As can be seen in Fig. 1, RNAi degradation induced by Cand5 siRNA significantly reduces the concentration of VEGF produced by the hypoxic 293 and HeLa cells. There was essentially no difference in the amount of VEGF produced by hypoxic cells treated with either no siRNA or the non-specific siRNA control. Similar results were also seen with human ARPE19 cells treated under the same conditions. Thus, RNA interference with VEGF-targeted siRNA disrupts the pathogenic up-regulation of VEGF in human cultured cells *in vitro.*

The experiment outlined above was repeated on mouse NIH 3T3 cells using a mouse-specific VEGF siRNA (see Example 6 below), and VEGF production was quantified with a mouse VEGF ELISA (R&D systems, Minneapolis, MN) as described in the Quantikine mouse VEGF ELISA protocol available from the manufacturer, the entire disclosure of which is herein incorporated by reference. Results similar to those reported in Fig. 1 for the human cell lines were obtained.

### Example 2 - Effect of Increasing siRNA Concentration on VEGF Production in Human Cultured Cells

The experiment outlined in Example 1 was repeated with human 293, HeLa and ARPE19 cells using a range of siRNA concentrations from 10 nM to 50 nM. The ability of the Cand5 siRNA to down-regulate VEGF production increased moderately up to approximately 13 nM siRNA, but a plateau effect was seen above this concentration. These results highlight the catalytic nature of siRNA-mediated RNAi degradation of mRNA, as the plateau effect appears to reflect VEGF production from the few cells not transfected with the siRNA. For the majority of cells which had been transfected with the siRNA, the increased VEGF mRNA production induced by the hypoxia is outstripped by the siRNA-induced degradation of the target mRNA at siRNA concentrations greater than about 13 nM.

### Example 3 - Specificity of siRNA Targeting

NIH 3T3 mouse fibroblasts were grown in 24-well plates under standard conditions, so that the cells were ∼50% confluent one day prior to transfection. The human VEGF siRNA Cand5 was transfected into a NIH 3T3 mouse fibroblasts as in Example 1. Hypoxia was then induced in the transfected cells, and murine VEGF concentrations were measured by ELISA as in Example 1.

The sequence targeted by the human VEGF siRNA Cand5 differs from the murine VEGF mRNA by one nucleotide. As can be seen in Fig. 2, the human VEGF siRNA has no affect on the ability of the mouse cells to up-regulate mouse VEGF after hypoxia. These results show that siRNA induced RNAi degradation is sequence-specific to within a one nucleotide resolution.

### Example 4 - In Vivo delivery of siRNA to Murine Retinal Pigment Epithelial Cells

VEGF is upregulated in the retinal pigment epithelial (RPE) cells of human patients with age-related macular degeneration (ARMD). To show that functional siRNA can be delivered to RPE cells *in vivo,* GFP was expressed in mouse retinas with a recombinant adenovirus, and GFP expression was silenced with siRNA. The experiment was conducted as follows.

One eye from each of five adult C57/Black6 mice (Jackson Labs, Bar Harbor, ME) was injected subretinally as described in Bennett et al. (1996), *supra*., with a mixture containing ∼1x10⁸ particles of adenovirus containing eGFP driven by the CMV promoter and 20 picomoles of siRNA targeting eGFP conjugated with transit TKO reagent (Mirus).

As positive control, the contralateral eyes were injected with a mixture containing ∼1x10⁸ particles of adenovirus containing eGFP driven by the CMV promoter and 20 picomoles of siRNA targeting human VEGF conjugated with transit TKO reagent (Mirus). Expression of GFP was detected by fundus ophthalmoscopy 48 hours and 60 hours after injection. Animals were sacrificed at either 48 hours or 60 hours post-injection. The eyes were enucleated and fixed in 4% paraformaldehyde, and were prepared either as flat mounts or were processed into 10 micron cryosections for fluorescent microscopy.

No GFP fluorescence was detectable by ophthalmoscopy in the eyes which received the siRNA targeted to GFP mRNA in 4 out of 5 mice, whereas GFP fluorescence was detectable in the contralateral eye which received the non-specific control siRNA. A representative flat mount analyzed by fluorescence microscopy showed a lack of GFP fluorescence in the eye which received GFP siRNA, as compared to an eye that received the non-specific control siRNA. Cryosections of another retina showed that the recombinant adenovirus efficiently targets the RPE cells, and when the adenovirus is accompanied by siRNA targeted to GFP mRNA, expression of the GFP transgene is halted.

While there is some GFP fluorescence detectable by fluorescence microscopy in eyes that received siRNA targeted to GFP mRNA, the fluorescence is greatly suppressed as compared to controls that received non-specific siRNA. These data demonstrate that functional siRNA can be delivered *in vivo* to RPE cells.

### Example 5 In Vivo Expression and siRNA-Induced RNAi Degradation of Human VEGF in Murine Retinas

In order to demonstrate that siRNA targeted to VEGF functioned *in vivo*, an exogenous human VEGF expression cassette was delivered to mouse RPE cells via an adenovirus by subretinal injection, as in Example 4. One eye received Cand5 siRNA, and the contralateral eye received siRNA targeted to GFP mRNA. The animals were sacrificed 60 hours post-injection, and the injected eyes were removed and snap frozen in liquid N₂ following enucleation. The eyes were then homogenized in lysis buffer, and total protein was measured using a standard Bradford protein assay (Roche, Germany). The samples were normalized for total protein prior to assaying for human VEGF by ELISA as described in Example 1.

The expression of VEGF was somewhat variable from animal to animal. The variability of VEGF levels correlated well to those observed in the GFP experiments of Example 4, and can be attributed to some error from injection to injection, and the differential ability of adenovirus to delivery the target gene in each animal. However, there was a significant attenuation of VEGF expression in each eye that received VEGF siRNA, as compared to the eyes receiving the non-specific control siRNA (Figure 4). These data indicate that the Cand5 siRNA was potent and effective in silencing human VEGF expression in murine RPE cells *in vivo.*

### Example 6 - Inhibition of Choroidal Neovascularization in the Mouse CNV Model

There is evidence that choroidal neovascularization in ARMD is due to the upregulation of VEGF in the RPE cells. This human pathologic condition can be modeled in the mouse by using a laser to burn a spot on the retina ("laser photo-coagulation" or "laser induction"). During the healing process, VEGF is believed to be up-regulated in the RPE cells of the burned region, leading to re-vascularization of the choroid. This model is called the mouse choroidal neovascularization ("CNV") model.

For rescue of the mouse CNV model, a mouse siRNA was designed that incorporated a one nucleotide change from the human "Cand5" siRNA from Example 1. The mouse siRNA specifically targeted mouse VEGF mRNA at the sequence AAACCUCACCAAAGCCAGCAC (SEQ ID NO: 80). Other siRNA that target mouse VEGF were also designed and tested. The GFP siRNA used as a nonspecific control in Example 1 was also used as a non-specific control here.

Twenty four hours after laser induction, one eye from each of eleven adult C57/Black6 mice (Jackson Labs, Bar Harbor, ME) was injected subretinally with a mixture containing ∼1x10⁸ particles of adenovirus containing LacZ driven by the CMV promoter and 20 picomoles of siRNA targeting mouse VEGF conjugated with transit TKO reagent (Mirus), as in Example 4. As a control, contralateral eyes received a mixture containing ∼1x10⁸ particles of adenovirus containing LacZ driven by the CMV promoter and 20 picomoles of siRNA targeting GFP conjugated with transit TKO reagent (Mirus).

Fourteen days after the laser treatment, the mice were perfused with fluorescein and the area of neovascularization was measured around the burn spots. Areas of the burn spots in the contra-lateral eye were used as a control. The site of neovascularization around the burn spots in animals that received siRNA targeting mouse VEGF was, on average, 1/4 the area of the control areas. These data support the use of VEGF-directed siRNA (also called "anti-VEGF siRNA") for therapy of ARMD.

### Example 7 - Generation of an Adeno-Associated Viral Vector for Expression of siRNA

A "cis-acting" plasmid for generating a recombinant AAV vector for delivering an siRNA of the invention was generated by PCR based subcloning, essentially as described in Samulski R et al. (1987), *supra.* The cis-acting plasmid was called "pAAVsiRNA."

The *rep* and *cap* genes of psub201 were replaced with the following sequences in this order: a 19 nt sense RNA strand coding sequence in operable connection with a polyT termination sequence under the control of a human U6 RNA promoter, and a 19 nt antisense RNA strand coding sequence in operable connection with a polyT termination sequence under the control of a human U6 RNA promoter. A schematic representation of pAAVsiRNA is given if Fig. 5.

A recombinant AAV siRNA vector was obtained by transfecting pAAVsiRNA into human 293 cells previously infected with El-deleted adenovirus, as described in Fisher KJ et al. (1996), *supra.* The AAV rep and cap functions were provided by a trans-acting plasmid pAAV/Ad as described in Samulski R et al. (1989), *supra.* Production lots of the recombinant AAV siRNA vector were titered according to the number of genome copies/ml, as described in Fisher KJ et al. (1996), *supra.*

### Example 8 - VEGF-Directed siRNA Inhibits Experimental Choroidal Neovascularization

The ability of murine VEGF-directed siRNA to inhibit experimental laser-induced choroidal neovascularization (CNV) in mice was tested as follows.

The retinas of adult female C57BL/6 mice were laser photocoagulated using an 810 nm diode laser (75 um, 140 mw, 0.10 seconds) (OcuLight Six; IRIS Medical, Mountain View, CA). Three laser spots were applied to both eyes of each mouse. Thirty-six hours following laser photocoagulation, an siRNA targeted to mouse VEGF ("mVEGF1.siRNA") was delivered subretinally or intravitreally to one eye of each mouse. For subretinal injection, the siRNA was conjugated with Transit TKO transfection reagent (Mirus) and mixed with recombinant adenovirus (rAdenovirus). For intravitreal injection, the siRNA was delivered in the absence of transfection reagent and rAdenovirus. As a control, the contralateral eyes of each mouse received subretinal or intravitreal injections of identical formulations with an siRNA targeted to GFP ("GFP1.siRNA"), which has no homology to mouse VEGF.

Fourteen days following laser treatment, all animals were perfused with high molecular weight FITC-dextran, choroidal flat mounts were prepared as described above, and the flat mounts were photographed and analyzed microscopically in a masked fashion. The area of CNV in each flat mount was measured with Openlab software (Improvision, Boston, MA). The mean areas of CNV in eyes treated with mVEGF1.siRNA were significantly smaller than those areas from GFP1.siRNA-treated eyes for both subretinal (Fig. 6A; P<0.003) and intravitreal (Fig. 6B; P<0.04) delivery.

In a second experiment, the retinas of adult female C57BL/6 mice were laser photocoagulated as described above, and the animals were divided into control and test groups. One day following laser photocoagulation, phosphate buffered saline was delivered intravitreally to the animals of the control group, which were perfused with dextran-fluorescein 14 days after laser treatment. Choroidal flat mounts were then prepared and the areas of CNV in each flat mount were measured as above.

Fourteen days following laser photocoagulation, mVEGF1.siRNA was delivered by intravitreal injection into one eye of each mouse in the test group. Contralateral eyes were injected with GFP1.siRNA as a control. The test group animals were perfused with high molecular weight dextran-fluorescein 21 days after laser treatment. Choroidal flat mounts were then prepared and the areas of CNV in each flat mount were measured, as above.

In this latter experiment, the anti-VEGF siRNA was administered during CNV growth, as opposed to before CNV growth, and thus is more representative of the condition of human patients presenting with wet AMD. As can be seen from Fig. 6, the mean areas of CNV in mVEGF1.siRNA-treated eyes were significantly smaller than those areas measured in GFP1.siRNA-treated eyes (Fig. 6C; P<0.05). The mean areas of CNV in mVEGF1.siRNA-treated eyes at day 21 and control ("PBS") eyes at day 14 were not significantly different (Fig. 6C; P=0.469).

The results of these experiments indicate that age-related macular degeneration can be treated with anti-VEGF siRNA.

### Example 9 - In Vivo RNA Interference of Human VEGF Induced by anti-VEGF siRNA in Murine RPE Cells

The ability of Cand5 siRNA to induce RNAi of VEGF *in vivo* over time was evaluated as follows.

AAV.CMV.VEGF, which expresses human VEGF from an adeno-associated viral vector, was generously provided by Dr. A. Auricchio. AAV.CMV.VEGF was injected subretinally and bilaterally in eyes of five C57Bl/6 mice. Twenty-eight days after injection of AAV.CMV.VEGF, Cand5 siRNA was delivered by intravitreal injection into one eye and control GFP1.siRNA was delivered by intravitreal injection in the contralateral eye of each animal.

At day 0 (pre-siRNA injection), and at 6, 10 and 14 days after siRNA injection, the mice were sacrificed and the eyes were snap frozen in liquid nitrogen following enucleation. The eyes were then homogenized in lysis buffer (Roche, Basel, Switzerland), and total protein was measured using a Bradford assay, as in Example 5 above. Two mice were used for the 0 day time point (n=2), and three mice each were used for the 6, 10 and 14 day time points (n=3). The samples were normalized for total protein prior to assaying for human VEGF by ELISA, according to the manufacturer's recommendations (R&D systems, Minneapolis, Minnesota). Percent of VEGF (%VEGF) for each mouse was calculated as the concentration of VEGF ("[VEGF]") in the eye injected with Cand5 divided by the [VEGF] in the eye injected with GFP1.siRNA, multiplied by 100.

As can be seen from Figure 7, a single injection of Cand5 induced an RNAi-mediated decrease in VEGF levels of approximately 70% by day 6 post-siRNA injection, with a reduction in VEGF production of approximately 35% continuing through at least day 14 post-siRNA injection. These results indicate that siRNA directed against human VEGF is capable of inducing RNAi of human VEGF *in vivo* for a sustained period of time.

### Example 10 - In Vivo RNA Interference of VEGF in Monkeys with Anti-VEGF siRNA

It is expected that 10 naïve cynomolgus monkeys (*Macaca fascicularis*) will be subjected to laser photocoagulation of the retina in both eyes to induce choroidal neovascularization ("CNV"). The following represents the intended experimental plan to evaluate the ability of Cand5 siRNA to reduce the area of CNV in the monkeys. As the VEGF RNA sequence targeted by Cand5 is identical in both *Homo sapiens* and *M. fascicularis,* Cand5 is expected to induce RNAi of *M. fascicularis* VEGF RNA.

The experiment will be carried out by Sierra Biomedical ("SBi"), 587 Dunn Circle, Sparks, NV, 89431, in accordance with SBi's Standard Operating Procedures. All monkeys will undergo a full pre-study health screen consisting of a physical examination, hematologic and ophthalmologic evaluations, serum chemistry, and electroretinography ("ERG"). A pre-study fluorescein angiography of the monkeys' eyes will also be performed, and intraocular pressure will be measured pre-study and at two additional time points during the study period.

On day zero, both eyes of all 10 monkeys will be laser photocoagulated to induce choroidal neovascularization. The animals will receive twice-daily cageside observations and a once-daily qualitative assessment of food consumption throughout the experiment. At 14 days post laser induction, the monkeys will receive intravitreally one 50 microliter dose of a Treatment or Control formulation (see below) in each eye, in a randomized, double-blind fashion. For example, a given animal could receive a Treatment 1 dose in the right eye, and a Control dose in the left eye. Another animal could receive a Treatment 1 dose in the right eye, and a Treatment 3 dose in the left eye. It is expected that four eyes will receive each formulation, for a total of 1 control and four treatment groups of four eyes each.

The formulations will be: Control - balanced saline solution ("BSS") alone; Treatment 1 - 1 mg/ml Cand5 in BSS; Treatment 2 - 2.5 mg/ml Cand5 in BSS; Treatment 3 - 5 mg/ml Cand5 in BSS; and Treatment 4 - 10 mg/ml Cand5 in BSS. Prior to injection, it is expected that the pH, osmolarity, and the absorbance at 260 nm of each formulation will be measured.

After laser induction, a fluorescein angiography will be performed on both eyes of each monkey weekly up through the seventh week after laser induction. The monkeys will then be sacrificed, and a complete necropsy will be performed with a full tissue collection (∼45 tissues including a vitreous sample) and histopathologic evaluation of the collected tissues. An ERG will be taken pre-necropsy.

It is expected that the laser-induced areas of CNV will be reduced upon administration of the Cand5 siRNA, with the CNV area decreasing with increasing dose of Can5. However, a plateau effect such as is described above in Example 2 may be observed. The Cand5 siRNA is not expected to effect any other organ or tissue except the choroid in the eye.

## Claims

1. An isolated short interfering ribonucleic acid (siRNA) comprising a sense RNA strand from about 19 to about 25 nucleotides in length and an antisense RNA strand from about 19 to about 25 nucleotides in length, wherein the sense and antisense RNA strands form an RNA duplex, and wherein the sense RNA strand comprises a nucleotide sequence identical to a target sequence in human vascular endothelial growth factor (VEGF) mRNA wherein said target sequence is selected from SEQ ID No. 47, SEQ ID No. 48, SEQ ID No. 50, SEQ ID No. 51, SEQ ID No. 52 and SEQ ID No. 56.

2. The short interfering ribonucleic acid (siRNA) of claim 1, wherein the human VEGF mRNA is selected from the group consisting of VEGF₁₂₁ mRNA (SEQ ID NO: 2); VEGF₁₆₅ mRNA (SEQ ID NO: 3); VEGF₁₈₉ mRNA (SEQ ID NO: 4) and VEGF₂₀₆ mRNA (SEQ ID NO: 5).

3. The short interfering ribonucleic acid (siRNA) of claim 1, wherein the sense RNA strand comprises SEQ ID NO: 77, and the antisense strand comprises SEQ ID NO: 78.

4. The short interfering ribonucleic acid (siRNA) of claim 1, wherein the sense RNA strand comprises one RNA molecule, and the antisense RNA strand comprises one RNA molecule.

5. The short interfering ribonucleic acid (siRNA) of claim 1, wherein the sense and antisense RNA strands forming the RNA duplex are covalently linked by a single-stranded hairpin.

6. The short interfering ribonucleic acid (siRNA) of claim 1, wherein the siRNA further comprises non-nucleotide material.

7. The short interfering ribonucleic acid (siRNA) of claim 1, wherein the sense and antisense RNA strands are stabilized against nuclease degradation.

8. The short interfering ribonucleic acid (siRNA) of claim 1, further comprising a 3' overhang.

9. The short interfering ribonucleic acid (siRNA) of claim 8, wherein the 3' overhang comprises from 1 to about 6 nucleotides.

10. The short interfering ribonucleic acid (siRNA) of claim 8, wherein the 3' overhang comprises about 2 nucleotides.

11. The short interfering ribonucleic acid (siRNA) of claim 4, wherein the sense RNA strand comprises a first 3' overhang, and the antisense RNA strand comprises a second 3' overhang.

12. The short interfering ribonucleic acid (siRNA) of claim 11, wherein the first and second 3' overhangs separately comprise from 1 to about 6 nucleotides.

13. The short interfering ribonucleic acid (siRNA) of claim 12, wherein the first 3' overhang comprises a dinucleotide and the second 3' overhang comprises a dinucleotide.

14. The short interfering ribonucleic acid (siRNA) of claim 13, where the dinucleotide comprising the first and second 3' overhang is dithymidylic acid (TT) or diuridylic acid (uu).

15. The short interfering ribonucleic acid (siRNA) of claim 8, wherein the 3' overhang is stabilized against nuclease degradation.

16. A retinal pigment epithelial cell comprising the short interfering ribonucleic acid (siRNA) of claim 1.

17. A recombinant plasmid comprising nucleic acid sequences for expressing a short interfering ribonucleic acid (siRNA) comprising a sense RNA strand and an antisense RNA strand, wherein the sense and antisense RNA strands form an RNA duplex, and wherein the sense RNA strand comprises a nucleotide sequence identical to a target sequence in human vascular endothelial growth factor (VEGF) mRNA wherein said target sequence is selected from SEQ ID No. 47, SEQ ID No. 48, SEQ ID No. 50, SEQ ID No. 51, SEQ ID No. 52 and SEQ ID No. 56.

18. The recombinant plasmid of claim 17 wherein the nucleic acid sequences express a short interfering ribonucleic acid (siRNA) wherein the sense RNA strand comprises SEQ ID NO: 77, and the antisense RNA strand comprises SEQ ID NO: 78.

19. The recombinant plasmid of claim 17, wherein the nucleic acid sequences for expressing the short interfering ribonucleic acid (siRNA) comprise an inducible or regulatable promoter.

20. The recombinant plasmid of claim 17, wherein the nucleic acid sequences for expressing the short interfering ribonucleic acid (siRNA) comprise a sense RNA strand coding sequence in operable connection with a polyT termination sequence under the control of a human U6 RNA promoter, and an antisense RNA strand coding sequence in operable connection with a polyT termination sequence under the control of a human U6 RNA promoter.

21. The recombinant plasmid of claim 18, wherein the plasmid is pAAV short interfering ribonucleic acid (siRNA).

22. A recombinant viral vector comprising nucleic acid sequences for expressing a short interfering ribonucleic acid (siRNA) comprising a sense RNA strand and an antisense RNA strand, wherein the sense and antisense RNA strands form an RNA duplex, and wherein the sense RNA strand comprises a nucleotide sequence identical to a target sequence in human vascular endothelial growth factor (VEGF) mRNA wherein said target sequence is selected from SEQ ID No. 47, SEQ ID No. 48, SEQ ID No. 50, SEQ ID No. 51, SEQ ID No. 52 and SEQ ID No. 56.

23. The recombinant viral vector of claim 22 wherein the nucleic acid sequences express a short interfering ribonucleic acid (siRNA) wherein the sense RNA strand comprises SEQ ID NO: 77, and the antisense RNA strand comprises SEQ ID NO: 78.

24. The recombinant viral vector of claim 22, wherein the nucleic acid sequences for expressing the short interfering ribonucleic acid (siRNA) comprise an inducible or regulatable promoter.

25. The recombinant viral vector of claim 22, wherein the nucleic acid sequences for expressing the short interfering ribonucleic acid (siRNA) comprise a sense RNA strand coding sequence in operable connection with a polyT termination sequence under the control of a human U6 RNA promoter, and an antisense RNA stand coding sequence in operable connection with a polyT termination sequence under the control of a human U6 RNA promoter.

26. The recombinant viral vector of claim 22, wherein the recombinant viral vector is selected from the group consisting of an adenoviral vector, an adeno-associated viral vector, a lentiviral vector, a retroviral vector, and a herpes virus vector.

27. The recombinant viral vector of claim 22, wherein the recombinant viral vector is pseudotyped with surface proteins from vesicular stomatitis virus, rabies virus, Ebola virus, or Mokola virus.

28. The recombinant viral vector of claim 27, wherein the recombinant viral vector comprises an adeno-associated viral vector.

29. A pharmaceutical composition comprising an effective amount of a short interfering ribonucleic acid (siRNA) and a pharmaceutically acceptable carrier, wherein the siRNA comprises a sense RNA strand and an antisense RNA strand, wherein the sense and antisense RNA strands form an RNA duplex, and wherein the sense RNA strand comprises a nucleotide sequence identical to a target sequence in human vascular endothelial growth factor (VEGF) mRNA wherein said target sequence is selected from SEQ ID No. 47, SEQ ID No. 48, SEQ ID No. 50, SEQ ID No. 51, SEQ ID No. 52 and SEQ ID No. 56.

30. The pharmaceutical composition as claimed in claim 29 wherein the sense RNA strand comprises SEQ ID NO: 77, and the antisense RNA strand comprises SEQ ID NO: 78.

31. The pharmaceutical composition of claim 29, further comprising lipofectin, lipofectamine, cellfectin, polycations, or liposomes.

32. A pharmaceutical composition comprising the plasmid of claim 17, or a physiologically acceptable salt thereof, and a pharmaceutically acceptable carrier.

33. The pharmaceutical composition of claim 32, further comprising lipofectin, lipofectamine, cellfectin, polycations, or liposomes.

34. A pharmaceutical composition comprising the viral vector of claim 22 and a pharmaceutically acceptable carrier.

35. An effective amount of short interfering ribonucleic acid (siRNA) comprising a sense RNA strand and an antisense RNA strand, wherein the sense and antisense RNA strands form an RNA duplex, and wherein the sense RNA strand comprises a nucleotide sequence identical to a target sequence in human vascular endothelial growth factor (VEGF) mRNA for use in inhibiting expression of human vascular endothelial growth factor (VEGF) mRNA in a subject wherein said target sequence is selected from SEQ ID No. 47, SEQ ID No. 48, SEQ ID No. 50, SEQ ID No. 51, SEQ ID No. 52 and SEQ ID No. 56.

36. The short interfering ribonucleic acid (siRNA) of claim 35 wherein the sense RNA strand comprises SEQ ID NO: 77, and the antisense RNA strand comprises SEQ ID NO: 78.

37. The short interfering ribonucleic acid (siRNA) of claim 35, wherein the subject is a human being.

38. The short interfering ribonucleic acid (siRNA) of claim 35, wherein expression of human vascular endothelial growth factor (VEGF) mRNA is inhibited in one or both eyes of the subject.

39. The short interfering ribonucleic acid (siRNA) of claim 35, wherein expression of human vascular endothelial growth factor (VEGF) mRNA is inhibited in retinal pigment epithelial cells of the subject.

40. The short interfering ribonucleic acid (siRNA) of claim 35, wherein the effective amount of the short interfering ribonucleic acid (siRNA) is from about 1 nM to about 100nM.

41. The short interfering ribonucleic acid (siRNA) of claim 35, wherein the short interfering ribonucleic acid (siRNA) is administered in conjunction with a delivery agent.

42. The short interfering ribonucleic acid (siRNA) of claim 41, wherein the delivery agent is selected from the group consisting of lipofectin, lipofectamine, cellfectin, polycations, and liposomes.

43. The short interfering ribonucleic acid (siRNA) of claim 42, wherein the delivery agent is a liposome.

44. The short interfering ribonucleic acid (siRNA) of claim 43, wherein the liposome comprises a ligand which targets the liposome to cells at or near the site of angiogenesis.

45. The short interfering ribonucleic acid (siRNA) of claim 44, wherein the ligand binds to receptors on tumor cells or vascular endothelial cells.

46. The short interfering ribonucleic acid (siRNA) of claim 45, wherein the ligand comprises a monoclonal antibody.

47. The short interfering ribonucleic acid (siRNA) of claim 44, wherein the liposome is modified with an opsonization-inhibition moiety.

48. The short interfering ribonucleic acid (siRNA) of claim 47, wherein the opsonization-inhibiting moiety comprises a PEG, PPG, or derivatives thereof.

49. The short interfering ribonucleic acid (siRNA) of claim 36, wherein the short interfering ribonucleic acid (siRNA) is expressed from a recombinant plasmid.

50. The short interfering ribonucleic acid (siRNA) of claim 36, wherein the short interfering ribonucleic acid (siRNA) is expressed from a recombinant viral vector.

51. The short interfering ribonucleic acid (siRNA) of claim 50, wherein the recombinant viral vector comprises an adenoviral vector, an adeno-associated viral vector, a lentiviral vector, a retroviral vector, or a herpes virus vector.

52. The short interfering ribonucleic acid (siRNA) of claim 51, wherein the recombinant viral vector is pseudotyped with surface proteins from vesicular stomatitis virus, rabies virus, Ebola virus, or Mokola virus.

53. The short interfering ribonucleic acid (siRNA) of claim 36, wherein the siRNA is administered by an enteral administration route.

54. The short interfering ribonucleic acid (siRNA) of claim 53, wherein the enteral administration route is selected from the group consisting of oral, rectal, and intranasal.

55. The short interfering ribonucleic acid (siRNA) of claim 36, wherein the short interfering ribonucleic acid (siRNA) is administered by a parenteral administration route.

56. The short interfering ribonucleic acid (siRNA) of claim 55, wherein the parenteral administration route is selected from the group consisting of intraocular administration, intravascular administration, peri- and intra-tissue injection, subcutaneous injection or deposition, subcutaneous infusion, and direct application at or near the site of neovascularization.

57. The short interfering ribonucleic acid (siRNA) of claim 56, wherein the intravascular administration is selected from the group consisting of intravenous bolus injection, intravenous infusion, intra-arterial bolus injection, intra-arterial infusion and catheter instillation into the vasculature.

58. The short interfering ribonucleic acid (siRNA) of claim 56, wherein the peri- and intra-tissue injection is comprises peri-tumoral injection or intra-tumoral injection.

59. The short interfering ribonucleic acid (siRNA) of claim 56, wherein the intraocular administration comprises intravitreal, intraretinal, subretinal, subtenon, peri- and retro-orbital, trans-corneal or trans-scleral administration.

60. The short interfering ribonucleic acid (siRNA) of claim 56, wherein the direct application at or near the site of neovascularization comprises application by catheter, corneal pellet, eye dropper, suppository, an implant comprising a porous material, an implant comprising a non-porous material, or an implant comprising a gelatinous material.

61. The short interfering ribonucleic acid (siRNA) of claim 56, wherein the site of neovascularization is in the eye, and the direct application at or near the site of neovascularization comprises application by an ocular implant.

62. The short interfering ribonucleic acid (siRNA) of claim 61, wherein the ocular implant is biodegradable.

63. An effective amount of short interfering ribonucleic acid (siRNA) comprising a sense RNA strand and an antisense RNA strand, wherein the sense and antisense RNA strands form an RNA duplex, and wherein the sense RNA strand comprises a nucleotide sequence identical to a target sequence in human vascular endothelial growth factor (VEGF) mRNA wherein said target sequence is selected from SEQ ID No. 47, SEQ ID No. 48, SEQ ID No. 50, SEQ ID No. 51, SEQ ID No. 52 and SEQ ID No. 56 for use in preventing angiogenesis.

64. The short interfering ribonucleic acid (siRNA) of claim 63 wherein the sense RNA strand comprises SEQ ID NO: 77, and the antisense RNA strand comprises SEQ ID NO: 78.

65. The short interfering ribonucleic acid (siRNA) of claim 63, wherein the angiogenesis is pathogenic.

66. The short interfering ribonucleic acid (siRNA) of claim 63, wherein the angiogenesis is non-pathogenic.

67. The short interfering ribonucleic acid (siRNA) of claim 66, wherein the non-pathogenic angiogenesis is associated with production of fatty tissues or cholesterol production.

68. The short interfering ribonucleic acid (siRNA) of claim 66, wherein the non-pathogenic angiogenesis comprises endometrial neovascularization.

69. The short interfering ribonucleic acid (siRNA) of claim 63, wherein the angiogenesis is inhibited in one or both eyes of the subject.

70. The short interfering ribonucleic acid (siRNA) of claim 63, wherein the angiogenesis is inhibited in retinal pigment epithelial cells of the subject.

71. An effective amount of short interfering ribonucleic acid (siRNA) comprising a sense RNA strand and an antisense RNA strand, wherein the sense and antisense RNA strands form an RNA duplex, and wherein the sense RNA strand comprises a nucleotide sequence identical to a target sequence in human vascular endothelial growth factor (VEGF) mRNA wherein said target sequence is selected from SEQ ID No. 47, SEQ ID No. 48, SEQ ID No. 50, SEQ ID No. 51, SEQ ID No. 52 and SEQ ID No. 56 for use in treating angiogenic disease such that angiogenesis associated with angiogenic disease is inhibited.

72. The short interfering ribonucleic acid (siRNA) of claim 71 wherein the sense RNA strand comprises SEQ ID NO: 77, and the antisense RNA strand comprises SEQ ID NO: 78.

73. The short interfering ribonucleic acid (siRNA) of claim 71, wherein the angiogenic disease comprises a tumor associated with a cancer.

74. The short interfering ribonucleic acid (siRNA) of claim 73, wherein the cancer is selected from the group consisting of breast cancer, lung cancer, head and neck cancer, brain cancer, abdominal cancer, colon caner, colorectal cancer, esophagus cancer, gastrointestinal cancer, glioma, liver cancer, tongue cancer, neuroblastoma, osteosarcoma, ovarian cancer, pancreatic cancer, prostate cancer, retinoblastoma, Wilm's tumor, multiple myeloma, skin cancer, lymphoma, and blood cancer.

75. The short interfering ribonucleic acid (siRNA) of claim 71, wherein the angiogenic disease is selected from the group consisting of diabetic retinopathy, age-related macular degeneration, and inflammatory diseases.

76. The short interfering ribonucleic acid (siRNA) of claim 75, wherein the inflammatory disease is psoriasis or rheumatoid arthritis.

77. The short interfering ribonucleic acid (siRNA) of claim 71, wherein the short interfering ribonucleic acid (siRNA) is administered in combination with a pharmaceutical agent for treating the angiogenic disease, which pharmaceutical agent is different from the short interfering ribonucleic acid (siRNA).

78. The short interfering ribonucleic acid (siRNA) of claim 77 wherein the angiogenic disease is cancer, and the pharmaceutical agent comprises a chemotherapeutic agent.

79. The short interfering ribonucleic acid (siRNA) of claim 78, wherein the chemotherapeutic agent is selected from the group consisting of cisplatin, carboplatin, cyclophosphamide, 5-fluorouracil, adriamycin, daunorubicin, and tamoxifen.

80. The short interfering ribonucleic acid (siRNA) of claim 71, wherein the short interfering ribonucleic acid (siRNA) is administered to a subject in combination with another therapeutic method designed to treat the angiogenic disease.

81. The short interfering ribonucleic acid (siRNA) of claim 80, wherein the angiogenic disease is cancer, and the short interfering ribonucleic acid (siRNA) is administered in combination with radiation therapy, chemotherapy or surgery.

82. The short interfering ribonucleic acid (siRNA) of claim 1, wherein the short interfering ribonucleic acid (siRNA) further comprises altered RNA.

## Patentansprüche

1. Eine isolierte kurze interferierende Ribonukleinsäure (siRNA), die einen RNA-Sinnstrang von etwa 19 bis zu etwa 25 Nukleotiden Länge und einen RNA-Gegensinnstrang von etwa 19 bis zu etwa 25 Nukleotiden Länge beinhaltet, wobei der RNA-Sinnstrang und der RNA -Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist.

2. Kurze interferierende Ribonukleinsäure (siRNA) gemäß Anspruch 1, wobei die menschliche VEGF-mRNA aus der Gruppe, die aus VEGF₁₂₁-mRNA (SEQ ID NO: 2); VEGF₁₆₅-mRNA (SEQ ID NO: 3); VEGF₁₈₉-mRNA (SEQ ID NO: 4) und VEGF₂₀₆-mRNA (SEQ ID NO: 5) besteht, ausgewählt ist.

3. Kurze interferierende Ribonukleinsäure (siRNA) gemäß Anspruch 1, wobei der RNA-Sinnstrang SEQ ID NO: 77 beinhaltet und der Gegensinnstrang SEQ ID NO: 78 beinhaltet.

4. Kurze interferierende Ribonukleinsäure (siRNA) gemäß Anspruch 1, wobei der RNA-Sinnstrang ein RNA-Molekül beinhaltet und der RNA-Gegensinnstrang ein RNA-Molekül beinhaltet.

5. Kurze interferierende Ribonukleinsäure (siRNA) gemäß Anspruch 1, wobei der RNA-Sinnstrang und der RNA-Gegensinnstrang, die den RNA-Doppelstrang bilden, durch eine Einzelstrang-Haarnadelstruktur kovalent verbunden sind.

6. Kurze interferierende Ribonukleinsäure (siRNA) gemäß Anspruch 1, wobei die siRNA ferner Nicht-Nukleotid-Material beinhaltet.

7. Kurze interferierende Ribonukleinsäure (siRNA) gemäß Anspruch 1, wobei der RNA-Sinnstrang und der RNA-Gegensinnstrang gegenüber Nukleaseabbau stabilisiert sind.

8. Kurze interferierende Ribonukleinsäure (siRNA) gemäß Anspruch 1, die ferner einen 3'-Überhang beinhaltet.

9. Kurze interferierende Ribonukleinsäure (siRNA) gemäß Anspruch 8, wobei der 3'-Überhang etwa 1 bis 6 Nukleotide beinhaltet.

10. Kurze interferierende Ribonukleinsäure (siRNA) gemäß Anspruch 8, wobei der 3'-Überhang etwa 2 Nukleotide beinhaltet.

11. Kurze interferierende Ribonukleinsäure (siRNA) gemäß Anspruch 4, wobei der RNA-Sinnstrang einen ersten 3'-Überhang beinhaltet und der RNA-Gegensinnstrang einen zweiten 3'-Überhang beinhaltet.

12. Kurze interferierende Ribonukleinsäure (siRNA) gemäß Anspruch 11, wobei der erste und der zweite 3'-Überhang getrennt etwa 1 bis 6 Nukleotide beinhalten.

13. Kurze interferierende Ribonukleinsäure (siRNA) gemäß Anspruch 12, wobei der erste 3'-Überhang ein Dinukleotid beinhaltet und der zweite 3'-Überhang ein Dinukleotid beinhaltet.

14. Kurze interferierende Ribonukleinsäure (siRNA) gemäß Anspruch 13, wobei das Dinukleotid, das den ersten und den zweiten 3'-Überhang beinhaltet, Dithymidylsäure (TT) oder Diuridylsäure (uu) ist.

15. Kurze interferierende Ribonukleinsäure (siRNA) gemäß Anspruch 8, wobei der 3'-Überhang gegenüber Nukleaseabbau stabilisiert ist.

16. Eine Zelle eines retinalen Pigmentepithels, die die kurze interferierende Ribonukleinsäure (siRNA) gemäß Anspruch 1 beinhaltet.

17. Ein rekombinantes Plasmid, das Nukleinsäuresequenzen zum Exprimieren einer kurzen interferierenden Ribonukleinsäure (siRNA), die einen RNA-Sinnstrang und einen RNA-Gegensinnstrang beinhaltet, beinhaltet, wobei der RNA-Sinnstrang und der RNA-Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist.

18. Rekombinantes Plasmid gemäß Anspruch 17, wobei die Nukleinsäuresequenzen eine kurze interferierende Ribonukleinsäure (siRNA) exprimieren, wobei der RNA-Sinnstrang SEQ ID NO: 77 beinhaltet und der RNA-Gegensinnstrang SEQ ID NO: 78 beinhaltet.

19. Rekombinantes Plasmid gemäß Anspruch 17, wobei die Nukleinsäuresequenzen zum Exprimieren der kurzen interferierenden Ribonukleinsäure (siRNA) einen induzierbaren oder regulierbaren Promotor beinhalten.

20. Rekombinantes Plasmid gemäß Anspruch 17, wobei die Nukleinsäuresequenzen zum Exprimieren der kurzen interferierenden Ribonukleinsäure (siRNA) eine RNA-Sinnstrangkodierungssequenz in funktionsbereiter Verbindung mit einer poly-T-Terminationssequenz unter Kontrolle eines menschlichen U6-RNA-Promotors und eine RNA-Gegensinnstrangkodierungssequenz in funktionsbereiter Verbindung mit einer poly-T-Terminationssequenz unter Kontrolle eines menschlichen U6-RNA-Promotors beinhalten.

21. Rekombinantes Plasmid gemäß Anspruch 18, wobei das Plasmid pAAV-kurze-interterierende-Ribonukleinsäure (pAAVsiRNA) ist.

22. Ein rekombinanter viraler Vektor, der Nukleinsäuresequenzen zum Exprimieren einer kurzen interferierenden Ribonukleinsäure (siRNA), die einen RNA-Sinnstrang und einen RNA-Gegensinnstrang beinhaltet, beinhaltet, wobei der RNA-Sinnstrang und der RNA-Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist.

23. Rekombinanter viraler Vektor gemäß Anspruch 22, wobei die Nukleinsäuresequenzen eine kurze interferierende Ribonukleinsäure (siRNA) exprimieren, wobei der RNA-Sinnstrang SEQ ID NO: 77 beinhaltet und der RNA-Gegensinnstrang SEQ ID NO: 78 beinhaltet.

24. Rekombinanter viraler Vektor gemäß Anspruch 22, wobei die Nukleinsäuresequenzen zum Exprimieren der kurzen interferierenden Ribonukleinsäure (siRNA) einen induzierbaren oder regulierbaren Promotor beinhalten.

25. Rekombinanter viraler Vektor gemäß Anspruch 22, wobei die Nukleinsäuresequenzen zum Exprimieren der kurzen interferierenden Ribonukleinsäure (siRNA) eine RNA-Sinnstrangkodierungssequenz in funktionsbereiter Verbindung mit einer poly-T-Terminationssequenz unter Kontrolle eines menschlichen U6-RNA-Promotors und eine RNA-Gegensinnstrangkodierungssequenz in funktionsbereiter Verbindung mit einer poly-T-Terminationssequenz unter Kontrolle eines menschlichen U6-RNA-Promotors beinhalten.

26. Rekombinanter viraler Vektor gemäß Anspruch 22, wobei der rekombinante virale Vektor aus der Gruppe, die aus einem adenoviralen Vektor, einem adeno-assoziierten viralen Vektor, einem lentiviralen Vektor und einem Herpesvirusvektor besteht, ausgewählt ist.

27. Rekombinanter viraler Vektor gemäß Anspruch 22, wobei der rekombinante virale Vektor mit Oberflächenproteinen von dem Vesicular-Stomatitis-Virus, Tollwutvirus, Ebolavirus oder Mokolavirus pseudotypisiert ist.

28. Rekombinanter viraler Vektor gemäß Anspruch 27, wobei der rekombinante virale Vektor einen adeno-assoziierten viralen Vektor beinhaltet.

29. Eine pharmazeutische Zusammensetzung, die eine wirksame Menge einer kurzen interferierenden Ribonukleinsäure (siRNA) und einen pharmazeutisch zulässigen Träger beinhaltet, wobei die siRNA einen RNA-Sinnstrang und einen RNA-Gegensinnstrang beinhaltet, wobei der RNA-Sinnstrang und der RNA-Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist.

30. Pharmazeutische Zusammensetzung gemäß Anspruch 29, wobei der RNA-Sinnstrang SEQ ID NO: 77 beinhaltet und der Gegensinnstrang SEQ ID NO: 78 beinhaltet.

31. Pharmazeutische Zusammensetzung gemäß Anspruch 29, die ferner Lipofectin, Lipofectamin, Cellfectin, Polykationen oder Liposomen beinhaltet.

32. Eine pharmazeutische Zusammensetzung, die das Plasmid gemäß Anspruch 17 oder ein physiologisch zulässiges Salz davon und einen pharmazeutisch zulässigen Träger beinhaltet.

33. Pharmazeutische Zusammensetzung gemäß Anspruch 32, die ferner Lipofectin, Lipofectamin, Cellfectin, Polykationen oder Liposomen beinhaltet.

34. Eine pharmazeutische Zusammensetzung, die den viralen Vektor gemäß Anspruch 22 und einen pharmazeutisch zulässigen Träger beinhaltet.

35. Eine wirksame Menge kurzer interferierender Ribonukleinsäure (siRNA), die einen RNA-Sinnstrang und einen RNA-Gegensinnstrang beinhaltet, wobei der RNA-Sinnstrang und der RNA-Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, zur Verwendung beim Inhibieren der Expression von mRNA eines menschlichen vaskulären endothelialen Wachstumsfaktors (VEGF) bei einem Patienten, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist.

36. Kurze interferierende Ribonukleinsäure (siRNA) gemäß Anspruch 35, wobei der RNA-Sinnstrang SEQ ID NO: 77 beinhaltet und der RNA-Gegensinnstrang SEQ ID NO: 78 beinhaltet.

37. Kurze interferierende Ribonukleinsäure (siRNA) gemäß Anspruch 35, wobei der Patient ein Mensch ist.

38. Kurze interferierende Ribonukleinsäure (siRNA) gemäß Anspruch 35, wobei die Expression von mRNA eines menschlichen vaskulären endothelialen Wachstumsfaktors (VEGF) in einem Auge oder in beiden Augen des Patienten inhibiert wird.

39. Kurze interferierende Ribonukleinsäure (siRNA) gemäß Anspruch 35, wobei die Expression von mRNA eines menschlichen vaskulären endothelialen Wachstumsfaktors in Zellen des retinalen Pigmentepithels des Patienten inhibiert wird.

40. Kurze interferierende Ribonukleinsäure (siRNA) gemäß Anspruch 35, wobei die wirksame Menge der kurzen interferierenden Ribonukleinsäure (siRNA) von etwa 1 nM bis zu etwa 100 n M beträgt.

41. Kurze interferierende Ribonukleinsäure (siRNA) gemäß Anspruch 35, wobei die kurze interferierende Ribonukleinsäure (siRNA) zusammen mit einem Übergabemittel verabreicht wird.

42. Kurze interferierende Ribonukleinsäure (siRNA) gemäß Anspruch 41, wobei das Übergabemittel aus der Gruppe, die aus Lipofectin, Lipofectamin, Cellfectin, Polykationen und Liposomen besteht, ausgewählt ist.

43. Kurze interferierende Ribonukleinsäure (siRNA) gemäß Anspruch 42, wobei das Übergabemittel ein Liposom ist.

44. Kurze interferierende Ribonukleinsäure (siRNA) gemäß Anspruch 43, wobei das Liposom einen Liganden, der das Liposom auf Zellen an oder nahe der Stelle der Angiogenese als Ziele festlegt, beinhaltet.

45. Kurze interferierende Ribonukleinsäure (siRNA) gemäß Anspruch 44, wobei sich der Ligand an Rezeptoren auf Tumorzellen oder Zellen des vaskulären Endothels bindet.

46. Kurze interferierende Ribonukleinsäure (siRNA) gemäß Anspruch 45, wobei der Ligand einen monoklonalen Antikörper beinhaltet.

47. Kurze interferierende Ribonukleinsäure (siRNA) gemäß Anspruch 44, wobei das Liposom mit einem Opsonisierungs-Inhibitionsteil modifiziert wird.

48. Kurze interferierende Ribonukleinsäure (siRNA) gemäß Anspruch 47, wobei der Opsonisierungs-Inhibitionsrest ein PEG, PPG oder Derivate davon beinhaltet.

49. Kurze interferierende Ribonukleinsäure (siRNA) gemäß Anspruch 36, wobei die kurze interferierende Ribonukleinsäure (siRNA) von einem rekombinanten Plasmid exprimiert wird.

50. Kurze interferierende Ribonukleinsäure (siRNA) gemäß Anspruch 36, wobei die kurze interferierende Ribonukleinsäure (siRNA) von einem rekombinanten viralen Vektor exprimiert wird.

51. Kurze interferierende Ribonukleinsäure (siRNA) gemäß Anspruch 50, wobei der rekombinante virale Vektor einen adenoviralen Vektor, einen adeno-assoziierten viralen Vektor, einen lentiviralen Vektor, einen retroviralen Vektor oder einen Herpesvirusvektor beinhaltet.

52. Kurze interferierende Ribonukleinsäure (siRNA) gemäß Anspruch 51, wobei der rekombinante virale Vektor mit Oberflächenproteinen von dem Vesicular-Stomatitis-Virus, Tollwutvirus, Ebolavirus oder Mokolavirus pseudotypisiert ist.

53. Kurze interferierende Ribonukleinsäure (siRNA) gemäß Anspruch 36, wobei die siRNA auf einem enteralen Verabreichungsweg verabreicht wird.

54. Kurze interferierende Ribonukleinsäure (siRNA) gemäß Anspruch 53, wobei der enterale Verabreichungsweg aus der Gruppe, die aus oral, rektal und intranasal besteht, ausgewählt ist.

55. Kurze interferierende Ribonukleinsäure (siRNA) gemäß Anspruch 36, wobei die kurze interferierende Ribonukleinsäure (siRNA) auf einem parenteralen Verabreichungsweg verabreicht wird.

56. Kurze interferierende Ribonukleinsäure (siRNA) gemäß Anspruch 55, wobei die parenterale Verabreichungsform aus der Gruppe, die aus intraokulärer Verabreichung, intravaskulärer Verabreichung, Injektion in und um Gewebe, subkutaner Injektion oder Ablagerung, subkutaner Infusion und Direktapplikation an oder nahe der Stelle einer Neovaskularisation besteht, ausgewählt ist.

57. Kurze interferierende Ribonukleinsäure (siRNA) gemäß Anspruch 56, wobei die intravaskuläre Verabreichung aus der Gruppe, die aus intravenöser Bolusinjektion, intravenöser Infusion, intraarterieller Bolusinjektion, intraarterieller Infusion und Einträufelung in die Blutgefäße durch Katheter besteht, ausgewählt ist.

58. Kurze interferierende Ribonukleinsäure (siRNA) gemäß Anspruch 56, wobei die Injektion in und um Gewebe peritumorale Injektion oder intratumorale Injektion beinhaltet.

59. Kurze interferierende Ribonukleinsäure (siRNA) gemäß Anspruch 56, wobei die intraokuläre Verbreichung intravitreale, intraretinale, subretinale Verabreichung, Subtenon-Verabreichung, peri- und retroorbitale, transkorneale oder transsklerale Verabreichung beinhaltet.

60. Kurze interferierende Ribonukleinsäure (siRNA) gemäß Anspruch 56, wobei die Direktapplikation an oder nahe der Stelle einer Neovaskularisation Applikation durch Katheter, korneale Pellets, Pipette, Zäpfchen, ein Implantat, das ein poröses Material beinhaltet, ein Implantat, das ein nicht poröses Material beinhaltet, oder ein Implantat, das ein gallertartiges Material beinhaltet, beinhaltet.

61. Kurze interferierende Ribonukleinsäure (siRNA) gemäß Anspruch 56, wobei sich die Stelle einer Neovaskularisation im Auge befindet und die Direktapplikation an oder nahe der Stelle einer Neovaskularisation Applikation durch ein Okularimplantat beinhaltet.

62. Kurze interferierende Ribonukleinsäure (siRNA) gemäß Anspruch 61, wobei das Okularimplantat biologisch abbaubar ist.

63. Eine wirksame Menge kurzer interferierender Ribonukleinsäure (siRNA), die einen RNA-Sinnstrang und einen RNA-Gegensinnstrang beinhaltet, wobei der RNA-Sinnstrang und der RNA-Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist, zur Verwendung bei der Prävention von Angiogenese.

64. Kurze interferierende Ribonukleinsäure (siRNA) gemäß Anspruch 63, wobei der RNA-Sinnstrang SEQ ID NO: 77 beinhaltet und der RNA-Gegensinnstrang SEQ ID NO: 78 beinhaltet.

65. Kurze interferierende Ribonukleinsäure (siRNA) gemäß Anspruch 63, wobei die Angiogenese pathogen ist.

66. Kurze interferierende Ribonukleinsäure (siRNA) gemäß Anspruch 63, wobei die Angiogenese nicht pathogen ist.

67. Kurze interferierende Ribonukleinsäure (siRNA) gemäß Anspruch 66, wobei die nicht pathogene Angiogenese mit der Erzeugung von Fettgeweben oder der Erzeugung von Cholesterin assoziiert wird.

68. Kurze interferierende Ribonukleinsäure (siRNA) gemäß Anspruch 66, wobei die nicht pathogene Angiogenese endometriale Neovaskularisation beinhaltet.

69. Kurze interferierende Ribonukleinsäure (siRNA) gemäß Anspruch 63, wobei die Angiogenese in einem Auge oder in beiden Augen des Patienten inhibiert wird.

70. Kurze interferierende Ribonukleinsäure (siRNA) gemäß Anspruch 63, wobei die Angiogenese in Zellen des retinalen Pigmentepithels des Patienten inhibiert wird.

71. Eine wirksame Menge kurzer interferierender Ribonukleinsäure (siRNA), die einen RNA-Sinnstrang und einen RNA-Gegensinnstrang beinhaltet, wobei der RNA-Sinnstrang und der RNA-Gegensinnstrang einen RNA-Doppelstrang bilden und wobei der RNA-Sinnstrang eine Nukleotidsequenz beinhaltet, die mit einer Zielsequenz in mRNA eines menschlichen vaskulären endothelialen Wachstumsfaktors (VEGF) identisch ist, wobei die Zielsequenz aus SEQ ID NO. 47, SEQ ID NO. 48, SEQ ID NO. 50, SEQ ID NO. 51, SEQ ID NO. 52 und SEQ ID NO. 56 ausgewählt ist, zur Verwendung bei der Behandlung angiogener Erkrankung, so dass die mit der angiogenen Erkrankung assoziierte Angiogenese inhibiert wird.

72. Kurze interferierende Ribonukleinsäure (siRNA) gemäß Anspruch 71, wobei der RNA-Sinnstrang SEQ ID NO: 77 beinhaltet und der RNA-Gegensinnstrang SEQ ID NO: 78 beinhaltet.

73. Kurze interferierende Ribonukleinsäure (siRNA) gemäß Anspruch 71, wobei die angiogene Erkrankung einen mit einem Krebs assoziierten Tumor beinhaltet.

74. Kurze interferierende Ribonukleinsäure (siRNA) gemäß Anspruch 73, wobei der Krebs aus der Gruppe, die aus Brustkrebs, Lungenkrebs, Kopf- und Halskrebs, Gehimkrebs, Unterleibskrebs, Darmkrebs, kolorektalem Karzinom, Speiseröhrenkrebs, Magen-Darm-Krebs, Gliom, Leberkrebs, Zungenkrebs, Neuroblastom, Osteosarkom, Eierstockkrebs, Bauchspeicheldrüsenkrebs, Prostatakrebs, Retinoblastom, Wilms-Tumor, multiplem Myelom, Hautkrebs, Lymphom, und Blutkrebs besteht, ausgewählt ist.

75. Kurze interferierende Ribonukleinsäure (siRNA) gemäß Anspruch 71, wobei die angiogene Erkrankung aus der Gruppe, die aus diabetischer Retinopathie, altersbedingter Makuladegeneration und entzündlichen Erkrankungen besteht, ausgewählt ist.

76. Kurze interferierende Ribonukleinsäure (siRNA) gemäß Anspruch 75, wobei die entzündliche Erkrankung Psoriasis oder rheumatoide Arthritis ist.

77. Kurze interferierende Ribonukleinsäure (siRNA) gemäß Anspruch 71, wobei die kurze interferierende Ribonukleinsäure (siRNA) in Kombination mit einem pharmazeutischen Wirkstoff zur Behandlung der angiogenen Erkrankung verabreicht wird, wobei sich der pharmazeutische Wirkstoff von der kurzen interferierenden Ribonukleinsäure (siRNA) unterscheidet.

78. Kurze interferierende Ribonukleinsäure (siRNA) gemäß Anspruch 77, wobei die angiogene Erkrankung Krebs ist und der pharmazeutische Wirkstoff einen chemotherapeutischen Wirkstoff beinhaltet.

79. Kurze interferierende Ribonukleinsäure (siRNA) gemäß Anspruch 78, wobei der chemotherapeutische Wirkstoff aus der Gruppe, die aus Cisplatin, Carboplatin, Cyclophosphamid, 5-Fluorouracil, Adriamycin, Daunorubicin und Tamoxifen besteht, ausgewählt ist.

80. Kurze interferierende Ribonukleinsäure (siRNA) gemäß Anspruch 71, wobei einem Patienten die kurze interferierende Ribonukleinsäure (siRNA) in Kombination mit einem anderen therapeutischen Verfahren, das zur Behandlung der angiogenen Erkrankung ausgerichtet ist, verabreicht wird.

81. Kurze interferierende Ribonukleinsäure (siRNA) gemäß Anspruch 80, wobei die angiogene Erkrankung Krebs ist und die kurze interferierende Ribonukleinsäure (siRNA) in Kombination mit Strahlungstherapie, Chemotherapie oder chirurgischem Eingriff verabreicht wird.

82. Kurze interferierende Ribonukleinsäure (siRNA) gemäß Anspruch 1, wobei die kurze interferierende Ribonukleinsäure (siRNA) ferner modifizierte RNA beinhaltet.

## Revendications

1. Un acide ribonucléique interférant court (ARNic) isolé comprenant un brin ARN sens d'environ 19 à environ 25 nucléotides de long et un brin ARN antisens d'environ 19 à environ 25 nucléotides de long, où les brins ARN sens et antisens forment un duplex ARN, et où le brin ARN sens comprend une séquence de nucléotides identique à une séquence cible de l'ARNm de facteur de croissance endothélial vasculaire humain (VEGF) où ladite séquence cible est sélectionnée parmi SEQ ID N° 47, SEQ ID N° 48, SEQ ID N° 50, SEQ ID N° 51, SEQ ID N° 52 et SEQ ID N° 56.

2. L'acide ribonucléique interférant court (ARNic) de la revendication 1, où l'ARNm de VEGF humain est sélectionné dans le groupe consistant en ARNm de VEGF₁₂₁ (SEQ ID N° : 2) ; ARNm de VEGF₁₆₅ (SEQ ID N° : 3) ; ARNm de VEGF₁₈₉ (SEQ ID N° : 4) et ARNm de VEGF₂₀₆ (SEQ ID N°: 5).

3. L'acide ribonucléique interférant court (ARNic) de la revendication 1, où le brin ARN sens comprend SEQ ID N°: 77, et le brin antisens comprend SEQ ID N°: 78.

4. L'acide ribonucléique interférant court (ARNic) de la revendication 1, où le brin ARN sens comprend une molécule ARN, et le brin ARN antisens comprend une molécule ARN.

5. L'acide ribonucléique interférant court (ARNic) de la revendication 1, où les brins ARN sens et antisens formant le duplex ARN sont liés de façon covalente par une épingle à cheveux à brin unique.

6. L'acide ribonucléique interférant court (ARNic) de la revendication 1, où l'ARNic comprend en outre du matériau non nucléotidique.

7. L'acide ribonucléique interférant court (ARNic) de la revendication 1, où les brins ARN sens et antisens sont stabilisés contre une dégradation par nucléase.

8. L'acide ribonucléique interférant court (ARNic) de la revendication 1, comprenant en outre un débordement en 3'.

9. L'acide ribonucléique interférant court (ARNic) de la revendication 8, où le débordement en 3' comprend de 1 à environ 6 nucléotides.

10. L'acide ribonucléique interférant court (ARNic) de la revendication 8, où le débordement en 3' comprend environ 2 nucléotides.

11. L'acide ribonucléique interférant court (ARNic) de la revendication 4, où le brin ARN sens comprend un premier débordement en 3', et le brin ARN antisens comprend un deuxième débordement en 3'.

12. L'acide ribonucléique interférant court (ARNic) de la revendication 11, où les premier et deuxième débordements en 3' comprennent séparément de 1 à environ 6 nucléotides.

13. L'acide ribonucléique interférant court (ARNic) de la revendication 12, où le premier débordement en 3' comprend un dinucléotide et le deuxième débordement en 3' comprend un dinucléotide.

14. L'acide ribonucléique interférant court (ARNic) de la revendication 13, où le dinucléotide comprenant le premier et le deuxième débordement en 3' est un acide dithymidylique (TT) ou un acide diuridylique (uu).

15. L'acide ribonucléique interférant court (ARNic) de la revendication 8, où le débordement en 3' est stabilisé contre une dégradation par nucléase.

16. Une cellule épithéliale pigmentaire rétinienne comprenant l'acide ribonucléique interférant court (ARNic) de la revendication 1.

17. Un plasmide recombiné comprenant des séquences d'acide nucléique pour exprimer un acide ribonucléique interférant court (ARNic) comprenant un brin ARN sens et un brin ARN antisens, où les brins ARN sens et antisens forment un duplex ARN, et où le brin ARN sens comprend une séquence nucléotidique identique à une séquence cible dans un ARNm de facteur de croissance endothélial vasculaire humain (VEGF) où ladite séquence cible est sélectionnée parmi SEQ ID N° 47, SEQ ID N° 48, SEQ ID N° 50, SEQ ID N° 51, SEQ ID N° 52 et SEQ ID N° 56.

18. Le plasmide recombiné de la revendication 17 où les séquences d'acide nucléique expriment un acide ribonucléique interférant court (ARNic) où le brin ARN sens comprend SEQ ID N° : 77, et le brin ARN antisens comprend SEQ ID N°: 78.

19. Le plasmide recombiné de la revendication 17, où les séquences d'acide nucléique pour exprimer l'acide ribonucléique interférant court (ARNic) comprennent un promoteur inductible ou régulable.

20. Le plasmide recombiné de la revendication 17, où les séquences d'acide nucléique pour exprimer l'acide ribonucléique interférant court (ARNic) comprennent une séquence codante de brin ARN sens en connexion exploitable avec une séquence de terminaison polyT sous le contrôle d'un promoteur ARN U6 humain, et une séquence codante de brin ARN antisens en connexion exploitable avec une séquence de terminaison polyT sous le contrôle d'un promoteur ARN U6 humain.

21. Le plasmide recombiné de la revendication 18, où le plasmide est l'acide ribonucléique interférant court pAAV (ARNic).

22. Un vecteur viral recombiné comprenant des séquences d'acide nucléique pour exprimer un acide ribonucléique interférant court (ARNic) comprenant un brin ARN sens et un brin ARN antisens, où les brins ARN sens et antisens forment un duplex ARN, et où le brin ARN sens comprend une séquence nucléotidique identique à une séquence cible dans de l'ARNm de facteur de croissance endothélial vasculaire humain (VEGF) où ladite séquence cible est sélectionnée parmi SEQ ID N° 47, SEQ ID N° 48, SEQ ID N° 50, SEQ ID N° 51, SEQ ID N° 52 et SEQ ID N° 56.

23. Le vecteur viral recombiné de la revendication 22 où les séquences d'acide nucléique expriment un acide ribonucléique interférant court (ARNic) où le brin ARN sens comprend SEQ ID N° : 77, et le brin ARN antisens comprend SEQ ID N° : 78.

24. Le vecteur viral recombiné de la revendication 22, où les séquences d'acide nucléique pour exprimer l'acide ribonucléique interférant court (ARNic) comprennent un promoteur inductible ou régulable.

25. Le vecteur viral recombiné de la revendication 22, où les séquences d'acide nucléique pour exprimer l'acide ribonucléique interférant court (ARNic) comprennent une séquence codante de brin ARN sens en connexion exploitable avec une séquence de terminaison polyT sous le contrôle d'un promoteur ARN U6 humain, et une séquence codante de brin ARN antisens en connexion exploitable avec une séquence de terminaison polyT sous le contrôle d'un promoteur ARN U6 humain.

26. Le vecteur viral recombiné de la revendication 22, où le vecteur viral recombiné est sélectionné dans le groupe consistant en un vecteur adénoviral, un vecteur viral adéno-associé, un vecteur lentiviral, un vecteur rétroviral, et un vecteur du virus de l'herpès.

27. Le vecteur viral recombiné de la revendication 22, où le vecteur viral recombiné est pseudotypé avec des protéines de surface provenant de virus de stomatite vésiculaire, virus rabique, virus d'Ebola, ou virus Mokola.

28. Le vecteur viral recombiné de la revendication 27, où le vecteur viral recombiné comprend un vecteur viral adéno-associé.

29. Une composition pharmaceutique comprenant une quantité efficace d'un acide ribonucléique interférant court (ARNic) et un support acceptable d'un point de vue pharmaceutique, où l'ARNic comprend un brin ARN sens et un brin ARN antisens, où les brins ARN sens et antisens forment un duplex ARN, et où le brin ARN sens comprend une séquence nucléotidique identique à une séquence cible dans de l'ARNm de facteur de croissance endothélial vasculaire humain (VEGF) où ladite séquence cible est sélectionnée parmi SEQ ID N° 47, SEQ ID N° 48, SEQ ID N° 50, SEQ ID N° 51, SEQ ID N° 52 et SEQ ID N° 56.

30. La composition pharmaceutique telle que revendiquée dans la revendication 29 où le brin ARN sens comprend SEQ ID N° : 77, et le brin ARN antisens comprend SEQ ID N° : 78.

31. La composition pharmaceutique de la revendication 29, comprenant en outre de la lipofectine, de la lipofectamine, de la cellfectine, des polycations, ou des liposomes.

32. Une composition pharmaceutique comprenant le plasmide de la revendication 17, ou un sel acceptable d'un point de vue physiologique de celui-ci, et un support acceptable d'un point de vue pharmaceutique.

33. La composition pharmaceutique de la revendication 32, comprenant en outre de la lipofectine, de la lipofectamine, de la cellfectine, des polycations, ou des liposomes.

34. Une composition pharmaceutique comprenant le vecteur viral de la revendication 22 et un support acceptable d'un point de vue pharmaceutique.

35. Une quantité efficace d'acide ribonucléique interférant court (ARNic) comprenant un brin ARN sens et un brin ARN antisens, où les brins ARN sens et antisens forment un duplex ARN, et où le brin ARN sens comprend une séquence nucléotidique identique à une séquence cible dans de l'ARNm de facteur de croissance endothélial vasculaire humain (VEGF) destiné à être utilisé pour inhiber l'expression du ARNm de facteur de croissance endothélial vasculaire humain (VEGF) chez un sujet où ladite séquence cible est sélectionnée parmi SEQ ID N° 47, SEQ ID N° 48, SEQ ID N° 50, SEQ ID N° 51, SEQ ID N° 52 et SEQ ID N° 56.

36. L'acide ribonucléique interférant court (ARNic) de la revendication 35 où le brin ARN sens comprend SEQ ID N° : 77, et le brin ARN antisens comprend SEQ ID N° : 78.

37. L'acide ribonucléique interférant court (ARNic) de la revendication 35, où le sujet est un être humain.

38. L'acide ribonucléique interférant court (ARNic) de la revendication 35, où l'expression d'ARNm de facteur de croissance endothélial vasculaire humain (VEGF) est inhibée dans un oeil ou dans les deux yeux du sujet.

39. L'acide ribonucléique interférant court (ARNic) de la revendication 35, où l'expression d'ARNm de facteur de croissance endothélial vasculaire humain (VEGF) est inhibée dans des cellules épithéliales pigmentaires rétiniennes du sujet.

40. L'acide ribonucléique interférant court (ARNic) de la revendication 35, où la quantité efficace de l'acide ribonucléique interférant court (ARNic) est d'environ 1 nM à environ 100 nM.

41. L'acide ribonucléique interférant court (ARNic) de la revendication 35, où l'acide ribonucléique interférant court (ARNic) est administré en conjonction avec un agent de délivrance.

42. L'acide ribonucléique interférant court (ARNic) de la revendication 41, où l'agent de délivrance est sélectionné dans le groupe consistant en lipofectine, lipofectamine, cellfectine, polycations, et liposomes.

43. L'acide ribonucléique interférant court (ARNic) de la revendication 42, où l'agent de délivrance est un liposome.

44. L'acide ribonucléique interférant court (ARNic) de la revendication 43, où le liposome comprend un ligand qui dirige le liposome vers des cellules au niveau ou à proximité du site d'angiogenèse.

45. L'acide ribonucléique interférant court (ARNic) de la revendication 44, où le ligand se lie à des récepteurs sur des cellules tumorales ou des cellules endothéliales vasculaires.

46. L'acide ribonucléique interférant court (ARNic) de la revendication 45, où le ligand comprend un anticorps monoclonal.

47. L'acide ribonucléique interférant court (ARNic) de la revendication 44, où le liposome est modifié avec une fraction opsonisation-inhibition.

48. L'acide ribonucléique interférant court (ARNic) de la revendication 47, où la fraction opsonisation-inhibition comprend un PEG, un PPG, ou des dérivés de ceux-ci.

49. L'acide ribonucléique interférant court (ARNic) de la revendication 36, où l'acide ribonucléique interférant court (ARNic) est exprimé à partir d'un plasmide recombiné.

50. L'acide ribonucléique interférant court (ARNic) de la revendication 36, où l'acide ribonucléique interférant court (ARNic) est exprimé à partir d'un vecteur viral recombiné.

51. L'acide ribonucléique interférant court (ARNic) de la revendication 50, où le vecteur viral recombiné comprend un vecteur adénoviral, un vecteur viral adéno-associé, un vecteur lentiviral, un vecteur rétroviral, ou un vecteur du virus de l'herpès.

52. L'acide ribonucléique interférant court (ARNic) de la revendication 51, où le vecteur viral recombiné est pseudotypé avec des protéines de surface provenant de virus stomatite vésiculaire, virus rabique, virus d'Ebola, ou virus Mokola.

53. L'acide ribonucléique interférant court (ARNic) de la revendication 36, où l'ARNic est administré par une voie d'administration entérale.

54. L'acide ribonucléique interférant court (ARNic) de la revendication 53, où la voie d'administration entérale est sélectionnée dans le groupe consistant en voie orale, rectale, et intranasale.

55. L'acide ribonucléique interférant court (ARNic) de la revendication 36, où l'acide ribonucléique interférant court (ARNic) est administré par une voie d'administration parentérale.

56. L'acide ribonucléique interférant court (ARNic) de la revendication 55, où la voie d'administration parentérale est sélectionnée dans le groupe consistant en administration intraoculaire, administration intravasculaire, injection péri- et intra-tissulaire, injection ou dépôt sous-cutanées, perfusion sous-cutanée, et application directe au niveau ou à proximité du site de néovascularisation.

57. L'acide ribonucléique interférant court (ARNic) de la revendication 56, où l'administration intravasculaire est sélectionnée dans le groupe consistant en injection bolus intraveineuse, perfusion intraveineuse, injection bolus intra-artérielle, perfusion intra-artérielle et instillation par sonde dans la vasculature.

58. L'acide ribonucléique interférant court (ARNic) de la revendication 56, où l'injection péri- et intra-tissulaire se compose d'injection péri-tumorale ou d'injection intratumorale.

59. L'acide ribonucléique interférant court (ARNic) de la revendication 56, où l'administration intraoculaire comprend une administration intravitréenne, intrarétinienne, sous-rétinienne, sous-Tenon, péri- et rétro-orbitaire, trans-cornéenne ou trans-sclérale.

60. L'acide ribonucléique interférant court (ARNic) de la revendication 56, où l'application directe au niveau ou à proximité du site de néovascularisation comprend une application par sonde, pastille cornéenne, compte-gouttes oculaire, suppositoire, un implant comprenant un matériau poreux, un implant comprenant un matériau non poreux, ou un implant comprenant un matériau gélatineux.

61. L'acide ribonucléique interférant court (ARNic) de la revendication 56, où le site de néovascularisation se trouve dans l'oeil, et l'application directe au niveau ou à proximité du site de néovascularisation comprend une application par un implant oculaire.

62. L'acide ribonucléique interférant court (ARNic) de la revendication 61, où l'implant oculaire est biodégradable.

63. Une quantité efficace d'acide ribonucléique interférant court (ARNic) comprenant un brin ARN sens et un brin ARN antisens, où les brins ARN sens et antisens forment un duplex ARN, et où le brin ARN sens comprend une séquence nucléotidique identique à une séquence cible dans de l'ARNm de facteur de croissance endothélial vasculaire humain (VEGF) où ladite séquence cible est sélectionnée parmi SEQ ID N° 47, SEQ ID N° 48, SEQ ID N° 50, SEQ ID N° 51, SEQ ID N° 52 et SEQ ID N° 56 afin d'être utilisée pour empêcher une angiogenèse.

64. L'acide ribonucléique interférant court (ARNic) de la revendication 63 où le brin ARN sens comprend SEQ ID N° : 77, et le brin ARN antisens comprend SEQ ID N° : 78.

65. L'acide ribonucléique interférant court (ARNic) de la revendication 63, où l'angiogenèse est pathogène.

66. L'acide ribonucléique interférant court (ARNic) de la revendication 63, où l'angiogenèse est non pathogène.

67. L'acide ribonucléique interférant court (ARNic) de la revendication 66, où l'angiogenèse non pathogène est associée à la production de tissus adipeux ou la production de cholestérol.

68. L'acide ribonucléique interférant court (ARNic) de la revendication 66, où l'angiogenèse non pathogène comprend une néovascularisation endométriale.

69. L'acide ribonucléique interférant court (ARNic) de la revendication 63, où l'angiogenèse est inhibée dans un oeil ou dans les deux yeux du sujet.

70. L'acide ribonucléique interférant court (ARNic) de la revendication 63, où l'angiogenèse est inhibée dans des cellules épithéliales pigmentaires rétiniennes du sujet.

71. Une quantité efficace d'acide ribonucléique interférant court (ARNic) comprenant un brin ARN sens et un brin ARN antisens, où les brins ARN sens et antisens forment un duplex ARN, et où le brin ARN sens comprend une séquence nucléotidique identique à une séquence cible dans de l'ARNm de facteur de croissance endothélial vasculaire humain (VEGF) où ladite séquence cible est sélectionnée parmi SEQ ID N° 47, SEQ ID N° 48, SEQ ID N° 50, SEQ ID N° 51, SEQ ID N° 52 et SEQ ID N° 56 afin d'être utilisée pour traiter une maladie angiogénique de telle sorte qu'une angiogenèse associée à une maladie angiogénique soit inhibée.

72. L'acide ribonucléique interférant court (ARNic) de la revendication 71 où le brin ARN sens comprend SEQ ID N° : 77, et le brin ARN antisens comprend SEQ ID N° : 78.

73. L'acide ribonucléique interférant court (ARNic) de la revendication 71, où la maladie angiogénique comprend une tumeur associée à un cancer.

74. L'acide ribonucléique interférant court (ARNic) de la revendication 73, où le cancer est sélectionné dans le groupe consistant en cancer du sein, cancer du poumon, cancer de la tête et du cou, cancer du cerveau, cancer abdominal, cancer du côlon, cancer colorectal, cancer de l'oesophage, cancer gastrointestinal, gliome, cancer du foie, cancer de la langue, neuroblastome, ostéosarcome, cancer ovarien, cancer du pancréas, cancer de la prostate, rétinoblastome, tumeur de Wilm, myélome multiple, cancer de la peau, lymphome, et cancer du sang.

75. L'acide ribonucléique interférant court (ARNic) de la revendication 71, où la maladie angiogénique est sélectionnée dans le groupe consistant en rétinopathie diabétique, dégénérescence maculaire liée à l'âge, et maladies inflammatoires.

76. L'acide ribonucléique interférant court (ARNic) de la revendication 75, où la maladie inflammatoire est du psoriasis ou de l'arthrite rhumatoïde.

77. L'acide ribonucléique interférant court (ARNic) de la revendication 71, où l'acide ribonucléique interférant court (ARNic) est administré en combinaison avec un agent pharmaceutique pour traiter la maladie angiogénique, lequel agent pharmaceutique est différent de l'acide ribonucléique interférant court (ARNic).

78. L'acide ribonucléique interférant court (ARNic) de la revendication 77 où la maladie angiogénique est un cancer, et l'agent pharmaceutique comprend un agent chimiothérapeutique.

79. L'acide ribonucléique interférant court (ARNic) de la revendication 78, où l'agent chimiothérapeutique est sélectionné dans le groupe consistant en cisplatine, carboplatine, cyclophosphamide, 5-fluorouracile, adriamycine, daunorubicine, et tamoxifène.

80. L'acide ribonucléique interférant court (ARNic) de la revendication 71, où l'acide ribonucléique interférant court (ARNic) est administré à un sujet en combinaison avec une autre méthode thérapeutique conçue pour traiter la maladie angiogénique.

81. L'acide ribonucléique interférant court (ARNic) de la revendication 80, où la maladie angiogénique est un cancer, et l'acide ribonucléique interférant court (ARNic) est administré en combinaison avec une radiothérapie, une chimiothérapie ou une intervention chirurgicale.

82. L'acide ribonucléique interférant court (ARNic) de la revendication 1, où l'acide ribonucléique interférant court (ARNic) comprend en outre un ARN modifié.


### SEQUENCE LISTING

<110> The Trustees of the University of Pennsylvania
   Tolentino, Michael J.
   Reich, Samuel Jotham
<120> Compositions and Methods for siRNA
   Inhibition of Angiogenesis
<130> 43826-1 PC
<150> US 60/398,417
   <151> 2002-07-24
<150> US 10/294,228
   <151> 2002-11-14
<160> 864
<170> FastSEQ for Windows Version 4.0
<210> 1
   <211> 2250
   <212> DNA
   <213> Mus musculus
<400> 1
   tgagccaggc tggcaggaag gagcctccct cagggtttcg ggaaccagac ctctcaccgg 60
   aaagaccgat taaccatgtc accaccacgc catcatcgtc accgttgaca gaacagtcct 120
   taatccagaa agcctgacat gaaggaagag gagactcttc gaggagcact ttgggtccgg 180
   agggcgagac tccggcagac gcattcccgg gcaggtgacc aagcacggtc cctcgtggga 240
   ctggattcgc cattttctta tatctgctgc taaatcgcca agcccggaag attagggttg 300
   tttctgggat tcctgtagac acacccaccc acatacacac atatatatat attatatata 360
   taaataaata tatatgtttt atatataaaa tatatatata ttcttttttt taaattaact 420
   ctgctaatgt tattggtgtc ttcactggat atgtttgact gctgtggact tgtgttggga 480
   ggaggatgtc ctcactcgga tgccgacatg ggagacaatg ggatgaaagg cttcagtgtg 540
   gtctgagaga ggccgaagtc cttttgcctg ccggggagca agcaaggcca gggcacgggg 600
   gcacattggc tcacttccag aaacacgaca aacccattcc tggccctgag tcaagaggac 660
   agagagacag atgatgacac agaaagagat aaagatgccg gttccaacca gaagtttggg 720
   gagcctcagg acatggcatg ctttgtggat ccccatgata gtctacaaaa gcaccccgcc 780
   cctctgggca ctgcctggaa gaatcgggag cctggccagc cttcagctcg ctcctccact 840
   tctgaggggc ctaggaggcc tcccacaggt gtcccggcaa gagaagacac ggtggtggaa 900
   gaagaggcct ggtaatggcc cctcctcctg ggaccccttc gtcctctcct taccccacct 960
   cctgggtaca gcccaggagg accttgtgtg atcagaccat tgaaaccact aattctgtcc 1020
   ccaggagact tggctctgtg tgtgagtggc ttacccttcc tcatcttccc ttcccaaggc 1080
   acagagcaat ggggcaggac ccgcaagccc ctcacggagg cagagaaaag agaaagtgtt 1140
   ttatatacgg tacttattta atagcccttt ttaattagaa attaaaacag ttaatttaat 1200
   taaagagtag ggtttttttc agtattcttg gttaatattt aatttcaact atttatgaga 1260
   tgtatctctc gctctctctt atttgtactt atgtgtgtgt gtgtgtgtgt gtgtgtgtgt 1320
   gtgtgtgtgt gtatgaaatc tgtgtttcca atctctctct cccagatcgg tgacagtcac 1380
   tagcttgtcc tgagaagata tttaattttg ctaacactca gctctgccct cccttgtccc 1440
   caccacacat tcctttgaaa taaggtttca atatacattt acatactata tatatatttg 1500
   gcaacttgtg tttgtatata aatatatata tatatatatg tttatgtata tatgtgattc 1560
   tgataaaata gacattgcta ttctgttttt tatatgtaaa aacaaaacaa gaaaaataga 1620
   gaattctaca tactaaatct ctctcctttt ttaattttaa tatttgttat catttattta 1680
   ttggtgctac tgtttatccg taataattgt gggggaaaaa gatattaaca tcacgtcttt 1740
   gtctctagag cagttttccg agatattccg tagtacatat ttatttttaa acagcaacaa 1800
   agaaatacag atatatctta aaaaaaaagc attttgtatt aaagaattga attctgatct 1860
   caaagctctc cctggtctct ccttctctcc tgggccctcc tgtctcgctt tccctcctcc 1920
   tttggggtac atagtttttg tcttaggttt gagaagcagt ccctggagta gaatatgggg 1980
   tgacccatcc attcctgggc ggaggggaga tggctccttt gccaagggtc ctcacactac 2040
   gtggtactct gttccttgtc agacaaggat gggggcatgt ctccaggtgc taactggaga 2100
   tcggagagag ctgttggctg cagctggcca ggatttgggc atgccgggga catgggaggc 2160
   tgtgagccca gcatgcagtt tacttctggg tgctaaatgg aagagtccag taaaaagagt 2220
   cttgcccatg ggattccatt ccgctttgtg                                  2250
<210> 2
   <211> 444
   <212> DNA
   <213> Homo sapiens
<400> 2
   atgaactttc tgctgtcttg ggtgcattgg agccttgcct tgctgctcta cctccaccat 60
   gccaagtggt cccaggctgc acccatggca gaaggaggag ggcagaatca tcacgaagtg 120
   gtgaagttca tggatgtcta tcagcgcagc tactgccatc caatcgagac cctggtggac 180
   atcttccagg agtaccctga tgagatcgag tacatcttca agccatcctg tgtgcccctg 240
   atgcgatgcg ggggctgctg caatgacgag ggcctggagt gtgtgcccac tgaggagtcc 300
   aacatcacca tgcagattat gcggatcaaa cctcaccaag gccagcacat aggagagatg 360
   agcttcctac agcacaacaa atgtgaatgc agaccaaaga aagatagagc aagacaagaa 420
   aaatgtgaca agccgaggcg gtga                                        444
<210> 3
   <211> 576
   <212> DNA
   <213> Homo sapiens
<400> 3
   atgaactttc tgctgtcttg ggtgcattgg agccttgcct tgctgctcta cctccaccat 60
   gccaagtggt cccaggctgc acccatggca gaaggaggag ggcagaatca tcacgaagtg 120
   gtgaagttca tggatgtcta tcagcgcagc tactgccatc caatcgagac cctggtggac 180
   atcttccagg agtaccctga tgagatcgag tacatcttca agccatcctg tgtgcccctg 240
   atgcgatgcg ggggctgctg caatgacgag ggcctggagt gtgtgcccac tgaggagtcc 300
   aacatcacca tgcagattat gcggatcaaa cctcaccaag gccagcacat aggagagatg 360
   agcttcctac agcacaacaa atgtgaatgc agaccaaaga aagatagagc aagacaagaa 420
   aatccctgtg ggccttgctc agagcggaga aagcatttgt ttgtacaaga tccgcagacg 480
   tgtaaatgtt cctgcaaaaa cacagactcg cgttgcaagg cgaggcagct tgagttaaac 540
   gaacgtactt gcagatgtga caagccgagg cggtga                            576
<210> 4
   <211> 648
   <212> DNA
   <213> Homo sapiens
<400> 4
   atgaactttc tgctgtcttg ggtgcattgg agccttgcct tgctgctcta cctccaccat 60
   gccaagtggt cccaggctgc acccatggca gaaggaggag ggcagaatca tcacgaagtg 120
   gtgaagttca tggatgtcta tcagcgcagc tactgccatc caatcgagac cctggtggac 180
   atcttccagg agtaccctga tgagatcgag tacatcttca agccatcctg tgtgcccctg 240
   atgcgatgcg ggggctgctg caatgacgag ggcctggagt gtgtgcccac tgaggagtcc 300
   aacatcacca tgcagattat gcggatcaaa cctcaccaag gccagcacat aggagagatg 360
   agcttcctac agcacaacaa atgtgaatgc agaccaaaga aagatagagc aagacaagaa 420
   aaaaaatcag ttcgaggaaa gggaaagggg caaaaacgaa agcgcaagaa atcccggtat 480
   aagtcctgga gcgttccctg tgggccttgc tcagagcgga gaaagcattt gtttgtacaa 540
   gatccgcaga cgtgtaaatg ttcctgcaaa aacacagact cgcgttgcaa ggcgaggcag 600
   cttgagttaa acgaacgtac ttgcagatgt gacaagccga ggcggtga                648
<210> 5
   <211> 670
   <212> DNA
   <213> Homo sapiens
<400> 5
   gccttgctgc tctacctcca ccatgccaag tggtcccagg ctgcacccat ggcagaagga 60
   ggagggcaga atcatcacga agtggtgaag ttcatggatg tctatcagcg cagctactgc 120
   catccaatcg agaccctggt ggacatcttc caggagtacc ctgatgagat cgagtacatc 180
   ttcaagccat cctgtgtgcc cctgatgcga tgcgggggct gctgcaatga cgagggcctg 240
   gagtgtgtgc ccactgagga gtccaacatc accatgcaga ttatgcggat caaacctcac 300
   caaggccagc acataggaga gatgagcttc ctacagcaca acaaatgtga atgcagacca 360
   aagaaggata gagcaagaca agaaaaaaaa tcagttcgag gaaagggaaa ggggcaaaaa 420
   cgaaagcgca agaaatcccg gtataagtcc tggagcgttt acgttggtgc ccgctgctgt 480
   ctaatgccct ggagcctccc tggcccccat ccctgtgggc cttgctcaga gcggagaaag 540
   catttgtttg tacaagatcc gcagacgtgt aaatgttcct gcaaaaacac agactcgcgt 600
   tgcaaggcga ggcagcttga gttaaacgaa cgtacttgca gatgtgacaa gccgaggcgg 660
   tgatgaatga                                                        670
<210> 6
   <211> 1137
   <212> DNA
   <213> Homo sapiens
<400> 6
   atgctcattg tccagactgg ggtcagatca gcaaacaaag ggcctctgat ggtgattgtt 60
   gaatattgca aatatggaaa tctatccaac tacctcaaga gcaaatatga cttatttttt 120
   ctcgacaagg atgtggcatc acacatggag cgtaagaaag aaaaaatgga gccaggcctg 180
   gaacaaggca agaaaccaaa actagatagc atcaccagca gcgagagctt tgggagctcc 240
   aagtttcagg aagataaaaa tctgagtgat gttgaggaag aggaggattc tgatggtttc 300
   taccaggagc ccatcactat ggaagatctg atttcttaca gttttcaagt ggccagaggc 360
   atgaagtttc tgtcttccag aaagtgcatt cattgggacc tggcagcaag aaacattctt 420
   ttatctgaga acaatgtggt gaagatttgt gattttggcc ttgcccagga tatttacaag 480
   aacgccgatt atgtgagaaa aggaggtggg tctccatacc caggagtgca aatggatgag 540
   cacttctgca gttgcctgag ggaaggcatg aggatgagag ctgctgagta ctccactcct 600
   gaaatctatc agatcatgct ggactgcagg cacaaagacc caaaagaaag gccaagattt 660
   gcagaacttg tggaaaaact agaaaatagt gggtttacat actcaactcc tgccttctct 720
   gaggacttct tcaaggaagg tatttcagct cccaagttta gttcaggaag ctctgatgat 780
   gtcagatacg taaatgcttt caagttcatg agcctggaaa gaatcaaaac ctttgaagaa 840
   cttttgccaa atgccacctc catgtttgat gactaccagg gggacagcag cgctctgctg 900
   gcctctccca tgctgaagcg cttcaccagg actgacagca aacccaaggc ctcgctcaag 960
   attgacttga gactaactag caaaagtaag aagtcggggc tttctgatgt cagcaggccc 1020
   agtttctgcc attccaacag tgggcacatc agcaaaggca agggcaggtt cacctacgac 1080
   aacgccgagc tggaaaggaa gacggcgtgc tgctccccgc ccctctggga gttgtag    1137
<210> 7
   <211> 5830
   <212> DNA
   <213> Homo sapiens
<400> 7
   actgagtccc gggaccccgg gagagcggtc agtgtgtggt cgctgcgttt cctctgcctg 60
   cgccgggcat cacttgcgcg ccgcagaaag tccgtctggc agcctggata tcctctccta 120
   ccggcacccg cagacgcccc tgcagccgcc ggtcggcgcc cgggctccct agccctgtgc 180
   gctcaactgt cctgcgctgc ggggtgccgc gagttccacc tccgcgcctc cttctctaga 240
   caggcgctgg gagaaagaac cggctcccga gttctgggca tttcgcccgg ctcgaggtgc 300
   aggatgcaga gcaaggtgct gctggccgtc gccctgtggc tctgcgtgga gacccgggcc 360
   gcctctgtgg gtttgcctag tgtttctctt gatctgccca ggctcagcat acaaaaagac 420
   atacttacaa ttaaggctaa tacaactctt caaattactt gcaggggaca gagggacttg 480
   gactggcttt ggcccaataa tcagagtggc agtgagcaaa gggtggaggt gactgagtgc 540
   agcgatggcc tcttctgtaa gacactcaca attccaaaag tgatcggaaa tgacactgga 600
   gcctacaagt gcttctaccg ggaaactgac ttggcctcgg tcatttatgt ctatgttcaa 660
   gattacagat ctccatttat tgcttctgtt agtgaccaac atggagtcgt gtacattact 720
   gagaacaaaa acaaaactgt ggtgattcca tgtctcgggt ccatttcaaa tctcaacgtg 780
   tcactttgtg caagataccc agaaaagaga tttgttcctg atggtaacag aatttcctgg 840
   gacagcaaga agggctttac tattcccagc tacatgatca gctatgctgg catggtcttc 900
   tgtgaagcaa aaattaatga tgaaagttac cagtctatta tgtacatagt tgtcgttgta 960
   gggtatagga tttatgatgt ggttctgagt ccgtctcatg gaattgaact atctgttgga 1020
   gaaaagcttg tcttaaattg tacagcaaga actgaactaa atgtggggat tgacttcaac 1080
   tgggaatacc cttcttcgaa gcatcagcat aagaaacttg taaaccgaga cctaaaaacc 1140
   cagtctggga gtgagatgaa gaaatttttg agcaccttaa ctatagatgg tgtaacccgg 1200
   agtgaccaag gattgtacac ctgtgcagca tccagtgggc tgatgaccaa gaagaacagc 1260
   acatttgtca gggtccatga aaaacctttt gttgcttttg gaagtggcat ggaatctctg 1320
   gtggaagcca cggtggggga gcgtgtcaga atccctgcga agtaccttgg ttacccaccc 1380
   ccagaaataa aatggtataa aaatggaata ccccttgagt ccaatcacac aattaaagcg 1440
   gggcatgtac tgacgattat ggaagtgagt gaaagagaca caggaaatta cactgtcatc 1500
   cttaccaatc ccatttcaaa ggagaagcag agccatgtgg tctctctggt tgtgtatgtc 1560
   ccaccccaga ttggtgagaa atctctaatc tctcctgtgg attcctacca gtacggcacc 1620
   actcaaacgc tgacatgtac ggtctatgcc attcctcccc cgcatcacat ccactggtat 1680
   tggcagttgg aggaagagtg cgccaacgag cccagccaag ctgtctcagt gacaaaccca 1740
   tacccttgtg aagaatggag aagtgtggag gacttccagg gaggaaataa aattgaagtt 1800
   aataaaaatc aatttgctct aattgaagga aaaaacaaaa ctgtaagtac ccttgttatc 1860
   caagcggcaa atgtgtcagc tttgtacaaa tgtgaagcgg tcaacaaagt cgggagagga 1920
   gagagggtga tctccttcca cgtgaccagg ggtcctgaaa ttactttgca acctgacatg 1980
   cagcccactg agcaggagag cgtgtctttg tggtgcactg cagacagatc tacgtttgag 2040
   aacctcacat ggtacaagct tggcccacag cctctgccaa tccatgtggg agagttgccc 2100
   acacctgttt gcaagaactt ggatactctt tggaaattga atgccaccat gttctctaat 2160
   agcacaaatg acattttgat catggagctt aagaatgcat ccttgcagga ccaaggagac 2220
   tatgtctgcc ttgctcaaga caggaagacc aagaaaagac attgcgtggt caggcagctc 2280
   acagtcctag agcgtgtggc acccacgatc acaggaaacc tggagaatca gacgacaagt 2340
   attggggaaa gcatcgaagt ctcatgcacg gcatctggga atccccctcc acagatcatg 2400
   tggtttaaag ataatgagac ccttgtagaa gactcaggca ttgtattgaa ggatgggaac 2460
   cggaacctca ctatccgcag agtgaggaag gaggacgaag gcctctacac ctgccaggca 2520
   tgcagtgttc ttggctgtgc aaaagtggag gcatttttca taatagaagg tgcccaggaa 2580
   aagacgaact tggaaatcat tattctagta ggcacggcgg tgattgccat gttcttctgg 2640
   ctacttcttg tcatcatcct acggaccgtt aagcgggcca atggagggga actgaagaca 2700
   ggctacttgt ccatcgtcat ggatccagat gaactcccat tggatgaaca ttgtgaacga 2760
   ctgccttatg atgccagcaa atgggaattc cccagagacc ggctgaagct aggtaagcct 2820
   cttggccgtg gtgcctttgg ccaagtgatt gaagcagatg cctttggaat tgacaagaca 2880
   gcaacttgca ggacagtagc agtcaaaatg ttgaaagaag gagcaacaca cagtgagcat 2940
   cgagctctca tgtctgaact caagatcctc attcatattg gtcaccatct caatgtggtc 3000
   aaccttctag gtgcctgtac caagccagga gggccactca tggtgattgt ggaattctgc 3060
   aaatttggaa acctgtccac ttacctgagg agcaagagaa atgaatttgt cccctacaag 3120
   accaaagggg cacgattccg tcaagggaaa gactacgttg gagcaatccc tgtggatctg 3180
   aaacggcgct tggacagcat caccagtagc cagagctcag ccagctctgg atttgtggag 3240
   gagaagtccc tcagtgatgt agaagaagag gaagctcctg aagatctgta taaggacttc 3300
   ctgaccttgg agcatctcat ctgttacagc ttccaagtgg ctaagggcat ggagttcttg 3360
   gcatcgcgaa agtgtatcca cagggacctg gcggcacgaa atatcctctt atcggagaag 3420
   aacgtggtta aaatctgtga ctttggcttg gcccgggata tttataaaga tccagattat 3480
   gtcagaaaag gagatgctcg cctccctttg aaatggatgg ccccagaaac aatttttgac 3540
   agagtgtaca caatccagag tgacgtctgg tcttttggtg ttttgctgtg ggaaatattt 3600
   tccttaggtg cttctccata tcctggggta aagattgatg aagaattttg taggcgattg 3660
   aaagaaggaa ctagaatgag ggcccctgat tatactacac cagaaatgta ccagaccatg 3720
   ctggactgct ggcacgggga gcccagtcag agacccacgt tttcagagtt ggtggaacat 3780
   ttgggaaatc tcttgcaagc taatgctcag caggatggca aagactacat tgttcttccg 3840
   atatcagaga ctttgagcat ggaagaggat tctggactct ctctgcctac ctcacctgtt 3900
   tcctgtatgg aggaggagga agtatgtgac cccaaattcc attatgacaa cacagcagga 3960
   atcagtcagt atctgcagaa cagtaagcga aagagccggc ctgtgagtgt aaaaacattt 4020
   gaagatatcc cgttagaaga accagaagta aaagtaatcc cagatgacaa ccagacggac 4080
   agtggtatgg ttcttgcctc agaagagctg aaaactttgg aagacagaac caaattatct 4140
   ccatcttttg gtggaatggt gcccagcaaa agcagggagt ctgtggcatc tgaaggctca 4200
   aaccagacaa gcggctacca gtccggatat cactccgatg acacagacac caccgtgtac 4260
   tccagtgagg aagcagaact tttaaagctg atagagattg gagtgcaaac cggtagcaca 4320
   gcccagattc tccagcctga ctcggggacc acactgagct ctcctcctgt ttaaaaggaa 4380
   gcatccacac cccaactccc ggacatcaca tgagaggtct gctcagattt tgaagtgttg 4440
   ttctttccac cagcaggaag tagccgcatt tgattttcat ttcgacaaca gaaaaaggac 4500
   ctcggactgc agggagccag tcttctaggc atatcctgga agaggcttgt gacccaagaa 4560
   tgtgtctgtg tcttctccca gtgttgacct gatcctcttt tttcattcat ttaaaaagca 4620
   ttatcatgcc cctgctgcgg gtctcaccat gggtttagaa caaagagctt caagcaatgg 4680
   ccccatcctc aaagaagtag cagtacctgg ggagctgaca cttctgtaaa actagaagat 4740
   aaaccaggca acgtaagtgt tcgaggtgtt gaagatggga aggatttgca gggctgagtc 4800
   tatccaagag gctttgttta ggacgtgggt cccaagccaa gccttaagtg tggaattcgg 4860
   attgatagaa aggaagacta acgttacctt gctttggaga gtactggagc ctgcaaatgc 4920
   attgtgtttg ctctggtgga ggtgggcatg gggtctgttc tgaaatgtaa agggttcaga 4980
   cggggtttct ggttttagaa ggttgcgtgt tcttcgagtt gggctaaagt agagttcgtt 5040
   gtgctgtttc tgactcctaa tgagagttcc ttccagaccg ttagctgtct ccttgccaag 5100
   ccccaggaag aaaatgatgc agctctggct ccttgtctcc caggctgatc ctttattcag 5160
   aataccacaa agaaaggaca ttcagctcaa ggctccctgc cgtgttgaag agttctgact 5220
   gcacaaacca gcttctggtt tcttctggaa tgaataccct catatctgtc ctgatgtgat 5280
   atgtctgaga ctgaatgcgg gaggttcaat gtgaagctgt gtgtggtgtc aaagtttcag 5340
   gaaggatttt acccttttgt tcttccccct gtccccaacc cactctcacc ccgcaaccca 5400
   tcagtatttt agttatttgg cctctactcc agtaaacctg attgggtttg ttcactctct 5460
   gaatgattat tagccagact tcaaaattat tttatagccc aaattataac atctattgta 5520
   ttatttagac ttttaacata tagagctatt tctactgatt tttgcccttg ttctgtcctt 5580
   tttttcaaaa aagaaaatgt gttttttgtt tggtaccata gtgtgaaatg ctgggaacaa 5640
   tgactataag acatgctatg gcacatatat ttatagtctg tttatgtaga aacaaatgta 5700
   atatattaaa gccttatata taatgaactt tgtactattc acattttgta tcagtattat 5760
   gtagcataac aaaggtcata atgctttcag caattgatgt cattttatta aagaacattg 5820
   aaaaacttga                                                        5830
<210> 8
   <211> 19
   <212> DNA
   <213> Artificial Sequence
<220>
   <223> Targeting sequence
<400> 8
   tcatcacgaa gtggtgaag 19
<210> 9
   <211> 21
   <212> RNA
   <213> Artificial Sequence
<220>
   <223> Targeting sequence
<400> 9
   ucaucacgaa guggugaagu u 21
<210> 10
   <211> 21
   <212> RNA
   <213> Artificial Sequence
<220>
   <223> Targeting sequence
<400> 10
   cuucaccacu ucgugaugau u 21
<210> 11
   <211> 21
   <212> DNA
   <213> Artificial Sequence
<220>
   <223> Targeting sequence
<221> misc_RNA
   <222> (1) ... (19)
   <223> ribonucleotide
<221> misc feature
   <222> (20) ... (21)
   <223> deoxyribonucleotide
<400> 11
   ucaucacgaa guggugaagt t 21
<210> 12
   <211> 21
   <212> DNA
   <213> Artificial Sequence
<220>
   <223> Targeting sequence
<221> misc_RNA
   <222> (1) ... (19)
   <223> ribonucleotides
<221> misc feature
   <222> (20) ... (21)
   <223> deoxyribonucleotides
<400> 12
   cuucaccacu ucgugaugat t 21
<210> 13
   <211> 21
   <212> DNA
   <213> Artificial Sequence
<220>
   <223> Targeting sequence
<400> 13
   aacgtacttg cagatgtgac a 21
<210> 14
   <211> 19
   <212> DNA
   <213> Artificial Sequence
<220>
   <223> Targeting sequence
<400> 14
   gttcatggat gtctatcag 19
<210> 15
   <211> 19
   <212> DNA
   <213> Artificial Sequence
<220>
   <223> Targeting sequence
<400> 15
   tcgagaccct ggtggacat 19
<210> 16
   <211> 19
   <212> DNA
   <213> Artificial Sequence
<220>
   <223> Targeting sequence
<400> 16
   tgacgagggc ctggagtgt 19
<210> 17
   <211> 19
   <212> DNA
   <213> Artificial Sequence
<220>
   <223> Targeting sequence
<400> 17
   tgacgagggc ctggagtgt 19
<210> 18
   <211> 19
   <212> DNA
   <213> Artificial Sequence
<220>
   <223> Targeting sequence
<400> 18
   catcaccatg cagattatg 19
<210> 19
   <211> 19
   <212> DNA
   <213> Artificial Sequence
<220>
   <223> Targeting sequence
<400> 19
   acctcaccaa ggccagcac 19
<210> 20
   <211> 19
   <212> DNA
   <213> Artificial Sequence
<220>
   <223> Targeting sequence
<400> 20
   ggccagcaca taggagaga 19
<210> 21
   <211> 19
   <212> DNA
   <213> Artificial Sequence
<220>
   <223> Targeting sequence
<400> 21
   caaatgtgaa tgcagacca 19
<210> 22
   <211> 19
   <212> DNA
   <213> Artificial Sequence
<220>
   <223> Targeting sequence
<400> 22
   atgtgaatgc agaccaaag 19
<210> 23
   <211> 19
   <212> DNA
   <213> Artificial Sequence
<220>
   <223> Targeting sequence
<400> 23
   tgcagaccaa agaaagata 19
<210> 24
   <211> 19
   <212> DNA
   <213> Artificial Sequence
<220>
   <223> Targeting sequence
<400> 24
   agaaagatag agcaagaca 19
<210> 25
   <211> 19
   <212> DNA
   <213> Artificial Sequence
<220>
   <223> Targeting sequence
<400> 25
   gaaagataga gcaagacaa 19
<210> 26
   <211> 19
   <212> DNA
   <213> Artificial Sequence
<220>
   <223> Targeting sequence
<400> 26
   gatagagcaa gacaagaaa 19
<210> 27
   <211> 19
   <212> DNA
   <213> Artificial Sequence
<220>
   <223> Targeting sequence
<400> 27
   gacaagaaaa tccctgtgg 19
<210> 28
   <211> 19
   <212> DNA
   <213> Artificial Sequence
<220>
   <223> Targeting sequence
<400> 28
   gaaaatccct gtgggcctt 19
<210> 29
   <211> 19
   <212> DNA
   <213> Artificial Sequence
<220>
   <223> Targeting sequence
<400> 29
   aatccctgtg ggccttgct 19
<210> 30
   <211> 19
   <212> DNA
   <213> Artificial Sequence
<220>
   <223> Targeting sequence
<400> 30
   tccctgtggg ccttgctca 19
<210> 31
   <211> 19
   <212> DNA
   <213> Artificial Sequence
<220>
   <223> Targeting sequence
<400> 31
   gcatttgttt gtacaagat 19
<210> 32
   <211> 19
   <212> DNA
   <213> Artificial Sequence
<220>
   <223> Targeting sequence
<400> 32 19
   gatccgcaga cgtgtaaat 19
<210> 33
   <211> 19
   <212> DNA
   <213> Artificial Sequence
<220>
   <223> Targeting sequence
<400> 33 19
   atgttcctgc aaaaacaca 19
<210> 34
   <211> 19
   <212> DNA
   <213> Artificial Sequence
<220>
   <223> Targeting sequence
<400> 34 19
   tgttcctgca aaaacacag 19
<210> 35
   <211> 19
   <212> DNA
   <213> Artificial Sequence
<220>
   <223> Targeting sequence
<400> 35 19
   aaacacagac tcgcgttgc 19
<210> 36
   <211> 19
   <212> DNA
   <213> Artificial Sequence
<220>
   <223> Targeting sequence
<400> 36 19
   aacacagact cgcgttgca 19
<210> 37
   <211> 19
   <212> DNA
   <213> Artificial Sequence
<220>
   <223> Targeting sequence
<400> 37
   acacagactc gcgttgcaa 19
<210> 38
   <211> 19
   <212> DNA
   <213> Artificial Sequence
<220>
   <223> Targeting sequence
<400> 38
   cacagactcg cgttgcaag 19
<210> 39
   <211> 19
   <212> DNA
   <213> Artificial Sequence
<220>
   <223> Targeting sequence
<400> 39
   ggcgaggcag cttgagtta 19
<210> 40
   <211> 19
   <212> DNA
   <213> Artificial Sequence
<220>
   <223> Targeting sequence
<400> 40
   acgaacgtac ttgcagatg 19
<210> 41
   <211> 19
   <212> DNA
   <213> Artificial Sequence
<220>
   <223> Targeting sequence
<400> 41
   cgaacgtact tgcagatgt 19
<210> 42
   <211> 19
   <212> DNA
   <213> Artificial Sequence
<220>
   <223> Targeting sequence
<400> 42
   cgtacttgca gatgtgaca 19
<210> 43
   <211> 19
   <212> DNA
   <213> Artificial Sequence
<220>
   <223> Targeting sequence
<400> 43
   gtggtcccag gctgcaccc 19
<210> 44
   <211> 19
   <212> DNA
   <213> Artificial Sequence
<220>
   <223> Targeting sequence
<400> 44
   ggaggagggc agaatcatc 19
<210> 45
   <211> 19
   <212> DNA
   <213> Artificial Sequence
<220>
   <223> Targeting sequence
<400> 45
   gtggtgaagt tcatggatg 19
<210> 46
   <211> 21
   <212> DNA
   <213> Artificial Sequence
<220>
   <223> Targeting sequence
<400> 46
   aatcatcacg aagtggtgaa g 21
<210> 47
   <211> 21
   <212> DNA
   <213> Artificial Sequence
<220>
   <223> Targeting sequence
<400> 47
   aagttcatgg atgtctatca g 21
<210> 48
   <211> 21
   <212> DNA
   <213> Artificial Sequence
<220>
   <223> Targeting sequence
<400> 48
   aatcgagacc ctggtggaca t 21
<210> 49
   <211> 21
   <212> DNA
   <213> Artificial Sequence
<220>
   <223> Targeting sequence
<400> 49 21
   aatgacgagg gcctggagtg t 21
<210> 50
   <211> 21
   <212> DNA
   <213> Artificial Sequence

<220>
   <223> Targeting sequence
<400> 50 21
   aacatcacca tgcagattat g 21
<210> 51
   <211> 21
   <212> DNA
   <213> Artificial Sequence
<220>
   <223> Targeting sequence
<400> 51 21
   aaacctcacc aaggccagca c 21
<210> 52
   <211> 21
   <212> DNA
   <213> Artificial Sequence
<220>
   <223> Targeting sequence
<400> 52 21
   aaggccagca cataggagag a 21
<210> 53
   <211> 21
   <212> DNA
   <213> Artificial Sequence
<220>
   <223> Targeting sequence
<400> 53 21
   aacaaatgtg aatgcagacc a 21
<210> 54
   <211> 21
   <212> DNA
   <213> Artificial Sequence
<220>
   <223> Targeting sequence
<400> 54
   aaatgtgaat gcagaccaaa g 21
<210> 55
   <211> 21
   <212> DNA
   <213> Artificial Sequence
<220>
   <223> Targeting sequence
<400> 55
   aatgcagacc aaagaaagat a 21
<210> 56
   <211> 21
   <212> DNA
   <213> Artificial Sequence
<220>
   <223> Targeting sequence
<400> 56
   aaagaaagat agagcaagac a 21
<210> 57
   <211> 21
   <212> DNA
   <213> Artificial Sequence
<220>
   <223> Targeting sequence
<400> 57
   aagaaagata gagcaagaca a 21
<210> 58
   <211> 23
   <212> DNA
   <213> Artificial Sequence
<220>
   <223> Targeting sequence
<400> 58
   aagatagagc aagacaagaa aat 23
<210> 59
   <211> 23
   <212> DNA
   <213> Artificial Sequence
<220>
   <223> Targeting sequence
<400> 59
   aagacaagaa aatccctgtg ggc 23
<210> 60
   <211> 23
   <212> DNA
   <213> Artificial Sequence
<220>
   <223> Targeting sequence
<400> 60
   aagaaaatcc ctgtgggcct tgc 23
<210> 61
   <211> 23
   <212> DNA
   <213> Artificial Sequence
<220>
   <223> Targeting sequence
<400> 61
   aatccctgtg ggccttgctc aga 23
<210> 62
   <211> 23
   <212> DNA
   <213> Artificial Sequence
<220>
   <223> Targeting sequence
<400> 62
   aagcatttgt ttgtacaaga tcc 23
<210> 63
   <211> 23
   <212> DNA
   <213> Artificial Sequence
<220>
   <223> Targeting sequence
<400> 63
   aagatccgca gacgtgtaaa tgt 23
<210> 64
   <211> 23
   <212> DNA
   <213> Artificial Sequence
<220>
   <223> Targeting sequence
<400> 64
   aaatgttcct gcaaaaacac aga 23
<210> 65
   <211> 23
   <212> DNA
   <213> Artificial Sequence
<220>
   <223> Targeting sequence
<400> 65
   aatgttcctg caaaaacaca gac 23
<210> 66
   <211> 23
   <212> DNA
   <213> Artificial Sequence
<220>
   <223> Targeting sequence
<400> 66
   aaaaacacag actcgcgttg caa 23
<210> 67
   <211> 23
   <212> DNA
   <213> Artificial Sequence
<220>
   <223> Targeting sequence
<400> 67
   aaaacacaga ctcgcgttgc aag 23
<210> 68
   <211> 23
   <212> DNA
   <213> Artificial Sequence
<220>
   <223> Targeting sequence
<400> 68
   aaacacagac tcgcgttgca agg 23
<210> 69
   <211> 23
   <212> DNA
   <213> Artificial Sequence
<220>
   <223> Targeting sequence
<400> 69
   aacacagact cgcgttgcaa ggc 23
<210> 70
   <211> 23
   <212> DNA
   <213> Artificial Sequence
<220>
   <223> Targeting sequence
<400> 70
   aaggcgaggc agcttgagtt aaa 23
<210> 71
   <211> 23
   <212> DNA
   <213> Artificial Sequence
<220>
   <223> Targeting sequence
<400> 71
   aaacgaacgt acttgcagat gtg 23
<210> 72
   <211> 23
   <212> DNA
   <213> Artificial Sequence
<220>
   <223> Targeting sequence
<400> 72
   aacgaacgta cttgcagatg tga 23
<210> 73
   <211> 23
   <212> DNA
   <213> Artificial Sequence
<220>
   <223> Targeting sequence
<400> 73
   aagtggtccc aggctgcacc cat 23
<210> 74
   <211> 23
   <212> DNA
   <213> Artificial Sequence
<220>
   <223> Targeting sequence
<400> 74
   aaggaggagg gcagaatcat cac 23
<210> 75
   <211> 23
   <212> DNA
   <213> Artificial Sequence
<220>
   <223> Targeting sequence
<400> 75
   aagtggtgaa gttcatggat gtc 23
<210> 76
   <211> 23
   <212> DNA
   <213> Artificial Sequence
<220>
   <223> Targeting sequence
<400> 76
   aaaatccctg tgggccttgc tca 23
<210> 77
   <211> 21
   <212> DNA
   <213> Artificial Sequence
<220>
   <223> Targeting sequence
<221> misc_RNA
   <222> (1)...(19)
   <223> ribonucleotides
<221> misc feature
   <222> (20) ... (21)
   <223> deoxyribonucleotides
<400> 77
   accucaccaa ggccagcact t 21
<210> 78
   <211> 21
   <212> DNA
   <213> Artificial Sequence
<220>
   <223> Targeting sequence
<221> misc_RNA
   <222> (1)...(19)
   <223> ribonucleotides
<221> misc_feature
   <222> (20) ... (21)
   <223> deoxyribonucleotides
<400> 78
   gugcuggccu uggugaggut t 21
<210> 79
   <211> 18
   <212> DNA
   <213> Artificial Sequence
<220>
   <223> Targeting sequence
<400> 79
   ggctacgtcc agcgcacc 18
<210> 80
   <211> 21
   <212> DNA
   <213> Artificial Sequence
<220>
   <223> Targeting sequence
<400> 80
   aaaccucacc aaagccagca c 21
<210> 81
   <211> 21
   <212> DNA
   <213> Artificial Sequence
<220>
   <223> Targeting sequence
<400> 81
   ggcagaatca tcacgaagtg g 21
<210> 82
   <211> 21
   <212> DNA
   <213> Artificial Sequence
<220>
   <223> Targeting sequence
<400> 82
   cctggtggac atcttccagg a 21
<210> 83
   <211> 21
   <212> DNA
   <213> Artificial Sequence
<220>
   <223> Targeting sequence
<400> 83
   gagatcgagt acatcttcaa g 21
<210> 84
   <211> 21
   <212> DNA
   <213> Artificial Sequence
<220>
   <223> Targeting sequence
<400> 84
   tggagtgtgt gcccactgag g 21
<210> 85
   <211> 21
   <212> DNA
   <213> Artificial Sequence
<220>
   <223> Targeting sequence
<400> 85
   gagcttccta cagcacaaca a 21
<210> 86
   <211> 21
   <212> DNA
   <213> Artificial Sequence
<220>
   <223> Targeting sequence
<400> 86
   ttgctcagag cggagaaagc a 21
<210> 87
   <211> 21
   <212> DNA
   <213> Artificial Sequence
<220>
   <223> Targeting sequence
<400> 87
   cacacactcg cgttgcaagg c 21
<210> 88
   <211> 21
   <212> DNA
   <213> Artificial Sequence
<220>
   <223> Targeting sequence
<400> 88
   tcaccatgca gattatgcgg a 21
<210> 89
   <211> 21
   <212> DNA
   <213> Artificial Sequence
<220>
   <223> Targeting sequence
<400> 89
   tagagcaaga caagaaaatc c 21
<210> 90
   <211> 21
   <212> DNA
   <213> Artificial Sequence
<220>
   <223> Targeting sequence
<400> 90
   ccgcagacgt gtaaatgttc c 21
<210> 91
   <211> 21
   <212> DNA
   <213> Artificial Sequence
<220>
   <223> Targeting sequence
<400> 91
   aagcaggcca gacactgcat c 21
<210> 92
   <211> 21
   <212> DNA
   <213> Artificial Sequence
<220>
   <223> Targeting sequence
<400> 92
   aatgcagggg ggaagcagcc c 21
<210> 93
   <211> 21
   <212> DNA
   <213> Artificial Sequence
<220>
   <223> Targeting sequence
<400> 93
   aagcagccca taaatggtct t 21
<210> 94
   <211> 21
   <212> DNA
   <213> Artificial Sequence
<220>
   <223> Targeting sequence
<400> 94
   aaatggtctt tgcctgaaat g 21
<210> 95
   <211> 21
   <212> DNA
   <213> Artificial Sequence
<220>
   <223> Targeting sequence
<400> 95
   aatggtcttt gcctgaaatg g 21
<210> 96
   <211> 21
   <212> DNA
   <213> Artificial Sequence
<220>
   <223> Targeting sequence
<400> 96
   aaatggtgag taaggaaagc g 21
<210> 97
   <211> 21
   <212> DNA
   <213> Artificial Sequence
<220>
   <223> Targeting sequence
<400> 97
   aatggtgagt aaggaaagcg a 21
<210> 98
   <211> 21
   <212> DNA
   <213> Artificial Sequence
<220>
   <223> Targeting sequence
<400> 98
   aaggaaagcg aaaggctgag c 21
<210> 99
   <211> 21
   <212> DNA
   <213> Artificial Sequence
<220>
   <223> Targeting sequence
<400> 99
   aaagcgaaag gctgagcata a 21
<210> 100
   <211> 21
   <212> DNA
   <213> Artificial Sequence
<220>
   <223> Targeting sequence
<400> 100
   aagcgaaagg ctgagcataa c 21
<210> 101
   <211> 21
   <212> DNA
   <213> Artificial Sequence
<220>
   <223> Targeting sequence
<400> 101
   aaaggctgag cataactaaa t 21
<210> 102
   <211> 21
   <212> DNA
   <213> Artificial Sequence
<220>
   <223> Targeting sequence
<400> 102
   aaggctgagc ataactaaat c 21
<210> 103
   <211> 21
   <212> DNA
   <213> Artificial Sequence
<220>
   <223> Targeting sequence
<400> 103
   aactaaatct gcctgtggaa g 21
<210> 104
   <211> 21
   <212> DNA
   <213> Artificial Sequence
<220>
   <223> Targeting sequence
<400> 104
   aaatctgcct gtggaagaaa t 21
<210> 105
   <211> 21
   <212> DNA
   <213> Artificial Sequence
<220>
   <223> Targeting sequence
<400> 105
   aatctgcctg tggaagaaat g 21
<210> 106
   <211> 21
   <212> DNA
   <213> Artificial Sequence
<220>
   <223> Targeting sequence
<400> 106
   aagaaatggc aaacaattct g 21
<210> 107
   <211> 21
   <212> DNA
   <213> Artificial Sequence
<220>
   <223> Targeting sequence
<400> 107
   aaatggcaaa caattctgca g 21
<210> 108
   <211> 21
   <212> DNA
   <213> Artificial Sequence
<220>
   <223> Targeting sequence
<400> 108
   aatggcaaac aattctgcag t 21
<210> 109
   <211> 21
   <212> DNA
   <213> Artificial Sequence
<220>
   <223> Targeting sequence
<400> 109
   aaacaattct gcagtacttt a 21
<210> 110
   <211> 21
   <212> DNA
   <213> Artificial Sequence
<220>
   <223> Targeting sequence
<400> 110
   aacaattctg cagtacttta a 21
<210> 111
   <211> 21
   <212> DNA
   <213> Artificial Sequence
<220>
   <223> Targeting sequence
<400> 111
   aattctgcag tactttaacc t 21
<210> 112
   <211> 21
   <212> DNA
   <213> Artificial Sequence
<220>
   <223> Targeting sequence
<400> 112
   aaccttgaac acagctcaag c 21
<210> 113
   <211> 21
   <212> DNA
   <213> Artificial Sequence
<220>
   <223> Targeting sequence
<400> 113
   aacacagctc aagcaaacca c 21
<210> 114
   <211> 21
   <212> DNA
   <213> Artificial Sequence
<220>
   <223> Targeting sequence
<400> 114
   aagcaaacca cactggcttc t 21
<210> 115
   <211> 21
   <212> DNA
   <213> Artificial Sequence
<220>
   <223> Targeting sequence
<400> 115
   aaaccacact ggcttctaca g 21
<210> 116
   <211> 21
   <212> DNA
   <213> Artificial Sequence
<220>
   <223> Targeting sequence
<400> 116
   aaccacactg gcttctacag c 21
<210> 117
   <211> 21
   <212> DNA
   <213> Artificial Sequence
<220>
   <223> Targeting sequence
<400> 117
   aaatatctag ctgtacctac t 21
<210> 118
   <211> 21
   <212> DNA
   <213> Artificial Sequence
<220>
   <223> Targeting sequence
<400> 118
   aatatctagc tgtacctact t 21
<210> 119
   <211> 21
   <212> DNA
   <213> Artificial Sequence
<220>
   <223> Targeting sequence
<400> 119
   aaagaagaag gaaacagaat c 21
<210> 120
   <211> 21
   <212> DNA
   <213> Artificial Sequence
<220>
   <223> Targeting sequence
<400> 120
   aagaagaagg aaacagaatc t 21
<210> 121
   <211> 21
   <212> DNA
   <213> Artificial Sequence
<220>
   <223> Targeting sequence
<400> 121
   aagaaggaaa cagaatctgc a 21
<210> 122
   <211> 21
   <212> DNA
   <213> Artificial Sequence
<220>
   <223> Targeting sequence
<400> 122
   aaggaaacag aatctgcaat c 21
<210> 123
   <211> 21
   <212> DNA
   <213> Artificial Sequence
<220>
   <223> Targeting sequence
<400> 123
   aaacagaatc tgcaatctat a 21
<210> 124
   <211> 21
   <212> DNA
   <213> Artificial Sequence
<220>
   <223> Targeting sequence
<400> 124
   aacagaatct gcaatctata t 21
<210> 125
   <211> 21
   <212> DNA
   <213> Artificial Sequence
<220>
   <223> Targeting sequence
<400> 125
   aatctgcaat ctatatattt a 21
<210> 126
   <211> 21
   <212> DNA
   <213> Artificial Sequence
<220>
   <223> Targeting sequence
<400> 126
   aatctatata tttattagtg a 21
<210> 127
   <211> 21
   <212> DNA
   <213> Artificial Sequence
<220>
   <223> Targeting sequence
<400> 127
   aaatccccga aattatacac a 21
<210> 128
   <211> 21
   <212> DNA
   <213> Artificial Sequence
<220>
   <223> Targeting sequence
<400> 128
   aatccccgaa attatacaca t 21
<210> 129
   <211> 21
   <212> DNA
   <213> Artificial Sequence
<220>
   <223> Targeting sequence
<400> 129
   aaattataca catgactgaa g 21
<210> 130
   <211> 21
   <212> DNA
   <213> Artificial Sequence
<220>
   <223> Targeting sequence
<400> 130
   aattatacac atgactgaag g 21
<210> 131
   <211> 21
   <212> DNA
   <213> Artificial Sequence
<220>
   <223> Targeting sequence
<400> 131
   aaggaaggga gctcgtcatt c 21
<210> 132
   <211> 21
   <212> DNA
   <213> Artificial Sequence
<220>
   <223> Targeting sequence
<400> 132
   aagggagctc gtcattccct g 21
<210> 133
   <211> 21
   <212> DNA
   <213> Artificial Sequence
<220>
   <223> Targeting sequence
<400> 133
   aacatcactg ttactttaaa a 21
<210> 134
   <211> 21
   <212> DNA
   <213> Artificial Sequence
<220>
   <223> Targeting sequence
<400> 134
   aaaaaagttt ccacttgaca c 21
<210> 135
   <211> 21
   <212> DNA
   <213> Artificial Sequence
<220>
   <223> Targeting sequence
<400> 135 21
   aaaaagtttc cacttgacac t 21
<210> 136
   <211> 21
   <212> DNA
   <213> Artificial Sequence
<220>
   <223> Targeting sequence
<400> 136
   aaaagtttcc acttgacact t 21
<210> 137
   <211> 21
   <212> DNA
   <213> Artificial Sequence
<220>
   <223> Targeting sequence
<400> 137
   aaagtttcca cttgacactt t 21
<210> 138
   <211> 21
   <212> DNA
   <213> Artificial Sequence
<220>
   <223> Targeting sequence
<400> 138
   aagtttccac ttgacacttt g 21
<210> 139
   <211> 21
   <212> DNA
   <213> Artificial Sequence
<220>
   <223> Targeting sequence
<400> 139
   aaaacgcata atctgggaca g 21
<210> 140
   <211> 21
   <212> DNA
   <213> Artificial Sequence
<220>
   <223> Targeting sequence
<400> 140
   aaacgcataa tctgggacag t 21
<210> 141
   <211> 21
   <212> DNA
   <213> Artificial Sequence
<220>
   <223> Targeting sequence
<400> 141
   aacgcataat ctgggacagt a 21
<210> 142
   <211> 21
   <212> DNA
   <213> Artificial Sequence
<220>
   <223> Targeting sequence
<400> 142
   aatctgggac agtagaaagg g 21
<210> 143
   <211> 21
   <212> DNA
   <213> Artificial Sequence
<220>
   <223> Targeting sequence
<400> 143
   aaagggcttc atcatatcaa a 21
<210> 144
   <211> 21
   <212> DNA
   <213> Artificial Sequence
<220>
   <223> Targeting sequence
<400> 144
   aagggcttca tcatatcaaa t 21
<210> 145
   <211> 21
   <212> DNA
   <213> Artificial Sequence
<220>
   <223> Targeting sequence
<400> 145
   aaatgcaacg tacaaagaaa t 21
<210> 146
   <211> 21
   <212> DNA
   <213> Artificial Sequence
<220>
   <223> Targeting sequence
<400> 146
   aatgcaacgt acaaagaaat a 21
<210> 147
   <211> 21
   <212> DNA
   <213> Artificial Sequence
<220>
   <223> Targeting sequence

<400> 147 21
   aacgtacaaa gaaatagggc t 21
<210> 148
   <211> 21
   <212> DNA
   <213> Artificial Sequence
<220>
   <223> Targeting sequence
<400> 148 21
   aaagaaatag ggcttctgac c 21
<210> 149
   <211> 21
   <212> DNA
   <213> Artificial Sequence
<220>
   <223> Targeting sequence
<400> 149 21
   aagaaatagg gcttctgacc t 21
<210> 150
   <211> 21
   <212> DNA
   <213> Artificial Sequence
<220>
   <223> Targeting sequence
<400> 150 21
   aaatagggct tctgacctgt g 21
<210> 151
   <211> 21
   <212> DNA
   <213> Artificial Sequence
<220>
   <223> Targeting sequence
<400> 151 21
   aatagggctt ctgacctgtg a 21
<210> 152
   <211> 21
   <212> DNA
   <213> Artificial Sequence
<220>
   <223> Targeting sequence
<400> 152
   aagcaacagt caatgggcat t 21
<210> 153
   <211> 21
   <212> DNA
   <213> Artificial Sequence
<220>
   <223> Targeting sequence
<400> 153
   aacagtcaat gggcatttgt a 21
<210> 154
   <211> 21
   <212> DNA
   <213> Artificial Sequence
<220>
   <223> Targeting sequence
<400> 154
   aatgggcatt tgtataagac a 21
<210> 155
   <211> 21
   <212> DNA
   <213> Artificial Sequence
<220>
   <223> Targeting sequence
<400> 155
   aagacaaact atctcacaca t 21
<210> 156
   <211> 21
   <212> DNA
   <213> Artificial Sequence
<220>
   <223> Targeting sequence
<400> 156
   aaactatctc acacatcgac a 21
<210> 157
   <211> 21
   <212> DNA
   <213> Artificial Sequence
<220>
   <223> Targeting sequence
<400> 157
   aactatctca cacatcgaca a 21
<210> 158
   <211> 21
   <212> DNA
   <213> Artificial Sequence
<220>
   <223> Targeting sequence
<400> 158
   aaaccaatac aatcatagat g 21
<210> 159
   <211> 21
   <212> DNA
   <213> Artificial Sequence
<220>
   <223> Targeting sequence
<400> 159
   aaccaataca atcatagatg t 21
<210> 160
   <211> 21
   <212> DNA
   <213> Artificial Sequence
<220>
   <223> Targeting sequence
<400> 160
   aatacaatca tagatgtcca a 21
<210> 161
   <211> 21
   <212> DNA
   <213> Artificial Sequence
<220>
   <223> Targeting sequence
<400> 161
   aatcatagat gtccaaataa g 21
<210> 162
   <211> 21
   <212> DNA
   <213> Artificial Sequence
<220>
   <223> Targeting sequence
<400> 162
   aaataagcac accacgccca g 21
<210> 163
   <211> 21
   <212> DNA
   <213> Artificial Sequence
<220>
   <223> Targeting sequence
<400> 163
   aataagcaca ccacgcccag t 21
<210> 164
   <211> 21
   <212> DNA
   <213> Artificial Sequence
<220>
   <223> Targeting sequence
<400> 164
   aagcacacca cgcccagtca a 21
<210> 165
   <211> 21
   <212> DNA
   <213> Artificial Sequence
<220>
   <223> Targeting sequence
<400> 165
   aaattactta gaggccatac t 21
<210> 166
   <211> 21
   <212> DNA
   <213> Artificial Sequence
<220>
   <223> Targeting sequence
<400> 166
   aattacttag aggccatact c 21
<210> 167
   <211> 21
   <212> DNA
   <213> Artificial Sequence
<220>
   <223> Targeting sequence
<400> 167
   aattgtactg ctaccactcc c 21
<210> 168
   <211> 21
   <212> DNA
   <213> Artificial Sequence
<220>
   <223> Targeting sequence
<400> 168
   aacacgagag ttcaaatgac c 21
<210> 169
   <211> 21
   <212> DNA
   <213> Artificial Sequence
<220>
   <223> Targeting sequence
<400> 169
   aaatgacctg gagttaccct g 21
<210> 170
   <211> 21
   <212> DNA
   <213> Artificial Sequence
<220>
   <223> Targeting sequence
<400> 170
   aatgacctgg agttaccctg a 21
<210> 171
   <211> 21
   <212> DNA
   <213> Artificial Sequence
<220>
   <223> Targeting sequence
<400> 171
   aaaaaaataa gagagcttcc g 21
<210> 172
   <211> 21
   <212> DNA
   <213> Artificial Sequence
<220>
   <223> Targeting sequence
<400> 172
   aaaaaataag agagcttccg t 21
<210> 173
   <211> 21
   <212> DNA
   <213> Artificial Sequence
<220>
   <223> Targeting sequence
<400> 173
   aaaaataaga gagcttccgt a 21
<210> 174
   <211> 21
   <212> DNA
   <213> Artificial Sequence
<220>
   <223> Targeting sequence
<400> 174
   aaaataagag agcttccgta a 21
<210> 175
   <211> 21
   <212> DNA
   <213> Artificial Sequence
<220>
   <223> Targeting sequence
<400> 175
   aaataagaga gcttccgtaa g 21
<210> 176
   <211> 21
   <212> DNA
   <213> Artificial Sequence
<220>
   <223> Targeting sequence
<400> 176
   aataagagag cttccgtaag g 21
<210> 177
   <211> 21
   <212> DNA
   <213> Artificial Sequence
<220>
   <223> Targeting sequence
<400> 177
   aagagagctt ccgtaaggcg a 21
<210> 178
   <211> 21
   <212> DNA
   <213> Artificial Sequence
<220>
   <223> Targeting sequence
<400> 178
   aaggcgacga attgaccaaa g 21
<210> 179
   <211> 21
   <212> DNA
   <213> Artificial Sequence
<220>
   <223> Targeting sequence
<400> 179
   aattgaccaa agcaattccc a 21
<210> 180
   <211> 21
   <212> DNA
   <213> Artificial Sequence
<220>
   <223> Targeting sequence
<400> 180
   aaagcaattc ccatgccaac a 21
<210> 181
   <211> 21
   <212> DNA
   <213> Artificial Sequence
<220>
   <223> Targeting sequence
<400> 181
   aagcaattcc catgccaaca t 21
<210> 182
   <211> 21
   <212> DNA
   <213> Artificial Sequence
<220>
   <223> Targeting sequence
<400> 182
   aattcccatg ccaacatatt c 21
<210> 183
   <211> 21
   <212> DNA
   <213> Artificial Sequence
<220>
   <223> Targeting sequence
<400> 183
   aacatattct acagtgttct t 21
<210> 184
   <211> 21
   <212> DNA
   <213> Artificial Sequence
<220>
   <223> Targeting sequence
<400> 184
   aaaatgcaga acaaagacaa a 21
<210> 185
   <211> 21
   <212> DNA
   <213> Artificial Sequence
<220>
   <223> Targeting sequence
<400> 185
   aatgcagaac aaagacaaag g 21
<210> 186
   <211> 21
   <212> DNA
   <213> Artificial Sequence
<220>
   <223> Targeting sequence
<400> 186
   aacaaagaca aaggacttta t 21
<210> 187
   <211> 21
   <212> DNA
   <213> Artificial Sequence
<220>
   <223> Targeting sequence
<400> 187
   aaagacaaag gactttatac t 21
<210> 188
   <211> 21
   <212> DNA
   <213> Artificial Sequence
<220>
   <223> Targeting sequence
<400> 188
   aagacaaagg actttatact t 21
<210> 189
   <211> 21
   <212> DNA
   <213> Artificial Sequence
<220>
   <223> Targeting sequence
<400> 189
   aaaggacttt atacttgtcg t 21
<210> 190
   <211> 21
   <212> DNA
   <213> Artificial Sequence
<220>
   <223> Targeting sequence
<400> 190
   aaggacttta tacttgtcgt g 21
<210> 191
   <211> 21
   <212> DNA
   <213> Artificial Sequence
<220>
   <223> Targeting sequence
<400> 191
   aaggagtgga ccatcattca a 21
<210> 192
   <211> 21
   <212> DNA
   <213> Artificial Sequence
<220>
   <223> Targeting sequence
<400> 192
   aaatctgtta acacctcagt g 21
<210> 193
   <211> 21
   <212> DNA
   <213> Artificial Sequence
<220>
   <223> Targeting sequence
<400> 193
   aatctgttaa cacctcagtg c 21
<210> 194
   <211> 21
   <212> DNA
   <213> Artificial Sequence
<220>
   <223> Targeting sequence
<400> 194
   aacacctcag tgcatatata t 21
<210> 195
   <211> 21
   <212> DNA
   <213> Artificial Sequence
<220>
   <223> Targeting sequence
<400> 195
   aaagcattca tcactgtgaa a 21
<210> 196
   <211> 21
   <212> DNA
   <213> Artificial Sequence
<220>
   <223> Targeting sequence
<400> 196
   aagcattcat cactgtgaaa c 21
<210> 197
   <211> 21
   <212> DNA
   <213> Artificial Sequence
<220>
   <223> Targeting sequence
<400> 197
   aaacatcgaa aacagcaggt g 21
<210> 198
   <211> 21
   <212> DNA
   <213> Artificial Sequence
<220>
   <223> Targeting sequence
<400> 198
   aacatcgaaa acagcaggtg c 21
<210> 199
   <211> 21
   <212> DNA
   <213> Artificial Sequence
<220>
   <223> Targeting sequence
<400> 199
   aaaacagcag gtgcttgaaa c 21
<210> 200
   <211> 21
   <212> DNA
   <213> Artificial Sequence
<220>
   <223> Targeting sequence
<400> 200
   aaacagcagg tgcttgaaac c 21
<210> 201
   <211> 21
   <212> DNA
   <213> Artificial Sequence
<220>
   <223> Targeting sequence
<400> 201
   aacagcaggt gcttgaaacc g 21
<210> 202
   <211> 21
   <212> DNA
   <213> Artificial Sequence
<220>
   <223> Targeting sequence
<400> 202
   aaaccgtagc tggcaagcgg t 21
<210> 203
   <211> 21
   <212> DNA
   <213> Artificial Sequence
<220>
   <223> Targeting sequence
<400> 203
   aaccgtagct ggcaagcggt c 21
<210> 204
   <211> 21
   <212> DNA
   <213> Artificial Sequence
<220>
   <223> Targeting sequence
<400> 204
   aaccgtagct ggcaagcggt c 21
<210> 205
   <211> 21
   <212> DNA
   <213> Artificial Sequence
<220>
   <223> Targeting sequence
<400> 205
   aaagtgaagg catttccctc g 21
<210> 206
   <211> 21
   <212> DNA
   <213> Artificial Sequence
<220>
   <223> Targeting sequence
<400> 206
   aagtgaaggc atttccctcg c 21
<210> 207
   <211> 21
   <212> DNA
   <213> Artificial Sequence
<220>
   <223> Targeting sequence
<400> 207
   aaggcatttc cctcgccgga a 21
<210> 208
   <211> 21
   <212> DNA
   <213> Artificial Sequence
<220>
   <223> Targeting sequence
<400> 208
   aagttgtatg gttaaaagat g 21
<210> 209
   <211> 21
   <212> DNA
   <213> Artificial Sequence
<220>
   <223> Targeting sequence
<400> 209
   aaaagatggg ttacctgcga c 21
<210> 210
   <211> 21
   <212> DNA
   <213> Artificial Sequence
<220>
   <223> Targeting sequence
<400> 210
   aaagatgggt tacctgcgac t 21
<210> 211
   <211> 21
   <212> DNA
   <213> Artificial Sequence
<220>
   <223> Targeting sequence
<400> 211
   aagatgggtt acctgcgact g 21
<210> 212
   <211> 21
   <212> DNA
   <213> Artificial Sequence
<220>
   <223> Targeting sequence
<400> 212
   aaatctgctc gctatttgac t 21
<210> 213
   <211> 21
   <212> DNA
   <213> Artificial Sequence
<220>
   <223> Targeting sequence
<400> 213
   aatctgctcg ctatttgact c 21
<210> 214
   <211> 21
   <212> DNA
   <213> Artificial Sequence
<220>
   <223> Targeting sequence
<400> 214
   aattatcaag gacgtaactg a 21
<210> 215
   <211> 21
   <212> DNA
   <213> Artificial Sequence
<220>
   <223> Targeting sequence
<400> 215
   aaggacgtaa ctgaagagga t 21
<210> 216
   <211> 21
   <212> DNA
   <213> Artificial Sequence
<220>
   <223> Targeting sequence
<400> 216
   aactgaagag gatgcaggga a 21
<210> 217
   <211> 21
   <212> DNA
   <213> Artificial Sequence
<220>
   <223> Targeting sequence
<400> 217
   aagaggatgc agggaattat a 21
<210> 218
   <211> 21
   <212> DNA
   <213> Artificial Sequence
<220>
   <223> Targeting sequence
<400> 218
   aattatacaa tcttgctgag c 21
<210> 219
   <211> 21
   <212> DNA
   <213> Artificial Sequence
<220>
   <223> Targeting sequence
<400> 219
   aatcttgctg agcataaaac a 21
<210> 220
   <211> 21
   <212> DNA
   <213> Artificial Sequence
<220>
   <223> Targeting sequence
<400> 220
   aaaacagtca aatgtgttta a 21
<210> 221
   <211> 21
   <212> DNA
   <213> Artificial Sequence
<220>
   <223> Targeting sequence
<400> 221
   aaacagtcaa atgtgtttaa a 21
<210> 222
   <211> 21
   <212> DNA
   <213> Artificial Sequence
<220>
   <223> Targeting sequence
<400> 222
   aacagtcaaa tgtgtttaaa a 21
<210> 223
   <211> 21
   <212> DNA
   <213> Artificial Sequence
<220>
   <223> Targeting sequence
<400> 223
   aaatgtgttt aaaaacctca c 21
<210> 224
   <211> 21
   <212> DNA
   <213> Artificial Sequence
<220>
   <223> Targeting sequence
<400> 224
   aatgtgttta aaaacctcac t 21
<210> 225
   <211> 21
   <212> DNA
   <213> Artificial Sequence
<220>
   <223> Targeting sequence
<400> 225
   aaaaacctca ctgccactct a 21
<210> 226
   <211> 21
   <212> DNA
   <213> Artificial Sequence
<220>
   <223> Targeting sequence
<400> 226
   aaaacctcac tgccactcta a 21
<210> 227
   <211> 21
   <212> DNA
   <213> Artificial Sequence
<220>
   <223> Targeting sequence
<400> 227
   aaacctcact gccactctaa t 21
<210> 228
   <211> 21
   <212> DNA
   <213> Artificial Sequence
<220>
   <223> Targeting sequence
<400> 228
   aacctcactg ccactctaat t 21
<210> 229
   <211> 21
   <212> DNA
   <213> Artificial Sequence

<220>
   <223> Targeting sequence
<400> 229
   aattgtcaat gtgaaacccc a 21
<210> 230
   <211> 21
   <212> DNA
   <213> Artificial Sequence
<220>
   <223> Targeting sequence
<400> 230
   aatgtgaaac cccagattta c 21
<210> 231
   <211> 21
   <212> DNA
   <213> Artificial Sequence
<220>
   <223> Targeting sequence
<400> 231
   aaaccccaga tttacgaaaa g 21
<210> 232
   <211> 21
   <212> DNA
   <213> Artificial Sequence
<220>
   <223> Targeting sequence
<400> 232
   aaccccagat ttacgaaaag g 21
<210> 233
   <211> 21
   <212> DNA
   <213> Artificial Sequence
<220>
   <223> Targeting sequence
<400> 233
   aaaaggccgt gtcatcgttt c 21
<210> 234
   <211> 21
   <212> DNA
   <213> Artificial Sequence
<220>
   <223> Targeting sequence
<400> 234
   aaaggccgtg tcatcgtttc c 21
<210> 235
   <211> 21
   <212> DNA
   <213> Artificial Sequence
<220>
   <223> Targeting sequence
<400> 235
   aaggccgtgt catcgtttcc a 21
<210> 236
   <211> 21
   <212> DNA
   <213> Artificial Sequence
<220>
   <223> Targeting sequence
<400> 236
   aaatcctgac ttgtaccgca t 21
<210> 237
   <211> 21
   <212> DNA
   <213> Artificial Sequence
<220>
   <223> Targeting sequence
<400> 237
   aatcctgact tgtaccgcat a 21
<210> 238
   <211> 21
   <212> DNA
   <213> Artificial Sequence
<220>
   <223> Targeting sequence
<400> 238
   aacctacaat caagtggttc t 21
<210> 239
   <211> 21
   <212> DNA
   <213> Artificial Sequence
<220>
   <223> Targeting sequence
<400> 239
   aatcaagtgg ttctggcacc c 21
<210> 240
   <211> 21
   <212> DNA
   <213> Artificial Sequence
<220>
   <223> Targeting sequence
<400> 240
   aagtggttct ggcacccctg t 21
<210> 241
   <211> 21
   <212> DNA
   <213> Artificial Sequence
<220>
   <223> Targeting sequence
<400> 241
   aaccataatc attccgaagc a 21
<210> 242
   <211> 21
   <212> DNA
   <213> Artificial Sequence
<220>
   <223> Targeting sequence
<400> 242
   aatcattccg aagcaaggtg t 21
<210> 243
   <211> 21
   <212> DNA
   <213> Artificial Sequence
<220>
   <223> Targeting sequence
<400> 243
   aagcaaggtg tgacttttgt t 21
<210> 244
   <211> 21
   <212> DNA
   <213> Artificial Sequence
<220>
   <223> Targeting sequence
<400> 244
   aaggtgtgac ttttgttcca a 21
<210> 245
   <211> 21
   <212> DNA
   <213> Artificial Sequence
<220>
   <223> Targeting sequence
<400> 245
   aataatgaag agtcctttat c 21
<210> 246
   <211> 21
   <212> DNA
   <213> Artificial Sequence
<220>
   <223> Targeting sequence
<400> 246
   aatgaagagt cctttatcct g 21
<210> 247
   <211> 21
   <212> DNA
   <213> Artificial Sequence
<220>
   <223> Targeting sequence
<400> 247
   aagagtcctt tatcctggat g 21
<210> 248
   <211> 21
   <212> DNA
   <213> Artificial Sequence
<220>
   <223> Targeting sequence
<400> 248
   aacatgggaa acagaattga g 21
<210> 249
   <211> 21
   <212> DNA
   <213> Artificial Sequence
<220>
   <223> Targeting sequence
<400> 249
   aaacagaatt gagagcatca c 21
<210> 250
   <211> 21
   <212> DNA
   <213> Artificial Sequence
<220>
   <223> Targeting sequence
<400> 250
   aacagaattg agagcatcac t 21
<210> 251
   <211> 21
   <212> DNA
   <213> Artificial Sequence
<220>
   <223> Targeting sequence
<400> 251
   aattgagagc atcactcagc g 21
<210> 252
   <211> 21
   <212> DNA
   <213> Artificial Sequence
<220>
   <223> Targeting sequence
<400> 252
   aataatagaa ggaaagaata a 21
<210> 253
   <211> 21
   <212> DNA
   <213> Artificial Sequence
<220>
   <223> Targeting sequence
<400> 253
   aatagaagga aagaataaga t 21
<210> 254
   <211> 21
   <212> DNA
   <213> Artificial Sequence
<220>
   <223> Targeting sequence
<400> 254
   aaggaaagaa taagatggct a 21
<210> 255
   <211> 21
   <212> DNA
   <213> Artificial Sequence
<220>
   <223> Targeting sequence
<400> 255
   aaagaataag atggctagca c 21
<210> 256
   <211> 21
   <212> DNA
   <213> Artificial Sequence
<220>
   <223> Targeting sequence
<400> 256
   aagaataaga tggctagcac c 21
<210> 257
   <211> 21
   <212> DNA
   <213> Artificial Sequence
<220>
   <223> Targeting sequence
<400> 257
   aataagatgg ctagcacctt g 21
<210> 258
   <211> 21
   <212> DNA
   <213> Artificial Sequence
<220>
   <223> Targeting sequence
<400> 258
   aagatggcta gcaccttggt t 21
<210> 259
   <211> 21
   <212> DNA
   <213> Artificial Sequence

<220>
   <223> Targeting sequence
<400> 259
   aatttctgga atctacattt g 21
<210> 260
   <211> 21
   <212> DNA
   <213> Artificial Sequence
<220>
   <223> Targeting sequence
<400> 260
   aatctacatt tgcatagctt c 21
<210> 261
   <211> 21
   <212> DNA
   <213> Artificial Sequence
<220>
   <223> Targeting sequence
<400> 261
   aataaagttg ggactgtggg a 21
<210> 262
   <211> 21
   <212> DNA
   <213> Artificial Sequence
<220>
   <223> Targeting sequence
<400> 262
   aaagttggga ctgtgggaag a 21
<210> 263
   <211> 21
   <212> DNA
   <213> Artificial Sequence
<220>
   <223> Targeting sequence
<400> 263
   aagttgggac tgtgggaaga a 21
<210> 264
   <211> 21
   <212> DNA
   <213> Artificial Sequence
<220>
   <223> Targeting sequence
<400> 264
   aagaaacata agcttttata t 21
<210> 265
   <211> 21
   <212> DNA
   <213> Artificial Sequence
<220>
   <223> Targeting sequence
<400> 265
   aaacataagc ttttatatca c 21
<210> 266
   <211> 21
   <212> DNA
   <213> Artificial Sequence
<220>
   <223> Targeting sequence
<400> 266
   aacataagct tttatatcac a 21
<210> 267
   <211> 21
   <212> DNA
   <213> Artificial Sequence
<220>
   <223> Targeting sequence
<400> 267
   aagcttttat atcacagatg t 21
<210> 268
   <211> 21
   <212> DNA
   <213> Artificial Sequence
<220>
   <223> Targeting sequence
<400> 268
   aaatgggttt catgttaact t 21
<210> 269
   <211> 21
   <212> DNA
   <213> Artificial Sequence
<220>
   <223> Targeting sequence
<400> 269
   aatgggtttc atgttaactt g 21
<210> 270
   <211> 21
   <212> DNA
   <213> Artificial Sequence
<220>
   <223> Targeting sequence
<400> 270
   aacttggaaa aaatgccgac g 21
<210> 271
   <211> 21
   <212> DNA
   <213> Artificial Sequence
<220>
   <223> Targeting sequence
<400> 271
   aaaaaatgcc gacggaagga g 21
<210> 272
   <211> 21
   <212> DNA
   <213> Artificial Sequence
<220>
   <223> Targeting sequence
<400> 272
   aaaaatgccg acggaaggag a 21
<210> 273
   <211> 21
   <212> DNA
   <213> Artificial Sequence
<220>
   <223> Targeting sequence
<400> 273
   aaaatgccga cggaaggaga g 21
<210> 274
   <211> 21
   <212> DNA
   <213> Artificial Sequence
<220>
   <223> Targeting sequence
<400> 274
   aaatgccgac ggaaggagag g 21
<210> 275
   <211> 21
   <212> DNA
   <213> Artificial Sequence
<220>
   <223> Targeting sequence
<400> 275
   aatgccgacg gaaggagagg a 21
<210> 276
   <211> 21
   <212> DNA
   <213> Artificial Sequence
<220>
   <223> Targeting sequence
<400> 276
   aaggagagga cctgaaactg t 21
<210> 277
   <211> 21
   <212> DNA
   <213> Artificial Sequence
<220>
   <223> Targeting sequence
<400> 277
   aaactgtctt gcacagttaa c 21
<210> 278
   <211> 21
   <212> DNA
   <213> Artificial Sequence
<220>
   <223> Targeting sequence
<400> 278
   aactgtcttg cacagttaac a 21
<210> 279
   <211> 21
   <212> DNA
   <213> Artificial Sequence
<220>
   <223> Targeting sequence
<400> 279
   aacaagttct tatacagaga c 21
<210> 280
   <211> 21
   <212> DNA
   <213> Artificial Sequence
<220>
   <223> Targeting sequence
<400> 280
   aagttcttat acagagacgt t 21
<210> 281
   <211> 21
   <212> DNA
   <213> Artificial Sequence
<220>
   <223> Targeting sequence
<400> 281
   aataacagaa caatgcacta c 21
<210> 282
   <211> 21
   <212> DNA
   <213> Artificial Sequence
<220>
   <223> Targeting sequence
<400> 282
   aacagaacaa tgcactacag t 21
<210> 283
   <211> 21
   <212> DNA
   <213> Artificial Sequence
<220>
   <223> Targeting sequence
<400> 283
   aacaatgcac tacagtatta g 21
<210> 284
   <211> 21
   <212> DNA
   <213> Artificial Sequence
<220>
   <223> Targeting sequence
<400> 284
   aatgcactac agtattagca a 21
<210> 285
   <211> 21
   <212> DNA
   <213> Artificial Sequence
<220>
   <223> Targeting sequence
<400> 285
   aagcaaaaaa tggccatcac t 21
<210> 286
   <211> 21
   <212> DNA
   <213> Artificial Sequence
<220>
   <223> Targeting sequence
<400> 286
   aaaaaatggc catcactaag g 21
<210> 287
   <211> 21
   <212> DNA
   <213> Artificial Sequence
<220>
   <223> Targeting sequence
<400> 287
   aaaaatggcc atcactaagg a 21
<210> 288
   <211> 21
   <212> DNA
   <213> Artificial Sequence
<220>
   <223> Targeting sequence
<400> 288
   aaaatggcca tcactaagga g 21
<210> 289
   <211> 21
   <212> DNA
   <213> Artificial Sequence
<220>
   <223> Targeting sequence
<400> 289
   aaatggccat cactaaggag c 21
<210> 290
   <211> 21
   <212> DNA
   <213> Artificial Sequence
<220>
   <223> Targeting sequence
<400> 290
   aatggccatc actaaggagc a 21
<210> 291
   <211> 21
   <212> DNA
   <213> Artificial Sequence
<220>
   <223> Targeting sequence
<400> 291
   aaggagcact ccatcactct t 21
<210> 292
   <211> 21
   <212> DNA
   <213> Artificial Sequence
<220>
   <223> Targeting sequence
<400> 292
   aatcttacca tcatgaatgt t 21
<210> 293
   <211> 21
   <212> DNA
   <213> Artificial Sequence
<220>
   <223> Targeting sequence
<400> 293
   aatgtttccc tgcaagattc a 21
<210> 294
   <211> 21
   <212> DNA
   <213> Artificial Sequence
<220>
   <223> Targeting sequence
<400> 294
   aagattcagg cacctatgcc t 21
<210> 295
   <211> 21
   <212> DNA
   <213> Artificial Sequence
<220>
   <223> Targeting sequence
<400> 295
   aatgtataca caggggaaga a 21
<210> 296
   <211> 21
   <212> DNA
   <213> Artificial Sequence
<220>
   <223> Targeting sequence
<400> 296
   aagaaatcct ccagaagaaa g 21
<210> 297
   <211> 21
   <212> DNA
   <213> Artificial Sequence
<220>
   <223> Targeting sequence
<400> 297
   aaatcctcca gaagaaagaa a 21
<210> 298
   <211> 21
   <212> DNA
   <213> Artificial Sequence
<220>
   <223> Targeting sequence
<400> 298
   aatcctccag aagaaagaaa t 21
<210> 299
   <211> 21
   <212> DNA
   <213> Artificial Sequence
<220>
   <223> Targeting sequence
<400> 299
   aagaaagaaa ttacaatcag a 21
<210> 300
   <211> 21
   <212> DNA
   <213> Artificial Sequence
<220>
   <223> Targeting sequence
<400> 300
   aaagaaatta caatcagaga t 21
<210> 301
   <211> 21
   <212> DNA
   <213> Artificial Sequence
<220>
   <223> Targeting sequence
<400> 301
   aagaaattac aatcagagat c 21
<210> 302
   <211> 21
   <212> DNA
   <213> Artificial Sequence
<220>
   <223> Targeting sequence
<400> 302
   aaattacaat cagagatcag g 21
<210> 303
   <211> 21
   <212> DNA
   <213> Artificial Sequence
<220>
   <223> Targeting sequence
<400> 303
   aattacaatc agagatcagg a 21
<210> 304
   <211> 21
   <212> DNA
   <213> Artificial Sequence
<220>
   <223> Targeting sequence
<400> 304
   aatcagagat caggaagcac c 21
<210> 305
   <211> 21
   <212> DNA
   <213> Artificial Sequence
<220>
   <223> Targeting sequence
<400> 305
   aagcaccata cctcctgcga a 21
<210> 306
   <211> 21
   <212> DNA
   <213> Artificial Sequence
<220>
   <223> Targeting sequence
<400> 306
   aaacctcagt gatcacacag t 21
<210> 307
   <211> 20
   <212> DNA
   <213> Artificial Sequence
<220>
   <223> Targeting sequence
<400> 307
   aacctcagtg atcacacagt 20
<210> 308
   <211> 21
   <212> DNA
   <213> Artificial Sequence
<220>
   <223> Targeting sequence
<400> 308
   aatggtgtcc ccgagcctca g 21
<210> 309
   <211> 21
   <212> DNA
   <213> Artificial Sequence
<220>
   <223> Targeting sequence
<400> 309
   aaaaacaacc acaaaataca a 21
<210> 310
   <211> 21
   <212> DNA
   <213> Artificial Sequence
<220>
   <223> Targeting sequence
<400> 310
   aaaacaacca caaaatacaa c 21
<210> 311
   <211> 21
   <212> DNA
   <213> Artificial Sequence
<220>
   <223> Targeting sequence
<400> 311
   aaacaaccac aaaatacaac a 21
<210> 312
   <211> 21
   <212> DNA
   <213> Artificial Sequence
<220>
   <223> Targeting sequence
<400> 312
   aacaaccaca aaatacaaca a 21
<210> 313
   <211> 21
   <212> DNA
   <213> Artificial Sequence
<220>
   <223> Targeting sequence
<400> 313
   aaccacaaaa tacaacaaga g 21
<210> 314
   <211> 21
   <212> DNA
   <213> Artificial Sequence
<220>
   <223> Targeting sequence
<400> 314
   aaaatacaac aagagcctgg a 21
<210> 315
   <211> 21
   <212> DNA
   <213> Artificial Sequence
<220>
   <223> Targeting sequence
<400> 315
   aaatacaaca agagcctgga a 21
<210> 316
   <211> 21
   <212> DNA
   <213> Artificial Sequence
<220>
   <223> Targeting sequence
<400> 316
   aatacaacaa gagcctggaa t 21
<210> 317
   <211> 21
   <212> DNA
   <213> Artificial Sequence
<220>
   <223> Targeting sequence
<400> 317
   aacaagagcc tggaattatt t 21
<210> 318
   <211> 21
   <212> DNA
   <213> Artificial Sequence
<220>
   <223> Targeting sequence
<400> 318
   aagagcctgg aattatttta g 21
<210> 319
   <211> 21
   <212> DNA
   <213> Artificial Sequence
<220>
   <223> Targeting sequence
<400> 319
   aattatttta ggaccaggaa g 21
<210> 320
   <211> 21
   <212> DNA
   <213> Artificial Sequence
<220>
   <223> Targeting sequence
<400> 320
   aagcagcacg ctgtttattg a 21
<210> 321
   <211> 21
   <212> DNA
   <213> Artificial Sequence
<220>
   <223> Targeting sequence
<400> 321
   aaagagtcac agaagaggat g 21
<210> 322
   <211> 21
   <212> DNA
   <213> Artificial Sequence
<220>
   <223> Targeting sequence
<400> 322
   aagagtcaca gaagaggatg a 21
<210> 323
   <211> 21
   <212> DNA
   <213> Artificial Sequence
<220>
   <223> Targeting sequence
<400> 323
   aagaggatga aggtgtctat c 21
<210> 324
   <211> 21
   <212> DNA
   <213> Artificial Sequence
<220>
   <223> Targeting sequence
<400> 324
   aaggtgtcta tcactgcaaa g 21
<210> 325
   <211> 21
   <212> DNA
   <213> Artificial Sequence
<220>
   <223> Targeting sequence
<400> 325
   aaagccacca accagaaggg c 21
<210> 326
   <211> 21
   <212> DNA
   <213> Artificial Sequence
<220>
   <223> Targeting sequence
<400> 326
   aagccaccaa ccagaagggc t 21
<210> 327
   <211> 21
   <212> DNA
   <213> Artificial Sequence
<220>
   <223> Targeting sequence
<400> 327
   aaccagaagg gctctgtgga a 21
<210> 328
   <211> 21
   <212> DNA
   <213> Artificial Sequence
<220>
   <223> Targeting sequence
<400> 328
   aagggctctg tggaaagttc a 21
<210> 329
   <211> 21
   <212> DNA
   <213> Artificial Sequence
<220>
   <223> Targeting sequence
<400> 329
   aaagttcagc atacctcact g 21
<210> 330
   <211> 21
   <212> DNA
   <213> Artificial Sequence
<220>
   <223> Targeting sequence
<400> 330
   aagttcagca tacctcactg t 21
<210> 331
   <211> 21
   <212> DNA
   <213> Artificial Sequence
<220>
   <223> Targeting sequence
<400> 331
   aaggaacctc ggacaagtct a 21
<210> 332
   <211> 21
   <212> DNA
   <213> Artificial Sequence
<220>
   <223> Targeting sequence
<400> 332
   aacctcggac aagtctaatc t 21
<210> 333
   <211> 21
   <212> DNA
   <213> Artificial Sequence
<220>
   <223> Targeting sequence
<400> 333
   aagtctaatc tggagctgat c 21
<210> 334
   <211> 21
   <212> DNA
   <213> Artificial Sequence
<220>
   <223> Targeting sequence
<400> 334
   aatctggagc tgatcactct a 21
<210> 335
   <211> 21
   <212> DNA
   <213> Artificial Sequence
<220>
   <223> Targeting sequence
<400> 335
   aacatgcacc tgtgtggctg c 21
<210> 336
   <211> 21
   <212> DNA
   <213> Artificial Sequence
<220>
   <223> Targeting sequence
<400> 336
   aaccctcttt atccgaaaaa t 21
<210> 337
   <211> 21
   <212> DNA
   <213> Artificial Sequence
<220>
   <223> Targeting sequence
<400> 337
   aaaaatgaaa aggtcttctt c 21
<210> 338
   <211> 21
   <212> DNA
   <213> Artificial Sequence
<220>
   <223> Targeting sequence
<400> 338
   aaaatgaaaa ggtcttcttc t 21
<210> 339
   <211> 21
   <212> DNA
   <213> Artificial Sequence
<220>
   <223> Targeting sequence
<400> 339
   aaatgaaaag gtcttcttct g 21
<210> 340
   <211> 21
   <212> DNA
   <213> Artificial Sequence
<220>
   <223> Targeting sequence
<400> 340
   aatgaaaagg tcttcttctg a 21
<210> 341
   <211> 21
   <212> DNA
   <213> Artificial Sequence
<220>
   <223> Targeting sequence
<400> 341
   aaaaggtctt cttctgaaat a 21
<210> 342
   <211> 21
   <212> DNA
   <213> Artificial Sequence
<220>
   <223> Targeting sequence
<400> 342
   aaaggtcttc ttctgaaata a 21
<210> 343
   <211> 21
   <212> DNA
   <213> Artificial Sequence
<220>
   <223> Targeting sequence
<400> 343
   aaggtcttct tctgaaataa a 21
<210> 344
   <211> 21
   <212> DNA
   <213> Artificial Sequence
<220>
   <223> Targeting sequence
<400> 344
   aaataaagac tgactaccta t 21
<210> 345
   <211> 21
   <212> DNA
   <213> Artificial Sequence
<220>
   <223> Targeting sequence
<400> 345
   aataaagact gactacctat c 21
<210> 346
   <211> 21
   <212> DNA
   <213> Artificial Sequence
<220>
   <223> Targeting sequence
<400> 346
   aaagactgac tacctatcaa t 21
<210> 347
   <211> 21
   <212> DNA
   <213> Artificial Sequence
<220>
   <223> Targeting sequence
<400> 347
   aagactgact acctatcaat t 21
<210> 348
   <211> 21
   <212> DNA
   <213> Artificial Sequence
<220>
   <223> Targeting sequence
<400> 348
   aattataatg gacccagatg a 21
<210> 349
   <211> 21
   <212> DNA
   <213> Artificial Sequence
<220>
   <223> Targeting sequence
<400> 349
   aatggaccca gatgaagttc c 21
<210> 350
   <211> 21
   <212> DNA
   <213> Artificial Sequence
<220>
   <223> Targeting sequence
<400> 350
   aagttccttt ggatgagcag t 21
<210> 351
   <211> 21
   <212> DNA
   <213> Artificial Sequence
<220>
   <223> Targeting sequence
<400> 351
   aagtgggagt ttgcccggga g 21
<210> 352
   <211> 21
   <212> DNA
   <213> Artificial Sequence
<220>
   <223> Targeting sequence
<400> 352
   aaactgggca aatcacttgg a 21
<210> 353
   <211> 21
   <212> DNA
   <213> Artificial Sequence
<220>
   <223> Targeting sequence
<400> 353
   aactgggcaa atcacttgga a 21
<210> 354
   <211> 21
   <212> DNA
   <213> Artificial Sequence
<220>
   <223> Targeting sequence
<400> 354
   aaatcacttg gaagaggggc t 21
<210> 355
   <211> 21
   <212> DNA
   <213> Artificial Sequence
<220>
   <223> Targeting sequence
<400> 355
   aatcacttgg aagaggggct t 21
<210> 356
   <211> 21
   <212> DNA
   <213> Artificial Sequence
<220>
   <223> Targeting sequence
<400> 356
   aagaggggct tttggaaaag t 21
<210> 357
   <211> 21
   <212> DNA
   <213> Artificial Sequence
<220>
   <223> Targeting sequence
   <400> 357
   aaaagtggtt caagcatcag c 21
<210> 358
   <211> 21
   <212> DNA
   <213> Artificial Sequence
<220>
   <223> Targeting sequence
<400> 358
   aaagtggttc aagcatcagc a 21
<210> 359
   <211> 21
   <212> DNA
   <213> Artificial Sequence
<220>
   <223> Targeting sequence
<400> 359
   aagtggttca agcatcagca t 21
<210> 360
   <211> 21
   <212> DNA
   <213> Artificial Sequence
<220>
   <223> Targeting sequence
<400> 360
   aagcatcagc atttggcatt a 21
<210> 361
   <211> 21
   <212> DNA
   <213> Artificial Sequence
<220>
   <223> Targeting sequence
<400> 361
   aagaaatcac ctacgtgccg g 21
<210> 362
   <211> 21
   <212> DNA
   <213> Artificial Sequence
<220>
   <223> Targeting sequence
<400> 362
   aaatcaccta cgtgccggac t 21
<210> 363
   <211> 21
   <212> DNA
   <213> Artificial Sequence
<220>
   <223> Targeting sequence
<400> 363
   aatcacctac gtgccggact g 21
<210> 364
   <211> 21
   <212> DNA
   <213> Artificial Sequence
<220>
   <223> Targeting sequence
<400> 364
   aaaatgctga aagagggggc c 21
<210> 365
   <211> 21
   <212> DNA
   <213> Artificial Sequence
<220>
   <223> Targeting sequence
<400> 365
   aaatgctgaa agagggggcc a 21
<210> 366
   <211> 21
   <212> DNA
   <213> Artificial Sequence
<220>
   <223> Targeting sequence
<400> 366
   aatgctgaaa gagggggcca c 21
<210> 367
   <211> 21
   <212> DNA
   <213> Artificial Sequence
<220>
   <223> Targeting sequence
<400> 367
   aaagaggggg ccacggccag c 21
<210> 368
   <211> 21
   <212> DNA
   <213> Artificial Sequence
<220>
   <223> Targeting sequence
<400> 368
   aagagggggc cacggccagc g 21
<210> 369
   <211> 21
   <212> DNA
   <213> Artificial Sequence
<220>
   <223> Targeting sequence

<400> 369
   aaagctctga tgactgagct a 21
<210> 370
   <211> 21
   <212> DNA
   <213> Artificial Sequence
<220>
   <223> Targeting sequence
<400> 370
   aagctctgat gactgagcta a 21
<210> 371
   <211> 21
   <212> DNA
   <213> Artificial Sequence
<220>
   <223> Targeting sequence
<400> 371
   aaaaatcttg acccacattg g 21
<210> 372
   <211> 21
   <212> DNA
   <213> Artificial Sequence
<220>
   <223> Targeting sequence
<400> 372
   aaaatcttga cccacattgg c 21
<210> 373
   <211> 21
   <212> DNA
   <213> Artificial Sequence
<220>
   <223> Targeting sequence
<400> 373
   aaatcttgac ccacattggc c 21
<210> 374
   <211> 21
   <212> DNA
   <213> Artificial Sequence
<220>
   <223> Targeting sequence
<400> 374
   aatcttgacc cacattggcc a 21
<210> 375
   <211> 21
   <212> DNA
   <213> Artificial Sequence
<220>
   <223> Targeting sequence
<400> 375
   aacgtggtta acctgctggg a 21
<210> 376
   <211> 21
   <212> DNA
   <213> Artificial Sequence
<220>
   <223> Targeting sequence
<400> 376
   aacctgctgg gagcctgcac c 21
<210> 377
   <211> 21
   <212> DNA
   <213> Artificial Sequence
<220>
   <223> Targeting sequence
<400> 377
   aagcaaggag ggcctctgat g 21
<210> 378
   <211> 21
   <212> DNA
   <213> Artificial Sequence
<220>
   <223> Targeting sequence
<400> 378
   aaggagggcc tctgatggtg a 21
<210> 379
   <211> 21
   <212> DNA
   <213> Artificial Sequence
<220>
   <223> Targeting sequence
<400> 379
   aatactgcaa atatggaaat c 21 <210> 380
   <211> 21
   <212> DNA
   <213> Artificial Sequence
<220>
   <223> Targeting sequence
<400> 380
   aaatatggaa atctctccaa c 21
<210> 381
   <211> 21
   <212> DNA
   <213> Artificial Sequence
<220>
   <223> Targeting sequence
<400> 381
   aatatggaaa tctctccaac t 21
<210> 382
   <211> 21
   <212> DNA
   <213> Artificial Sequence
<220>
   <223> Targeting sequence
<400> 382
   aaatctctcc aactacctca a 21
<210> 383
   <211> 21
   <212> DNA
   <213> Artificial Sequence
<220>
   <223> Targeting sequence
<400> 383
   aatctctcca actacctcaa g 21
<210> 384
   <211> 21
   <212> DNA
   <213> Artificial Sequence
<220>
   <223> Targeting sequence
<400> 384
   aactacctca agagcaaacg t 21
<210> 385
   <211> 21
   <212> DNA
   <213> Artificial Sequence
<220>
   <223> Targeting sequence
<400> 385
   aagagcaaac gtgacttatt t 21
<210> 386
   <211> 21
   <212> DNA
   <213> Artificial Sequence
<220>
   <223> Targeting sequence
<400> 386
   aaacgtgact tattttttct c 21
<210> 387
   <211> 21
   <212> DNA
   <213> Artificial Sequence
<220>
   <223> Targeting sequence
<400> 387
   aacgtgactt attttttctc a 21
<210> 388
   <211> 21
   <212> DNA
   <213> Artificial Sequence
<220>
   <223> Targeting sequence
<400> 388
   aacaaggatg cagcactaca c 21
<210> 389
   <211> 21
   <212> DNA
   <213> Artificial Sequence
<220>
   <223> Targeting sequence
<400> 389
   aaggatgcag cactacacat g 21
<210> 390
   <211> 21
   <212> DNA
   <213> Artificial Sequence
<220>
   <223> Targeting sequence
<400> 390
   aagaaagaaa aaatggagcc a 21
<210> 391
   <211> 21
   <212> DNA
   <213> Artificial Sequence
<220>
   <223> Targeting sequence
<400> 391
   aaagaaaaaa tggagccagg c 21
<210> 392
   <211> 21
   <212> DNA
   <213> Artificial Sequence
<220>
   <223> Targeting sequence
<400> 392
   aagaaaaaat ggagccaggc c 21
<210> 393
   <211> 21
   <212> DNA
   <213> Artificial Sequence
<220>
   <223> Targeting sequence
<400> 393
   aaaaaatgga gccaggcctg g 21
<210> 394
   <211> 21
   <212> DNA
   <213> Artificial Sequence
<220>
   <223> Targeting sequence
<400> 394
   aaaaatggag ccaggcctgg a 21
<210> 395
   <211> 21
   <212> DNA
   <213> Artificial Sequence
<220>
   <223> Targeting sequence
<400> 395
   aaaatggagc caggcctgga a 21
<210> 396
   <211> 21
   <212> DNA
   <213> Artificial Sequence
<220>
   <223> Targeting sequence
<400> 396
   aaatggagcc aggcctggaa c 21
<210> 397
   <211> 21
   <212> DNA
   <213> Artificial Sequence
<220>
   <223> Targeting sequence
<400> 397
   aatggagcca ggcctggaac a 21
<210> 398
   <211> 21
   <212> DNA
   <213> Artificial Sequence
<220>
   <223> Targeting sequence
<400> 398
   aacaaggcaa gaaaccaaga c 21
<210> 399
   <211> 21
   <212> DNA
   <213> Artificial Sequence
<220>
   <223> Targeting sequence
<400> 399
   aaggcaagaa accaagacta g 21
<210> 400
   <211> 21
   <212> DNA
   <213> Artificial Sequence
<220>
   <223> Targeting sequence
<400> 400
   aagaaaccaa gactagatag c 21
<210> 401
   <211> 21
   <212> DNA
   <213> Artificial Sequence
<220>
   <223> Targeting sequence
<400> 401
   aaaccaagac tagatagcgt c 21
<210> 402
   <211> 21
   <212> DNA
   <213> Artificial Sequence
<220>
   <223> Targeting sequence
<400> 402
   aaccaagact agatagcgtc a 21
<210> 403
   <211> 21
   <212> DNA
   <213> Artificial Sequence
<220>
   <223> Targeting sequence
<400> 403
   aagactagat agcgtcacca g 21
<210> 404
   <211> 21
   <212> DNA
   <213> Artificial Sequence
<220>
   <223> Targeting sequence
<400> 404
   aaagctttgc gagctccggc t 21
<210> 405
   <211> 20
   <212> DNA
   <213> Artificial Sequence
<220>
   <223> Targeting sequence
<400> 405
   aagctttgcg agctccggct 20
<210> 406
   <211> 21
   <212> DNA
   <213> Artificial Sequence
<220>
   <223> Targeting sequence
<400> 406
   aagataaaag tctgagtgat g 21
<210> 407
   <211> 21
   <212> DNA
   <213> Artificial Sequence
<220>
   <223> Targeting sequence
<400> 407
   aaaagtctga gtgatgttga g 21
<210> 408
   <211> 21
   <212> DNA
   <213> Artificial Sequence
<220>
   <223> Targeting sequence
<400> 408
   aaagtctgag tgatgttgag g 21
<210> 409
   <211> 21
   <212> DNA
   <213> Artificial Sequence
<220>
   <223> Targeting sequence
<400> 409
   aagtctgagt gatgttgagg a 21
<210> 410
   <211> 21
   <212> DNA
   <213> Artificial Sequence
<220>
   <223> Targeting sequence
<400> 410
   aagaggagga ttctgacggt t 21
<210> 411
   <211> 21
   <212> DNA
   <213> Artificial Sequence
<220>
   <223> Targeting sequence
<400> 411
   aaggagccca tcactatgga a 21
<210> 412
   <211> 21
   <212> DNA
   <213> Artificial Sequence
<220>
   <223> Targeting sequence
<400> 412
   aagatctgat ttcttacagt t 21
<210> 413
   <211> 21
   <212> DNA
   <213> Artificial Sequence
<220>
   <223> Targeting sequence
<400> 413
   aagtggccag aggcatggag t 21
<210> 414
   <211> 21
   <212> DNA
   <213> Artificial Sequence
<220>
   <223> Targeting sequence
<400> 414
   aaagtgcatt catcgggacc t 21
<210> 415
   <211> 21
   <212> DNA
   <213> Artificial Sequence
<220>
   <223> Targeting sequence
<400> 415
   aagtgcattc atcgggacct g 21
<210> 416
   <211> 21
   <212> DNA
   <213> Artificial Sequence
<220>
   <223> Targeting sequence
<400> 416
   aaacattctt ttatctgaga a 21
<210> 417
   <211> 21
   <212> DNA
   <213> Artificial Sequence
<220>
   <223> Targeting sequence
<400> 417
   aacattcttt tatctgagaa c 21
<210> 418
   <211> 21
   <212> DNA
   <213> Artificial Sequence
<220>
   <223> Targeting sequence
   <400> 418
   aacaacgtgg tgaagatttg t 21
<210> 419
   <211> 21
   <212> DNA
   <213> Artificial Sequence
<220>
   <223> Targeting sequence
<400> 419
   aacgtggtga agatttgtga t 21
<210> 420
   <211> 21
   <212> DNA
   <213> Artificial Sequence
<220>
   <223> Targeting sequence
<400> 420
   aagatttgtg attttggcct t 21
<210> 421
   <211> 21
   <212> DNA
   <213> Artificial Sequence
<220>
   <223> Targeting sequence
<400> 421
   aagaaccccg attatgtgag a 21
<210> 422
   <211> 21
   <212> DNA
   <213> Artificial Sequence
<220>
   <223> Targeting sequence
<400> 422
   aaccccgatt atgtgagaaa a 21
<210> 423
   <211> 21
   <212> DNA
   <213> Artificial Sequence
<220>
   <223> Targeting sequence
<400> 423
   aaaaggagat actcgacttc c 21
<210> 424
   <211> 21
   <212> DNA
   <213> Artificial Sequence
<220>
   <223> Targeting sequence
<400> 424
   aaaggagata ctcgacttcc t 21
<210> 425
   <211> 21
   <212> DNA
   <213> Artificial Sequence
<220>
   <223> Targeting sequence
<400> 425
   aaggagatac tcgacttcct c 21
<210> 426
   <211> 21
   <212> DNA
   <213> Artificial Sequence
<220>
   <223> Targeting sequence
<400> 426
   aaatggatgg ctcctgaatc t 21
<210> 427
   <211> 21
   <212> DNA
   <213> Artificial Sequence
<220>
   <223> Targeting sequence
<400> 427
   aatggatggc tcctgaatct a 21
<210> 428
   <211> 21
   <212> DNA
   <213> Artificial Sequence
<220>
   <223> Targeting sequence
<400> 428
   aatctatctt tgacaaaatc t 21
<210> 429
   <211> 21
   <212> DNA
   <213> Artificial Sequence
<220>
   <223> Targeting sequence
<400> 429
   aaaatctaca gcaccaagag c 21
<210> 430
   <211> 21
   <212> DNA
   <213> Artificial Sequence
<220>
   <223> Targeting sequence
<400> 430
   aaatctacag caccaagagc g 21
<210> 431
   <211> 21
   <212> DNA
   <213> Artificial Sequence
<220>
   <223> Targeting sequence
<400> 431
   aatctacagc accaagagcg a 21
<210> 432
   <211> 21
   <212> DNA
   <213> Artificial Sequence
<220>
   <223> Targeting sequence
<400> 432
   aagagcgacg tgtggtctta c 21
<210> 433
   <211> 21
   <212> DNA
   <213> Artificial Sequence
<220>
   <223> Targeting sequence
<400> 433
   aaatcttctc cttaggtggg t 21
<210> 434
   <211> 21
   <212> DNA
   <213> Artificial Sequence
<220>
   <223> Targeting sequence

<400> 434
   aatcttctcc ttaggtgggt c 21
<210> 435
   <211> 21
   <212> DNA
   <213> Artificial Sequence
<220>
   <223> Targeting sequence
<400> 435
   aaatggatga ggacttttgc a 21
<210> 436
   <211> 21
   <212> DNA
   <213> Artificial Sequence
<220>
   <223> Targeting sequence
<400> 436
   aatggatgag gacttttgca g 21
<210> 437
   <211> 21
   <212> DNA
   <213> Artificial Sequence
<220>
   <223> Targeting sequence
<400> 437
   aaggcatgag gatgagagct c 21
<210> 438
   <211> 21
   <212> DNA
   <213> Artificial Sequence
<220>
   <223> Targeting sequence
<400> 438
   aaatctatca gatcatgctg g 21
<210> 439
   <211> 21
   <212> DNA
   <213> Artificial Sequence
<220>
   <223> Targeting sequence
<400> 439
   aatctatcag atcatgctgg a 21
<210> 440
   <211> 21
   <212> DNA
   <213> Artificial Sequence
<220>
   <223> Targeting sequence
<400> 440
   aaaagaaagg ccaagatttg c 21
<210> 441
   <211> 21
   <212> DNA
   <213> Artificial Sequence
<220>
   <223> Targeting sequence
<400> 441
   aaagaaaggc caagatttgc a 21
<210> 442
   <211> 21
   <212> DNA
   <213> Artificial Sequence
<220>
   <223> Targeting sequence
<400> 442
   aagaaaggcc aagatttgca g 21
<210> 443
   <211> 21
   <212> DNA
   <213> Artificial Sequence
<220>
   <223> Targeting sequence
<400> 443
   aaaggccaag atttgcagaa c 21
<210> 444
   <211> 21
   <212> DNA
   <213> Artificial Sequence
<220>
   <223> Targeting sequence
<400> 444
   aaggccaaga tttgcagaac t 21
<210> 445
   <211> 21
   <212> DNA
   <213> Artificial Sequence
<220>
   <223> Targeting sequence
<400> 445
   aagatttgca gaacttgtgg a 21
<210> 446
   <211> 21
   <212> DNA
   <213> Artificial Sequence
<220>
   <223> Targeting sequence
<400> 446
   aacttgtgga aaaactaggt g 21
<210> 447
   <211> 21
   <212> DNA
   <213> Artificial Sequence
<220>
   <223> Targeting sequence
<400> 447
   aaaaactagg tgatttgctt c 21
<210> 448
   <211> 21
   <212> DNA
   <213> Artificial Sequence
<220>
   <223> Targeting sequence
<400> 448
   aaaactaggt gatttgcttc a 21
<210> 449
   <211> 21
   <212> DNA
   <213> Artificial Sequence
<220>
   <223> Targeting sequence
<400> 449
   aaactaggtg atttgcttca a 21
<210> 450
   <211> 21
   <212> DNA
   <213> Artificial Sequence
<220>
   <223> Targeting sequence
<400> 450
   aactaggtga tttgcttcaa g 21
<210> 451
   <211> 21
   <212> DNA
   <213> Artificial Sequence
<220>
   <223> Targeting sequence
<400> 451
   aagcaaatgt acaacaggat g 21
<210> 452
   <211> 21
   <212> DNA
   <213> Artificial Sequence
<220>
   <223> Targeting sequence
<400> 452
   aaatgtacaa caggatggta a 21
<210> 453
   <211> 21
   <212> DNA
   <213> Artificial Sequence
<220>
   <223> Targeting sequence
<400> 453
   aatgtacaac aggatggtaa a 21
<210> 454
   <211> 21
   <212> DNA
   <213> Artificial Sequence
<220>
   <223> Targeting sequence
<400> 454
   aacaggatgg taaagactac a 21
<210> 455
   <211> 21
   <212> DNA
   <213> Artificial Sequence
<220>
   <223> Targeting sequence
<400> 455
   aaagactaca tcccaatcaa t 21
<210> 456
   <211> 21
   <212> DNA
   <213> Artificial Sequence
<220>
   <223> Targeting sequence
<400> 456
   aagactacat cccaatcaat g 21
<210> 457
   <211> 21
   <212> DNA
   <213> Artificial Sequence
<220>
   <223> Targeting sequence
<400> 457
   aatcaatgcc atactgacag g 21
<210> 458
   <211> 21
   <212> DNA
   <213> Artificial Sequence
<220>
   <223> Targeting sequence
<400> 458
   aatgccatac tgacaggaaa t 21
<210> 459
   <211> 21
   <212> DNA
   <213> Artificial Sequence
<220>
   <223> Targeting sequence
<400> 459
   aaatagtggg tttacatact c 21
<210> 460
   <211> 21
   <212> DNA
   <213> Artificial Sequence
<220>
   <223> Targeting sequence
<400> 460
   aatagtgggt ttacatactc a 21
<210> 461
   <211> 21
   <212> DNA
   <213> Artificial Sequence
<220>
   <223> Targeting sequence
<400> 461
   aactcctgcc ttctctgagg a 21
<210> 462
   <211> 21
   <212> DNA
   <213> Artificial Sequence
<220>
   <223> Targeting sequence
<400> 462
   aaggaaagta tttcagctcc g 21
<210> 463
   <211> 21
   <212> DNA
   <213> Artificial Sequence
<220>
   <223> Targeting sequence
<400> 463
   aaagtatttc agctccgaag t 21
<210> 464
   <211> 21
   <212> DNA
   <213> Artificial Sequence
<220>
   <223> Targeting sequence
<400> 464
   aagtatttca gctccgaagt t 21
<210> 465
   <211> 21
   <212> DNA
   <213> Artificial Sequence
<220>
   <223> Targeting sequence
<400> 465
   aagtttaatt caggaagctc t 21
<210> 466
   <211> 21
   <212> DNA
   <213> Artificial Sequence
<220>
   <223> Targeting sequence
<400> 466
   aattcaggaa gctctgatga t 21
<210> 467
   <211> 21
   <212> DNA
   <213> Artificial Sequence
<220>
   <223> Targeting sequence
<400> 467
   aagctctgat gatgtcagat a 21
<210> 468
   <211> 21
   <212> DNA
   <213> Artificial Sequence
<220>
   <223> Targeting sequence
<400> 468
   aaatgctttc aagttcatga g 21
<210> 469
   <211> 21
   <212> DNA
   <213> Artificial Sequence
<220>
   <223> Targeting sequence
<400> 469
   aatgctttca agttcatgag c 21
<210> 470
   <211> 21
   <212> DNA
   <213> Artificial Sequence
<220>
   <223> Targeting sequence
<400> 470
   aagttcatga gcctggaaag a 21
<210> 471
   <211> 21
   <212> DNA
   <213> Artificial Sequence
<220>
   <223> Targeting sequence
<400> 471
   aaagaatcaa aacctttgaa g 21
<210> 472
   <211> 21
   <212> DNA
   <213> Artificial Sequence
<220>
   <223> Targeting sequence
<400> 472
   aagaatcaaa acctttgaag a 21
<210> 473
   <211> 21
   <212> DNA
   <213> Artificial Sequence
<220>
   <223> Targeting sequence
<400> 473
   aatcaaaacc tttgaagaac t 21
<210> 474
   <211> 21
   <212> DNA
   <213> Artificial Sequence
<220>
   <223> Targeting sequence
<400> 474
   aaaacctttg aagaactttt a 21
<210> 475
   <211> 21
   <212> DNA
   <213> Artificial Sequence
<220>
   <223> Targeting sequence
<400> 475
   aaacctttga agaactttta c 21
<210> 476
   <211> 21
   <212> DNA
   <213> Artificial Sequence
<220>
   <223> Targeting sequence
<400> 476
   aacctttgaa gaacttttac c 21
<210> 477
   <211> 21
   <212> DNA
   <213> Artificial Sequence
<220>
   <223> Targeting sequence
<400> 477
   aagaactttt accgaatgcc a 21
<210> 478
   <211> 21
   <212> DNA
   <213> Artificial Sequence
<220>
   <223> Targeting sequence
<400> 478
   aacttttacc gaatgccacc t 21
<210> 479
   <211> 21
   <212> DNA
   <213> Artificial Sequence
<220>
   <223> Targeting sequence
   <400> 479
   aatgccacct ccatgtttga t 21
<210> 480
   <211> 21
   <212> DNA
   <213> Artificial Sequence
<220>
   <223> Targeting sequence
<400> 480
   aaacccaagg cctcgctcaa g 21

<210> 481
   <211> 21
   <212> DNA
   <213> Artificial Sequence
<220>
   <223> Targeting sequence
<400> 481
   aaacccaagg cctcgctcaa g 21
<210> 482
   <211> 21
   <212> DNA
   <213> Artificial Sequence
<220>
   <223> Targeting sequence
<400> 482
   aacccaaggc ctcgctcaag a 21
<210> 483
   <211> 21
   <212> DNA
   <213> Artificial Sequence
<220>
   <223> Targeting sequence
<400> 483
   aaggcctcgc tcaagattga c 21
<210> 484
   <211> 21
   <212> DNA
   <213> Artificial Sequence
<220>
   <223> Targeting sequence
<400> 484
   aagattgact tgagagtaac c 21
<210> 485
   <211> 21
   <212> DNA
   <213> Artificial Sequence
<220>
   <223> Targeting sequence
<400> 485
   aaccagtaaa agtaaggagt c 21
<210> 486
   <211> 21
   <212> DNA
   <213> Artificial Sequence
<220>
   <223> Targeting sequence
<400> 486
   aaaagtaagg agtcggggct g 21
<210> 487
   <211> 21
   <212> DNA
   <213> Artificial Sequence
<220>
   <223> Targeting sequence
<400> 487
   aaagtaagga gtcggggctg t 21
<210> 488
   <211> 21
   <212> DNA
   <213> Artificial Sequence
<220>
   <223> Targeting sequence
<400> 488
   aagtaaggag tcggggctgt c 21
<210> 489
   <211> 21
   <212> DNA
   <213> Artificial Sequence
<220>
   <223> Targeting sequence
<400> 489
   aaggagtcgg ggctgtctga t 21
<210> 490
   <211> 21
   <212> DNA
   <213> Artificial Sequence
<220>
   <223> Targeting sequence
<400> 490
   aaggcaagcg caggttcacc t 21
<210> 491
   <211> 21
   <212> DNA
   <213> Artificial Sequence
<220>
   <223> Targeting sequence
<400> 491
   gcacccagca catcatgcaa g 21
<210> 492
   <211> 21
   <212> DNA
   <213> Artificial Sequence
<220>
   <223> Targeting sequence
<400> 492
   ggctgagcat aactaaatct g 21
<210> 493
   <211> 21
   <212> DNA
   <213> Artificial Sequence
<220>
   <223> Targeting sequence
<400> 493
   gcttctacag ctgcaaatat c 21
<210> 494
   <211> 21
   <212> DNA
   <213> Artificial Sequence
<220>
   <223> Targeting sequence
<400> 494
   gagctcgtca ttccctgccg g 21
<210> 495
   <211> 21
   <212> DNA
   <213> Artificial Sequence
<220>
   <223> Targeting sequence
<400> 495
   ccaaagcaat tcccatgcca a 21
<210> 496
   <211> 21
   <212> DNA
   <213> Artificial Sequence
<220>
   <223> Targeting sequence
<400> 496
   tgctgagcat aaaacagtca a 21
<210> 497
   <211> 21
   <212> DNA
   <213> Artificial Sequence
<220>
   <223> Targeting sequence
<400> 497
   ttgcatagct tccaataaag t 21
<210> 498
   <211> 21
   <212> DNA
   <213> Artificial Sequence
<220>
   <223> Targeting sequence
<400> 498
   agtgatcaca cagtggccat c 21
<210> 499
   <211> 21
   <212> DNA
   <213> Artificial Sequence
<220>
   <223> Targeting sequence
<400> 499
   gatgaagttc ctttggatga g 21
<210> 500
   <211> 21
   <212> DNA
   <213> Artificial Sequence
<220>
   <223> Targeting sequence
<400> 500
   tacacatgga gcctaagaaa g 21
<210> 501
   <211> 21
   <212> DNA
   <213> Artificial Sequence
<220>
   <223> Targeting sequence
<400> 501
   gagctccggc tttcaggaag a 21
<210> 502
   <211> 21
   <212> DNA
   <213> Artificial Sequence
<220>
   <223> Targeting sequence
<400> 502
   tctacagcac caagagcgac g 21
<210> 503
   <211> 21
   <212> DNA
   <213> Artificial Sequence
<220>
   <223> Targeting sequence
<400> 503
   tctgatgatg tcagatatgt a 21
<210> 504
   <211> 21
   <212> DNA
   <213> Artificial Sequence
<220>
   <223> Targeting sequence
<400> 504
   gcctcgctca agattgactt g 21
<210> 505
   <211> 21
   <212> DNA
   <213> Artificial Sequence
<220>
   <223> Targeting sequence
<400> 505
   aaaaagacat acttacaatt a 21
<210> 506
   <211> 21
   <212> DNA
   <213> Artificial Sequence
<220>
   <223> Targeting sequence
<400> 506
   aaaagacata cttacaatta a 21
<210> 507
   <211> 21
   <212> DNA
   <213> Artificial Sequence
<220>
   <223> Targeting sequence
<400> 507
   aaagacatac ttacaattaa g 21
<210> 508
   <211> 21
   <212> DNA
   <213> Artificial Sequence
<220>
   <223> Targeting sequence
<400> 508
   aagacatact tacaattaag g 21
<210> 509
   <211> 21
   <212> DNA
   <213> Artificial Sequence
<220>
   <223> Targeting sequence
<400> 509
   aattaaggct aatacaactc t 21
<210> 510
   <211> 21
   <212> DNA
   <213> Artificial Sequence
<220>
   <223> Targeting sequence
<400> 510
   aaggctaata caactcttca a 21
<210> 511
   <211> 21
   <212> DNA
   <213> Artificial Sequence
<220>
   <223> Targeting sequence
<400> 511
   aatacaactc ttcaaattac t 21
<210> 512
   <211> 21
   <212> DNA
   <213> Artificial Sequence
<220>
   <223> Targeting sequence
<400> 512
   aactcttcaa attacttgca g 21
<210> 513
   <211> 21
   <212> DNA
   <213> Artificial Sequence
<220>
   <223> Targeting sequence
<400> 513
   aaattacttg caggggacag a 21
<210> 514
   <211> 21
   <212> DNA
   <213> Artificial Sequence
<220>
   <223> Targeting sequence
<400> 514
   aattacttgc aggggacaga g 21
<210> 515
   <211> 21
   <212> DNA
   <213> Artificial Sequence
<220>
   <223> Targeting sequence
<400> 515
   aataatcaga gtggcagtga g 21
<210> 516
   <211> 21
   <212> DNA
   <213> Artificial Sequence
<220>
   <223> Targeting sequence
<400> 516
   aatcagagtg gcagtgagca a 21
<210> 517
   <211> 21
   <212> DNA
   <213> Artificial Sequence
<220>
   <223> Targeting sequence
<400> 517
   aaagggtgga ggtgactgag t 21
<210> 518
   <211> 21
   <212> DNA
   <213> Artificial Sequence
<220>
   <223> Targeting sequence
<400> 518
   aagggtggag gtgactgagt g 21
<210> 519
   <211> 21
   <212> DNA
   <213> Artificial Sequence
<220>
   <223> Targeting sequence
<400> 519
   aagacactca caattccaaa a 21
<210> 520
   <211> 21
   <212> DNA
   <213> Artificial Sequence
<220>
   <223> Targeting sequence
<400> 520
   aattccaaaa gtgatcggaa a 21
<210> 521
   <211> 21
   <212> DNA
   <213> Artificial Sequence
<220>
   <223> Targeting sequence
<400> 521
   aaaagtgatc ggaaatgaca c 21
<210> 522
   <211> 21
   <212> DNA
   <213> Artificial Sequence
<220>
   <223> Targeting sequence
<400> 522
   aaagtgatcg gaaatgacac t 21
<210> 523
   <211> 21
   <212> DNA
   <213> Artificial Sequence
<220>
   <223> Targeting sequence
<400> 523
   aagtgatcgg aaatgacact g 21
<210> 524
   <211> 21
   <212> DNA
   <213> Artificial Sequence
<220>
   <223> Targeting sequence
<400> 524
   aaatgacact ggagcctaca a 21
<210> 525
   <211> 21
   <212> DNA
   <213> Artificial Sequence
<220>
   <223> Targeting sequence
<400> 525
   aatgacactg gagcctacaa g 21
<210> 526
   <211> 21
   <212> DNA
   <213> Artificial Sequence
<220>
   <223> Targeting sequence
<400> 526
   aagtgcttct accgggaaac t 21
<210> 527
   <211> 21
   <212> DNA
   <213> Artificial Sequence
<220>
   <223> Targeting sequence
<400> 527
   aaactgactt ggcctcggtc a 21
<210> 528
   <211> 21
   <212> DNA
   <213> Artificial Sequence
<220>
   <223> Targeting sequence
<400> 528
   aactgacttg gcctcggtca t 21
<210> 529
   <211> 21
   <212> DNA
   <213> Artificial Sequence
<220>
   <223> Targeting sequence
   <400> 529
   aagattacag atctccattt a 21
<210> 530
   <211> 21
   <212> DNA
   <213> Artificial Sequence
<220>
   <223> Targeting sequence
<400> 530
   aacatggagt cgtgtacatt a 21
<210> 531
   <211> 21
   <212> DNA
   <213> Artificial Sequence
<220>
   <223> Targeting sequence
<400> 531
   aacaaaaaca aaactgtggt g 21
<210> 532
   <211> 21
   <212> DNA
   <213> Artificial Sequence
<220>
   <223> Targeting sequence
<400> 532
   aaaaacaaaa ctgtggtgat t 21
<210> 533
   <211> 21
   <212> DNA
   <213> Artificial Sequence
<220>
   <223> Targeting sequence
<400> 533
   aaaacaaaac tgtggtgatt c 21
<210> 534
   <211> 21
   <212> DNA
   <213> Artificial Sequence
<220>
   <223> Targeting sequence
<400> 534
   aaacaaaact gtggtgattc c 21
<210> 535
   <211> 21
   <212> DNA
   <213> Artificial Sequence
<220>
   <223> Targeting sequence
<400> 535
   aacaaaactg tggtgattcc a 21
<210> 536
   <211> 21
   <212> DNA
   <213> Artificial Sequence
<220>
   <223> Targeting sequence
<400> 536
   aaaactgtgg tgattccatg t 21
<210> 537
   <211> 21
   <212> DNA
   <213> Artificial Sequence
<220>
   <223> Targeting sequence
<400> 537
   aaactgtggt gattccatgt c 21
<210> 538
   <211> 21
   <212> DNA
   <213> Artificial Sequence
<220>
   <223> Targeting sequence
<400> 538
   aactgtggtg attccatgtc t 21
<210> 539
   <211> 21
   <212> DNA
   <213> Artificial Sequence
<220>
   <223> Targeting sequence
<400> 539
   aaatctcaac gtgtcacttt g 21
<210> 540
   <211> 21
   <212> DNA
   <213> Artificial Sequence
<220>
   <223> Targeting sequence
   <400> 540
   aatctcaacg tgtcactttg t 21
<210> 541
   <211> 21
   <212> DNA
   <213> Artificial Sequence
<220>
   <223> Targeting sequence
<400> 541
   aacgtgtcac tttgtgcaag a 21
<210> 542
   <211> 21
   <212> DNA
   <213> Artificial Sequence
<220>
   <223> Targeting sequence
<400> 542
   aagataccca gaaaagagat t 21
<210> 543
   <211> 21
   <212> DNA
   <213> Artificial Sequence
<220>
   <223> Targeting sequence
<400> 543
   aaaagagatt tgttcctgat g 21
<210> 544
   <211> 21
   <212> DNA
   <213> Artificial Sequence
<220>
   <223> Targeting sequence
<400> 544
   aaagagattt gttcctgatg g 21
<210> 545
   <211> 21
   <212> DNA
   <213> Artificial Sequence
<220>
   <223> Targeting sequence
<400> 545
   aagagatttg ttcctgatgg t 21
<210> 546
   <211> 21
   <212> DNA
   <213> Artificial Sequence
<220>
   <223> Targeting sequence
<400> 546
   aacagaattt cctgggacag c 21
<210> 547
   <211> 21
   <212> DNA
   <213> Artificial Sequence
<220>
   <223> Targeting sequence
<400> 547
   aatttcctgg gacagcaaga a 21
<210> 548
   <211> 21
   <212> DNA
   <213> Artificial Sequence
<220>
   <223> Targeting sequence
<400> 548
   aagaagggct ttactattcc c 21
<210> 549
   <211> 21
   <212> DNA
   <213> Artificial Sequence
<220>
   <223> Targeting sequence
<400> 549
   aagggcttta ctattcccag c 21
<210> 550
   <211> 21
   <212> DNA
   <213> Artificial Sequence
<220>
   <223> Targeting sequence
<400> 550
   aagcaaaaat taatgatgaa a 21
<210> 551
   <211> 21
   <212> DNA
   <213> Artificial Sequence
<220>
   <223> Targeting sequence
<400> 551
   aaaaattaat gatgaaagtt a 21
<210> 552
   <211> 21
   <212> DNA
   <213> Artificial Sequence
<220>
   <223> Targeting sequence
<400> 552
   aaaattaatg atgaaagtta c 21
<210> 553
   <211> 21
   <212> DNA
   <213> Artificial Sequence
<220>
   <223> Targeting sequence
<400> 553
   aaattaatga tgaaagttac c 21
<210> 554
   <211> 21
   <212> DNA
   <213> Artificial Sequence
<220>
   <223> Targeting sequence
<400> 554
   aattaatgat gaaagttacc a 21
<210> 555
   <211> 21
   <212> DNA
   <213> Artificial Sequence
<220>
   <223> Targeting sequence
<400> 555
   aatgatgaaa gttaccagtc t 21
<210> 556
   <211> 21
   <212> DNA
   <213> Artificial Sequence
<220>
   <223> Targeting sequence
<400> 556
   aaagttacca gtctattatg t 21
<210> 557
   <211> 21
   <212> DNA
   <213> Artificial Sequence
<220>
   <223> Targeting sequence
<400> 557
   aagttaccag tctattatgt a 21
<210> 558
   <211> 21
   <212> DNA
   <213> Artificial Sequence
<220>
   <223> Targeting sequence
<400> 558
   aattgaacta tctgttggag a 21
<210> 559
   <211> 21
   <212> DNA
   <213> Artificial Sequence
<220>
   <223> Targeting sequence
<400> 559
   aactatctgt tggagaaaag c 21
<210> 560
   <211> 21
   <212> DNA
   <213> Artificial Sequence
<220>
   <223> Targeting sequence
<400> 560
   aaaagcttgt cttaaattgt a 21
<210> 561
   <211> 21
   <212> DNA
   <213> Artificial Sequence
<220>
   <223> Targeting sequence
<400> 561
   aaagcttgtc ttaaattgta c 21
<210> 562
   <211> 21
   <212> DNA
   <213> Artificial Sequence
<220>
   <223> Targeting sequence
<400> 562
   aagcttgtct taaattgtac a 21
<210> 563
   <211> 21
   <212> DNA
   <213> Artificial Sequence
<220>
   <223> Targeting sequence

<400> 563
   aaattgtaca gcaagaactg a 21
<210> 564
   <211> 21
   <212> DNA
   <213> Artificial Sequence
<220>
   <223> Targeting sequence
<400> 564
   aattgtacag caagaactga a 21
<210> 565
   <211> 21
   <212> DNA
   <213> Artificial Sequence
<220>
   <223> Targeting sequence
<400> 565
   aagaactgaa ctaaatgtgg g 21
<210> 566
   <211> 21
   <212> DNA
   <213> Artificial Sequence
<220>
   <223> Targeting sequence
<400> 566
   aactgaacta aatgtgggga t 21
<210> 567
   <211> 21
   <212> DNA
   <213> Artificial Sequence
<220>
   <223> Targeting sequence
<400> 567
   aactaaatgt ggggattgac t 21
<210> 568
   <211> 21
   <212> DNA
   <213> Artificial Sequence
<220>
   <223> Targeting sequence
<400> 568
   aaatgtgggg attgacttca a 21
<210> 569
   <211> 21
   <212> DNA
   <213> Artificial Sequence
<220>
   <223> Targeting sequence
<400> 569
   aatgtgggga ttgacttcaa c 21
<210> 570
   <211> 21
   <212> DNA
   <213> Artificial Sequence
<220>
   <223> Targeting sequence
<400> 570
   aactgggaat acccttcttc g 21
<210> 571
   <211> 21
   <212> DNA
   <213> Artificial Sequence
<220>
   <223> Targeting sequence
<400> 571
   aatacccttc ttcgaagcat c 21
<210> 572
   <211> 21
   <212> DNA
   <213> Artificial Sequence
<220>
   <223> Targeting sequence
<400> 572
   aagcatcagc ataagaaact t 21
<210> 573
   <211> 21
   <212> DNA
   <213> Artificial Sequence
<220>
   <223> Targeting sequence
<400> 573
   aagaaacttg taaaccgaga c 21
<210> 574
   <211> 21
   <212> DNA
   <213> Artificial Sequence
<220>
   <223> Targeting sequence
<400> 574
   aaacttgtaa accgagacct a 21
<210> 575
   <211> 21
   <212> DNA
   <213> Artificial Sequence
<220>
   <223> Targeting sequence
<400> 575
   aacttgtaaa ccgagaccta a 21
<210> 576
   <211> 21
   <212> DNA
   <213> Artificial Sequence
<220>
   <223> Targeting sequence
<400> 576
   aaaccgagac ctaaaaaccc a 21
<210> 577
   <211> 21
   <212> DNA
   <213> Artificial Sequence
<220>
   <223> Targeting sequence
<400> 577
   aaccgagacc taaaaaccca g 21
<210> 578
   <211> 21
   <212> DNA
   <213> Artificial Sequence
<220>
   <223> Targeting sequence
<400> 578
   aaaaacccag tctgggagtg a 21
<210> 579
   <211> 21
   <212> DNA
   <213> Artificial Sequence
<220>
   <223> Targeting sequence
<400> 579
   aaaacccagt ctgggagtga g 21
<210> 580
   <211> 21
   <212> DNA
   <213> Artificial Sequence
<220>
   <223> Targeting sequence
<400> 580
   aaacccagtc tgggagtgag a 21
<210> 581
   <211> 21
   <212> DNA
   <213> Artificial Sequence
<220>
   <223> Targeting sequence
<400> 581
   aacccagtct gggagtgaga t 21
<210> 582
   <211> 21
   <212> DNA
   <213> Artificial Sequence
<220>
   <223> Targeting sequence
<400> 582
   aagaaatttt tgagcacctt a 21
<210> 583
   <211> 21
   <212> DNA
   <213> Artificial Sequence
<220>
   <223> Targeting sequence
<400> 583
   aaatttttga gcaccttaac t 21
<210> 584
   <211> 21
   <212> DNA
   <213> Artificial Sequence
<220>
   <223> Targeting sequence
<400> 584
   aatttttgag caccttaact a 21
<210> 585
   <211> 21
   <212> DNA
   <213> Artificial Sequence
<220>
   <223> Targeting sequence
<400> 585
   aactatagat ggtgtaaccc g 21
<210> 586
   <211> 21
   <212> DNA
   <213> Artificial Sequence
<220>
   <223> Targeting sequence
<400> 586
   aacccggagt gaccaaggat t 21
<210> 587
   <211> 21
   <212> DNA
   <213> Artificial Sequence
<220>
   <223> Targeting sequence
<400> 587
   aaggattgta cacctgtgca g 21
<210> 588
   <211> 21
   <212> DNA
   <213> Artificial Sequence
<220>
   <223> Targeting sequence
<400> 588
   aagaagaaca gcacatttgt c 21
<210> 589
   <211> 21
   <212> DNA
   <213> Artificial Sequence
<220>
   <223> Targeting sequence
<400> 589
   aagaacagca catttgtcag g 21
<210> 590
   <211> 21
   <212> DNA
   <213> Artificial Sequence
<220>
   <223> Targeting sequence
<400> 590
   aacagcacat ttgtcagggt c 21
<210> 591
   <211> 21
   <212> DNA
   <213> Artificial Sequence
<220>
   <223> Targeting sequence

<400> 591
   aaaaaccttt tgttgctttt g 21
<210> 592
   <211> 21
   <212> DNA
   <213> Artificial Sequence
<220>
   <223> Targeting sequence
<400> 592
   aaaacctttt gttgcttttg g 21
<210> 593
   <211> 21
   <212> DNA
   <213> Artificial Sequence
<220>
   <223> Targeting sequence
<400> 593
   aaaccttttg ttgcttttgg a 21
<210> 594
   <211> 21
   <212> DNA
   <213> Artificial Sequence
<220>
   <223> Targeting sequence
<400> 594
   aaccttttgt tgcttttgga a 21
<210> 595
   <211> 21
   <212> DNA
   <213> Artificial Sequence
<220>
   <223> Targeting sequence
<400> 595
   aagtggcatg gaatctctgg t 21
<210> 596
   <211> 21
   <212> DNA
   <213> Artificial Sequence
<220>
   <223> Targeting sequence
<400> 596
   aatctctggt ggaagccacg g 21
<210> 597
   <211> 21
   <212> DNA
   <213> Artificial Sequence
<220>
   <223> Targeting sequence
<400> 597
   aagccacggt gggggagcgt g 21
<210> 598
   <211> 21
   <212> DNA
   <213> Artificial Sequence
<220>
   <223> Targeting sequence
<400> 598
   aatccctgcg aagtaccttg g 21
<210> 599
   <211> 21
   <212> DNA
   <213> Artificial Sequence
<220>
   <223> Targeting sequence
<400> 599
   aagtaccttg gttacccacc c 21
<210> 600
   <211> 21
   <212> DNA
   <213> Artificial Sequence
<220>
   <223> Targeting sequence
<400> 600
   aaataaaatg gtataaaaat g 21
<210> 601
   <211> 21
   <212> DNA
   <213> Artificial Sequence
<220>
   <223> Targeting sequence
<400> 601
   aataaaatgg tataaaaatg g 21
<210> 602
   <211> 21
   <212> DNA
   <213> Artificial Sequence
<220>
   <223> Targeting sequence
<400> 602
   aaaatggtat aaaaatggaa t 21
<210> 603
   <211> 21
   <212> DNA
   <213> Artificial Sequence
<220>
   <223> Targeting sequence
<400> 603
   aaatggtata aaaatggaat a 21
<210> 604
   <211> 21
   <212> DNA
   <213> Artificial Sequence
<220>
   <223> Targeting sequence
<400> 604
   aatggtataa aaatggaata c 21
<210> 605
   <211> 21
   <212> DNA
   <213> Artificial Sequence
<220>
   <223> Targeting sequence
<400> 605
   aaaaatggaa taccccttga g 21
<210> 606
   <211> 21
   <212> DNA
   <213> Artificial Sequence
<220>
   <223> Targeting sequence
<400> 606
   aaaatggaat accccttgag t 21
<210> 607
   <211> 21
   <212> DNA
   <213> Artificial Sequence
<220>
   <223> Targeting sequence
<400> 607
   aaatggaata ccccttgagt c 21
<210> 608
   <211> 21
   <212> DNA
   <213> Artificial Sequence
<220>
   <223> Targeting sequence
<400> 608
   aatggaatac cccttgagtc c 21
<210> 609
   <211> 21
   <212> DNA
   <213> Artificial Sequence
<220>
   <223> Targeting sequence
<400> 609
   aatacccctt gagtccaatc a 21
<210> 610
   <211> 21
   <212> DNA
   <213> Artificial Sequence
<220>
   <223> Targeting sequence
<400> 610
   aatcacacaa ttaaagcggg g 21
<210> 611
   <211> 21
   <212> DNA
   <213> Artificial Sequence
<220>
   <223> Targeting sequence
<400> 611
   aattaaagcg gggcatgtac t 21
<210> 612
   <211> 21
   <212> DNA
   <213> Artificial Sequence
<220>
   <223> Targeting sequence
<400> 612
   aaagcggggc atgtactgac g 21
<210> 613
   <211> 21
   <212> DNA
   <213> Artificial Sequence
<220>
   <223> Targeting sequence
<400> 613
   aagcggggca tgtactgacg a 21
<210> 614
   <211> 21
   <212> DNA
   <213> Artificial Sequence
<220>
   <223> Targeting sequence
<400> 614
   aagtgagtga aagagacaca g 21
<210> 615
   <211> 21
   <212> DNA
   <213> Artificial Sequence
<220>
   <223> Targeting sequence
<400> 615
   aaagagacac aggaaattac a 21
<210> 616
   <211> 21
   <212> DNA
   <213> Artificial Sequence
<220>
   <223> Targeting sequence
<400> 616
   aagagacaca ggaaattaca c 21
<210> 617
   <211> 21
   <212> DNA
   <213> Artificial Sequence
<220>
   <223> Targeting sequence
<400> 617
   aaattacact gtcatcctta c 21
<210> 618
   <211> 21
   <212> DNA
   <213> Artificial Sequence
<220>
   <223> Targeting sequence
<400> 618
   aattacactg tcatccttac c 21
<210> 619
   <211> 21
   <212> DNA
   <213> Artificial Sequence
<220>
   <223> Targeting sequence
<400> 619
   aatcccattt caaaggagaa g 21
<210> 620
   <211> 21
   <212> DNA
   <213> Artificial Sequence
<220>
   <223> Targeting sequence
<400> 620
   aaaggagaag cagagccatg t 21
<210> 621
   <211> 21
   <212> DNA
   <213> Artificial Sequence
<220>
   <223> Targeting sequence
<400> 621
   aaggagaagc agagccatgt g 21
<210> 622
   <211> 21
   <212> DNA
   <213> Artificial Sequence
<220>
   <223> Targeting sequence
<400> 622
   aagcagagcc atgtggtctc t 21
<210> 623
   <211> 21
   <212> DNA
   <213> Artificial Sequence
<220>
   <223> Targeting sequence
<400> 623
   aaatctctaa tctctcctgt g 21
<210> 624
   <211> 21
   <212> DNA
   <213> Artificial Sequence
<220>
   <223> Targeting sequence

<400> 624
   aatctctaat ctctcctgtg g 21
<210> 625
   <211> 21
   <212> DNA
   <213> Artificial Sequence
<220>
   <223> Targeting sequence
<400> 625
   aatctctcct gtggattcct a 21
<210> 626
   <211> 21
   <212> DNA
   <213> Artificial Sequence
<220>
   <223> Targeting sequence
<400> 626
   aaacgctgac atgtacggtc t 21
<210> 627
   <211> 21
   <212> DNA
   <213> Artificial Sequence
<220>
   <223> Targeting sequence
<400> 627
   aacgctgaca tgtacggtct a 21
<210> 628
   <211> 21
   <212> DNA
   <213> Artificial Sequence
<220>
   <223> Targeting sequence
<400> 628
   aagagtgcgc caacgagccc a 21
<210> 629
   <211> 21
   <212> DNA
   <213> Artificial Sequence
<220>
   <223> Targeting sequence
<400> 629
   aacgagccca gccaagctgt c 21
<210> 630
   <211> 21
   <212> DNA
   <213> Artificial Sequence
<220>
   <223> Targeting sequence
<400> 630
   aagctgtctc agtgacaaac c 21
<210> 631
   <211> 21
   <212> DNA
   <213> Artificial Sequence
<220>
   <223> Targeting sequence
<400> 631
   aaacccatac ccttgtgaag a 21
<210> 632
   <211> 21
   <212> DNA
   <213> Artificial Sequence
<220>
   <223> Targeting sequence
<400> 632
   aacccatacc cttgtgaaga a 21
<210> 633
   <211> 21
   <212> DNA
   <213> Artificial Sequence
<220>
   <223> Targeting sequence
<400> 633
   aagaatggag aagtgtggag g 21
<210> 634
   <211> 21
   <212> DNA
   <213> Artificial Sequence
<220>
   <223> Targeting sequence
<400> 634
   aatggagaag tgtggaggac t 21
<210> 635
   <211> 21
   <212> DNA
   <213> Artificial Sequence
<220>
   <223> Targeting sequence
<400> 635
   aagtgtggag gacttccagg g 21
<210> 636
   <211> 21
   <212> DNA
   <213> Artificial Sequence
<220>
   <223> Targeting sequence
<400> 636
   aaataaaatt gaagttaata a 21
<210> 637
   <211> 21
   <212> DNA
   <213> Artificial Sequence
<220>
   <223> Targeting sequence
<400> 637
   aataaaattg aagttaataa a 21
<210> 638
   <211> 21
   <212> DNA
   <213> Artificial Sequence
<220>
   <223> Targeting sequence
<400> 638
   aaaattgaag ttaataaaaa t 21
<210> 639
   <211> 21
   <212> DNA
   <213> Artificial Sequence
<220>
   <223> Targeting sequence
<400> 639
   aaattgaagt taataaaaat c 21
<210> 640
   <211> 21
   <212> DNA
   <213> Artificial Sequence
<220>
   <223> Targeting sequence
<400> 640
   aattgaagtt aataaaaatc a 21
<210> 641
   <211> 21
   <212> DNA
   <213> Artificial Sequence
<220>
   <223> Targeting sequence
<400> 641
   aagttaataa aaatcaattt g 21
<210> 642
   <211> 21
   <212> DNA
   <213> Artificial Sequence
<220>
   <223> Targeting sequence
<400> 642
   aataaaaatc aatttgctct a 21
<210> 643
   <211> 21
   <212> DNA
   <213> Artificial Sequence
<220>
   <223> Targeting sequence
<400> 643
   aaaaatcaat ttgctctaat t 21
<210> 644
   <211> 21
   <212> DNA
   <213> Artificial Sequence
<220>
   <223> Targeting sequence
<400> 644
   aaaatcaatt tgctctaatt g 21
<210> 645
   <211> 21
   <212> DNA
   <213> Artificial Sequence
<220>
   <223> Targeting sequence
<400> 645
   aaatcaattt gctctaattg a 21
<210> 646
   <211> 21
   <212> DNA
   <213> Artificial Sequence
<220>
   <223> Targeting sequence
<400> 646
   aatcaatttg ctctaattga a 21
<210> 647
   <211> 21
   <212> DNA
   <213> Artificial Sequence
<220>
   <223> Targeting sequence
<400> 647
   aatttgctct aattgaagga a 21
<210> 648
   <211> 21
   <212> DNA
   <213> Artificial Sequence
<220>
   <223> Targeting sequence
<400> 648
   aattgaagga aaaaacaaaa c 21
<210> 649
   <211> 21
   <212> DNA
   <213> Artificial Sequence
<220>
   <223> Targeting sequence
<400> 649
   aaggaaaaaa caaaactgta a 21
<210> 650
   <211> 21
   <212> DNA
   <213> Artificial Sequence
<220>
   <223> Targeting sequence
<400> 650
   aaaaaacaaa actgtaagta c 21
<210> 651
   <211> 21
   <212> DNA
   <213> Artificial Sequence
<220>
   <223> Targeting sequence
<400> 651
   aaaaacaaaa ctgtaagtac c 21
<210> 652
   <211> 21
   <212> DNA
   <213> Artificial Sequence

<220>
   <223> Targeting sequence
<400> 652
   aaaacaaaac tgtaagtacc c 21
<210> 653
   <211> 21
   <212> DNA
   <213> Artificial Sequence
<220>
   <223> Targeting sequence
<400> 653
   aaacaaaact gtaagtaccc t 21
<210> 654
   <211> 21
   <212> DNA
   <213> Artificial Sequence
<220>
   <223> Targeting sequence
<400> 654
   aacaaaactg taagtaccct t 21
<210> 655
   <211> 21
   <212> DNA
   <213> Artificial Sequence
<220>
   <223> Targeting sequence
<400> 655
   aaaactgtaa gtacccttgt t 21
<210> 656
   <211> 21
   <212> DNA
   <213> Artificial Sequence
<220>
   <223> Targeting sequence
<400> 656
   aaactgtaag tacccttgtt a 21
<210> 657
   <211> 21
   <212> DNA
   <213> Artificial Sequence
<220>
   <223> Targeting sequence
<400> 657
   aactgtaagt acccttgtta t 21
<210> 658
   <211> 21
   <212> DNA
   <213> Artificial Sequence
<220>
   <223> Targeting sequence
<400> 658
   aagtaccctt gttatccaag c 21
<210> 659
   <211> 21
   <212> DNA
   <213> Artificial Sequence
<220>
   <223> Targeting sequence
<400> 659
   aagcggcaaa tgtgtcagct t 21
<210> 660
   <211> 21
   <212> DNA
   <213> Artificial Sequence
<220>
   <223> Targeting sequence
<400> 660
   aaatgtgtca gctttgtaca a 21
<210> 661
   <211> 21
   <212> DNA
   <213> Artificial Sequence
<220>
   <223> Targeting sequence
<400> 661
   aatgtgtcag ctttgtacaa a 21
<210> 662
   <211> 21
   <212> DNA
   <213> Artificial Sequence
<220>
   <223> Targeting sequence
<400> 662
   aaatgtgaag cggtcaacaa a 21
<210> 663
   <211> 21
   <212> DNA
   <213> Artificial Sequence
<220>
   <223> Targeting sequence
<400> 663
   aatgtgaagc ggtcaacaaa g 21
<210> 664
   <211> 21
   <212> DNA
   <213> Artificial Sequence
<220>
   <223> Targeting sequence
<400> 664
   aagcggtcaa caaagtcggg a 21
<210> 665
   <211> 21
   <212> DNA
   <213> Artificial Sequence
<220>
   <223> Targeting sequence
<400> 665
   aacaaagtcg ggagaggaga g 21
<210> 666
   <211> 21
   <212> DNA
   <213> Artificial Sequence
<220>
   <223> Targeting sequence
<400> 666
   aaagtcggga gaggagagag g 21
<210> 667
   <211> 21
   <212> DNA
   <213> Artificial Sequence
<220>
   <223> Targeting sequence
<400> 667
   aagtcgggag aggagagagg g 21
<210> 668
   <211> 21
   <212> DNA
   <213> Artificial Sequence
<220>
   <223> Targeting sequence
<400> 668
   aaattacttt gcaacctgac a 21
<210> 669
   <211> 21
   <212> DNA
   <213> Artificial Sequence
<220>
   <223> Targeting sequence
<400> 669
   aattactttg caacctgaca t 21
<210> 670
   <211> 21
   <212> DNA
   <213> Artificial Sequence
<220>
   <223> Targeting sequence
<400> 670
   aacctgacat gcagcccact g 21
<210> 671
   <211> 21
   <212> DNA
   <213> Artificial Sequence
<220>
   <223> Targeting sequence
<400> 671
   aacctcacat ggtacaagct t 21
<210> 672
   <211> 21
   <212> DNA
   <213> Artificial Sequence
<220>
   <223> Targeting sequence
<400> 672
   aagcttggcc cacagcctct g 21
<210> 673
   <211> 21
   <212> DNA
   <213> Artificial Sequence
<220>
   <223> Targeting sequence
<400> 673
   aatccatgtg ggagagttgc c 21
<210> 674
   <211> 21
   <212> DNA
   <213> Artificial Sequence
<220>
   <223> Targeting sequence
<400> 674
   aagaacttgg atactctttg g 21
<210> 675
   <211> 21
   <212> DNA
   <213> Artificial Sequence
<220>
   <223> Targeting sequence
<400> 675
   aacttggata ctctttggaa a 21
<210> 676
   <211> 21
   <212> DNA
   <213> Artificial Sequence
<220>
   <223> Targeting sequence
<400> 676
   aaattgaatg ccaccatgtt c 21
<210> 677
   <211> 21
   <212> DNA
   <213> Artificial Sequence
<220>
   <223> Targeting sequence
<400> 677
   aattgaatgc caccatgttc t 21
<210> 678
   <211> 21
   <212> DNA
   <213> Artificial Sequence
<220>
   <223> Targeting sequence
<400> 678
   aatgccacca tgttctctaa t 21
<210> 679
   <211> 21
   <212> DNA
   <213> Artificial Sequence
<220>
   <223> Targeting sequence
<400> 679
   aatagcacaa atgacatttt g 21
<210> 680
   <211> 21
   <212> DNA
   <213> Artificial Sequence
<220>
   <223> Targeting sequence
<400> 680
   aaatgacatt ttgatcatgg a 21
<210> 681
   <211> 21
   <212> DNA
   <213> Artificial Sequence
<220>
   <223> Targeting sequence
<400> 681
   aatgacattt tgatcatgga g 21
<210> 682
   <211> 21
   <212> DNA
   <213> Artificial Sequence
<220>
   <223> Targeting sequence
<400> 682
   aagaatgcat ccttgcagga c 21
<210> 683
   <211> 21
   <212> DNA
   <213> Artificial Sequence
<220>
   <223> Targeting sequence
<400> 683
   aatgcatcct tgcaggacca a 21
<210> 684
   <211> 21
   <212> DNA
   <213> Artificial Sequence
<220>
   <223> Targeting sequence
<400> 684
   aaggagacta tgtctgcctt g 21
<210> 685
   <211> 21
   <212> DNA
   <213> Artificial Sequence
<220>
   <223> Targeting sequence
<400> 685
   aagacaggaa gaccaagaaa a 21
<210> 686
   <211> 21
   <212> DNA
   <213> Artificial Sequence
<220>
   <223> Targeting sequence
<400> 686
   aagaccaaga aaagacattg c 21
<210> 687
   <211> 21
   <212> DNA
   <213> Artificial Sequence
<220>
   <223> Targeting sequence
<400> 687
   aagaaaagac attgcgtggt c 21
<210> 688
   <211> 21
   <212> DNA
   <213> Artificial Sequence
<220>
   <223> Targeting sequence
<400> 688
   aaaagacatt gcgtggtcag g 21
<210> 689
   <211> 21
   <212> DNA
   <213> Artificial Sequence
<220>
   <223> Targeting sequence
<400> 689
   aaagacattg cgtggtcagg c 21
<210> 690
   <211> 21
   <212> DNA
   <213> Artificial Sequence
<220>
   <223> Targeting sequence
<400> 690
   aagacattgc gtggtcaggc a 21
<210> 691
   <211> 21
   <212> DNA
   <213> Artificial Sequence
<220>
   <223> Targeting sequence
<400> 691 21
   aaacctggag aatcagacga c 21
<210> 692
   <211> 21
   <212> DNA
   <213> Artificial Sequence
<220>
   <223> Targeting sequence
<400> 692
   aacctggaga atcagacgac a 21
<210> 693
   <211> 21
   <212> DNA
   <213> Artificial Sequence
<220>
   <223> Targeting sequence
<400> 693
   aatcagacga caagtattgg g 21
<210> 694
   <211> 21
   <212> DNA
   <213> Artificial Sequence
<220>
   <223> Targeting sequence
<400> 694
   aagtattggg gaaagcatcg a 21
<210> 695
   <211> 21
   <212> DNA
   <213> Artificial Sequence
<220>
   <223> Targeting sequence
<400> 695
   aaagcatcga agtctcatgc a 21
<210> 696
   <211> 21
   <212> DNA
   <213> Artificial Sequence
<220>
   <223> Targeting sequence
<400> 696
   aagcatcgaa gtctcatgca c 21
<210> 697
   <211> 21
   <212> DNA
   <213> Artificial Sequence
<220>
   <223> Targeting sequence
<400> 697
   aatccccctc cacagatcat g 21
<210> 698
   <211> 21
   <212> DNA
   <213> Artificial Sequence
<220>
   <223> Targeting sequence
<400> 698
   aaagataatg agacccttgt a 21
<210> 699
   <211> 21
   <212> DNA
   <213> Artificial Sequence
<220>
   <223> Targeting sequence
<400> 699
   aagataatga gacccttgta g 21
<210> 700
   <211> 21
   <212> DNA
   <213> Artificial Sequence
<220>
   <223> Targeting sequence
<400> 700
   aatgagaccc ttgtagaaga c 21
<210> 701
   <211> 21
   <212> DNA
   <213> Artificial Sequence
<220>
   <223> Targeting sequence
<400> 701
   aagactcagg cattgtattg a 21
<210> 702
   <211> 21
   <212> DNA
   <213> Artificial Sequence
<220>
   <223> Targeting sequence
<400> 702
   aaggatggga accggaacct c 21
<210> 703
   <211> 21
   <212> DNA
   <213> Artificial Sequence
<220>
   <223> Targeting sequence
<400> 703
   aaccggaacc tcactatccg c 21

<210> 704
   <211> 21
   <212> DNA
   <213> Artificial Sequence
<220>
   <223> Targeting sequence
<400> 704
   aacctcacta tccgcagagt g 21
<210> 705
   <211> 21
   <212> DNA
   <213> Artificial Sequence
<220>
   <223> Targeting sequence
<400> 705
   aaggaggacg aaggcctcta c 21
<210> 706
   <211> 21
   <212> DNA
   <213> Artificial Sequence
<220>
   <223> Targeting sequence
<400> 706
   aaggcctcta cacctgccag g 21
<210> 707
   <211> 21
   <212> DNA
   <213> Artificial Sequence
<220>
   <223> Targeting sequence
<400> 707
   aaaagtggag gcatttttca t 21
<210> 708
   <211> 21
   <212> DNA
   <213> Artificial Sequence
<220>
   <223> Targeting sequence
<400> 708
   aaagtggagg catttttcat a 21
<210> 709
   <211> 21
   <212> DNA
   <213> Artificial Sequence
<220>
   <223> Targeting sequence
<400> 709
   aagtggaggc atttttcata a 21
<210> 710
   <211> 21
   <212> DNA
   <213> Artificial Sequence
<220>
   <223> Targeting sequence
<400> 710
   aatagaaggt gcccaggaaa a 21
<210> 711
   <211> 21
   <212> DNA
   <213> Artificial Sequence
<220>
   <223> Targeting sequence
<400> 711
   aaggtgccca ggaaaagacg a 21
<210> 712
   <211> 21
   <212> DNA
   <213> Artificial Sequence
<220>
   <223> Targeting sequence
<400> 712
   aaaagacgaa cttggaaatc a 21
<210> 713
   <211> 21
   <212> DNA
   <213> Artificial Sequence
<220>
   <223> Targeting sequence
<400> 713
   aaagacgaac ttggaaatca t 21
<210> 714
   <211> 21
   <212> DNA
   <213> Artificial Sequence
<220>
   <223> Targeting sequence
<400> 714
   aagacgaact tggaaatcat t 21
<210> 715
   <211> 21
   <212> DNA
   <213> Artificial Sequence
<220>
   <223> Targeting sequence
<400> 715
   aacttggaaa tcattattct a 21
<210> 716
   <211> 21
   <212> DNA
   <213> Artificial Sequence
<220>
   <223> Targeting sequence
<400> 716
   aaatcattat tctagtaggc a 21
<210> 717
   <211> 21
   <212> DNA
   <213> Artificial Sequence
<220>
   <223> Targeting sequence
<400> 717
   aatcattatt ctagtaggca c 21
<210> 718
   <211> 21
   <212> DNA
   <213> Artificial Sequence
<220>
   <223> Targeting sequence
<400> 718
   aagcgggcca atggagggga a 21
<210> 719
   <211> 21
   <212> DNA
   <213> Artificial Sequence
<220>
   <223> Targeting sequence
<400> 719
   aatggagggg aactgaagac a 21
<210> 720
   <211> 21
   <212> DNA
   <213> Artificial Sequence
<220>
   <223> Targeting sequence
<400> 720
   aactgaagac aggctacttg t 21
<210> 721
   <211> 21
   <212> DNA
   <213> Artificial Sequence
<220>
   <223> Targeting sequence
<400> 721
   aagacaggct acttgtccat c 21
<210> 722
   <211> 21
   <212> DNA
   <213> Artificial Sequence
<220>
   <223> Targeting sequence
<400> 722
   aactcccatt ggatgaacat t 21
<210> 723
   <211> 21
   <212> DNA
   <213> Artificial Sequence
<220>
   <223> Targeting sequence
<400> 723
   aacattgtga acgactgcct t 21
<210> 724
   <211> 21
   <212> DNA
   <213> Artificial Sequence
<220>
   <223> Targeting sequence
<400> 724
   aacgactgcc ttatgatgcc a 21
<210> 725
   <211> 21
   <212> DNA
   <213> Artificial Sequence
<220>
   <223> Targeting sequence
<400> 725
   aaatgggaat tccccagaga c 21
<210> 726
   <211> 21
   <212> DNA
   <213> Artificial Sequence
<220>
   <223> Targeting sequence
<400> 726
   aatgggaatt ccccagagac c 21
<210> 727
   <211> 21
   <212> DNA
   <213> Artificial Sequence
<220>
   <223> Targeting sequence
<400> 727
   aattccccag agaccggctg a 21
<210> 728
   <211> 21
   <212> DNA
   <213> Artificial Sequence
<220>
   <223> Targeting sequence
<400> 728
   aagctaggta agcctcttgg c 21
<210> 729
   <211> 21
   <212> DNA
   <213> Artificial Sequence
<220>
   <223> Targeting sequence
<400> 729
   aagcctcttg gccgtggtgc c 21
<210> 730
   <211> 21
   <212> DNA
   <213> Artificial Sequence
<220>
   <223> Targeting sequence
<400> 730
   aagtgattga agcagatgcc t 21
<210> 731
   <211> 21
   <212> DNA
   <213> Artificial Sequence
<220>
   <223> Targeting sequence
<400> 731
   aagcagatgc ctttggaatt g 21
<210> 732
   <211> 21
   <212> DNA
   <213> Artificial Sequence
<220>
   <223> Targeting sequence
<400> 732
   aattgacaag acagcaactt g 21
<210> 733
   <211> 21
   <212> DNA
   <213> Artificial Sequence
<220>
   <223> Targeting sequence
<400> 733
   aagacagcaa cttgcaggac a 21
<210> 734
   <211> 21
   <212> DNA
   <213> Artificial Sequence
<220>
   <223> Targeting sequence
<400> 734
   aacttgcagg acagtagcag t 21
<210> 735
   <211> 21
   <212> DNA
   <213> Artificial Sequence
<220>
   <223> Targeting sequence
<400> 735
   aaaatgttga aagaaggagc a 21
<210> 736
   <211> 21
   <212> DNA
   <213> Artificial Sequence
<220>
   <223> Targeting sequence
<400> 736
   aaatgttgaa agaaggagca a 21
<210> 737
   <211> 21
   <212> DNA
   <213> Artificial Sequence
<220>
   <223> Targeting sequence
<400> 737
   aatgttgaaa gaaggagcaa c 21
<210> 738
   <211> 21
   <212> DNA
   <213> Artificial Sequence
<220>
   <223> Targeting sequence
<400> 738
   aaagaaggag caacacacag t 21
<210> 739
   <211> 21
   <212> DNA
   <213> Artificial Sequence
<220>
   <223> Targeting sequence
<400> 739
   aagaaggagc aacacacagt g 21
<210> 740
   <211> 21
   <212> DNA
   <213> Artificial Sequence
<220>
   <223> Targeting sequence
<400> 740
   aaggagcaac acacagtgag c 21
<210> 741
   <211> 21
   <212> DNA
   <213> Artificial Sequence
<220>
   <223> Targeting sequence
<400> 741
   aacacacagt gagcatcgag c 21
<210> 742
   <211> 21
   <212> DNA
   <213> Artificial Sequence
<220>
   <223> Targeting sequence
<400> 742
   aactcaagat cctcattcat a 21
<210> 743
   <211> 21
   <212> DNA
   <213> Artificial Sequence
<220>
   <223> Targeting sequence
<400> 743
   aagatcctca ttcatattgg t 21
<210> 744
   <211> 21
   <212> DNA
   <213> Artificial Sequence
<220>
   <223> Targeting sequence
<400> 744
   aatgtggtca accttctagg t 21
<210> 745
   <211> 21
   <212> DNA
   <213> Artificial Sequence
<220>
   <223> Targeting sequence
<400> 745
   aaccttctag gtgcctgtac c 21
<210> 746
   <211> 21
   <212> DNA
   <213> Artificial Sequence
<220>
   <223> Targeting sequence
<400> 746
   aagccaggag ggccactcat g 21
<210> 747
   <211> 21
   <212> DNA
   <213> Artificial Sequence
<220>
   <223> Targeting sequence
<400> 747
   aattctgcaa atttggaaac c 21
<210> 748
   <211> 21
   <212> DNA
   <213> Artificial Sequence
<220>
   <223> Targeting sequence
<400> 748
   aaatttggaa acctgtccac t 21
<210> 749
   <211> 21
   <212> DNA
   <213> Artificial Sequence
<220>
   <223> Targeting sequence
<400> 749
   aatttggaaa cctgtccact t 21
<210> 750
   <211> 21
   <212> DNA
   <213> Artificial Sequence
<220>
   <223> Targeting sequence
<400> 750
   aaacctgtcc acttacctga g 21
<210> 751
   <211> 21
   <212> DNA
   <213> Artificial Sequence
<220>
   <223> Targeting sequence
<400> 751
   aacctgtcca cttacctgag g 21
<210> 752
   <211> 21
   <212> DNA
   <213> Artificial Sequence
<220>
   <223> Targeting sequence
<400> 752
   aagagaaatg aatttgtccc c 21
<210> 753
   <211> 21
   <212> DNA
   <213> Artificial Sequence
<220>
   <223> Targeting sequence
<400> 753
   aaatgaattt gtcccctaca a 21
<210> 754
   <211> 21
   <212> DNA
   <213> Artificial Sequence
<220>
   <223> Targeting sequence
<400> 754
   aatgaatttg tcccctacaa g 21
<210> 755
   <211> 21
   <212> DNA
   <213> Artificial Sequence
<220>
   <223> Targeting sequence
<400> 755
   aatttgtccc ctacaagacc a 21
<210> 756
   <211> 21
   <212> DNA
   <213> Artificial Sequence
<220>
   <223> Targeting sequence
<400> 756
   aagaccaaag gggcacgatt c 21
<210> 757
   <211> 21
   <212> DNA
   <213> Artificial Sequence
<220>
   <223> Targeting sequence
<400> 757
   aaaggggcac gattccgtca a 21
<210> 758
   <211> 21
   <212> DNA
   <213> Artificial Sequence
<220>
   <223> Targeting sequence
<400> 758
   aaggggcacg attccgtcaa g 21
<210> 759
   <211> 21
   <212> DNA
   <213> Artificial Sequence
<220>
   <223> Targeting sequence
<400> 759
   aagggaaaga ctacgttgga g 21
<210> 760
   <211> 21
   <212> DNA
   <213> Artificial Sequence
<220>
   <223> Targeting sequence
<400> 760
   aaagactacg ttggagcaat c 21
<210> 761
   <211> 21
   <212> DNA
   <213> Artificial Sequence
<220>
   <223> Targeting sequence
<400> 761
   aagactacgt tggagcaatc c 21
<210> 762
   <211> 21
   <212> DNA
   <213> Artificial Sequence
<220>
   <223> Targeting sequence
<400> 762
   aatccctgtg gatctgaaac g 21
<210> 763
   <211> 21
   <212> DNA
   <213> Artificial Sequence
<220>
   <223> Targeting sequence
<400> 763
   aaacggcgct tggacagcat c 21
<210> 764
   <211> 21
   <212> DNA
   <213> Artificial Sequence
<220>
   <223> Targeting sequence
<400> 764
   aacggcgctt ggacagcatc a 21
<210> 765
   <211> 21
   <212> DNA
   <213> Artificial Sequence
<220>
   <223> Targeting sequence
<400> 765
   aagtccctca gtgatgtaga a 21
<210> 766
   <211> 21
   <212> DNA
   <213> Artificial Sequence
<220>
   <223> Targeting sequence
<400> 766
   aagaagagga agctcctgaa g 21
<210> 767
   <211> 21
   <212> DNA
   <213> Artificial Sequence
<220>
   <223> Targeting sequence
<400> 767
   aagaggaagc tcctgaagat c 21
<210> 768
   <211> 21
   <212> DNA
   <213> Artificial Sequence
<220>
   <223> Targeting sequence
<400> 768
   aagctcctga agatctgtat a 21
<210> 769
   <211> 21
   <212> DNA
   <213> Artificial Sequence
<220>
   <223> Targeting sequence
<400> 769
   aagatctgta taaggacttc c 21
<210> 770
   <211> 21
   <212> DNA
   <213> Artificial Sequence
<220>
   <223> Targeting sequence
<400> 770
   aaggacttcc tgaccttgga g 21
<210> 771
   <211> 21
   <212> DNA
   <213> Artificial Sequence
<220>
   <223> Targeting sequence
<400> 771
   aagtggctaa gggcatggag t 21
<210> 772
   <211> 21
   <212> DNA
   <213> Artificial Sequence
<220>
   <223> Targeting sequence
<400> 772
   aagggcatgg agttcttggc a 21
<210> 773
   <211> 21
   <212> DNA
   <213> Artificial Sequence
<220>
   <223> Targeting sequence
<400> 773
   aaagtgtatc cacagggacc t 21
<210> 774
   <211> 21
   <212> DNA
   <213> Artificial Sequence
<220>
   <223> Targeting sequence
<400> 774
   aagtgtatcc acagggacct g 21
<210> 775
   <211> 21
   <212> DNA
   <213> Artificial Sequence
<220>
   <223> Targeting sequence
<400> 775
   aaatatcctc ttatcggaga a 21
<210> 776
   <211> 21
   <212> DNA
   <213> Artificial Sequence
<220>
   <223> Targeting sequence
<400> 776
   aatatcctct tatcggagaa g 21
<210> 777
   <211> 21
   <212> DNA
   <213> Artificial Sequence
<220>
   <223> Targeting sequence
<400> 777
   aatatcctct tatcggagaa g 21
<210> 778
   <211> 21
   <212> DNA
   <213> Artificial Sequence
<220>
   <223> Targeting sequence
<400> 778
   aagaacgtgg ttaaaatctg t 21
<210> 779
   <211> 21
   <212> DNA
   <213> Artificial Sequence
<220>
   <223> Targeting sequence
<400> 779
   aacgtggtta aaatctgtga c 21
<210> 780
   <211> 21
   <212> DNA
   <213> Artificial Sequence
<220>
   <223> Targeting sequence
<400> 780
   aaaatctgtg actttggctt g 21
<210> 781
   <211> 21
   <212> DNA
   <213> Artificial Sequence
<220>
   <223> Targeting sequence
<400> 781
   aaatctgtga ctttggcttg g 21
<210> 782
   <211> 21
   <212> DNA
   <213> Artificial Sequence
<220>
   <223> Targeting sequence
<400> 782
   aatctgtgac tttggcttgg c 21
<210> 783
   <211> 21
   <212> DNA
   <213> Artificial Sequence
<220>
   <223> Targeting sequence
<400> 783
   aaagatccag attatgtcag a 21
<210> 784
   <211> 21
   <212> DNA
   <213> Artificial Sequence
<220>
   <223> Targeting sequence
<400> 784
   aagatccaga ttatgtcaga a 21
<210> 785
   <211> 21
   <212> DNA
   <213> Artificial Sequence
<220>
   <223> Targeting sequence
<400> 785
   aaaaggagat gctcgcctcc c 21
<210> 786
   <211> 21
   <212> DNA
   <213> Artificial Sequence
<220>
   <223> Targeting sequence
<400> 786
   aaaggagatg ctcgcctccc t 21
<210> 787
   <211> 21
   <212> DNA
   <213> Artificial Sequence
<220>
   <223> Targeting sequence
<400> 787
   aaggagatgc tcgcctccct t 21
<210> 788
   <211> 21
   <212> DNA
   <213> Artificial Sequence
<220>
   <223> Targeting sequence
<400> 788
   aaatggatgg ccccagaaac a 21
<210> 789
   <211> 21
   <212> DNA
   <213> Artificial Sequence
<220>
   <223> Targeting sequence
<400> 789
   aatggatggc cccagaaaca a 21
<210> 790
   <211> 21
   <212> DNA
   <213> Artificial Sequence
<220>
   <223> Targeting sequence
<400> 790
   aaacaatttt tgacagagtg t 21
<210> 791
   <211> 21
   <212> DNA
   <213> Artificial Sequence
<220>
   <223> Targeting sequence
<400> 791
   aacaattttt gacagagtgt a 21
<210> 792
   <211> 21
   <212> DNA
   <213> Artificial Sequence
<220>
   <223> Targeting sequence
<400> 792
   aatttttgac agagtgtaca c 21
<210> 793
   <211> 21
   <212> DNA
   <213> Artificial Sequence
<220>
   <223> Targeting sequence
<400> 793
   aatccagagt gacgtctggt c 21
<210> 794
   <211> 21
   <212> DNA
   <213> Artificial Sequence
<220>
   <223> Targeting sequence
<400> 794
   aaatattttc cttaggtgct t 21
<210> 795
   <211> 21
   <212> DNA
   <213> Artificial Sequence
<220>
   <223> Targeting sequence
<400> 795
   aatattttcc ttaggtgctt c 21
<210> 796
   <211> 21
   <212> DNA
   <213> Artificial Sequence
<220>
   <223> Targeting sequence
<400> 796
   aaagattgat gaagaatttt g 21
<210> 797
   <211> 21
   <212> DNA
   <213> Artificial Sequence
<220>
   <223> Targeting sequence
<400> 797
   aagattgatg aagaattttg t 21
<210> 798
   <211> 21
   <212> DNA
   <213> Artificial Sequence
<220>
   <223> Targeting sequence
<400> 798
   aagaattttg taggcgattg a 21
<210> 799
   <211> 21
   <212> DNA
   <213> Artificial Sequence
<220>
   <223> Targeting sequence
<400> 799
   aattttgtag gcgattgaaa g 21
<210> 800
   <211> 21
   <212> DNA
   <213> Artificial Sequence
<220>
   <223> Targeting sequence
<400> 800
   aaagaaggaa ctagaatgag g 21
<210> 801
   <211> 21
   <212> DNA
   <213> Artificial Sequence
<220>
   <223> Targeting sequence
<400> 801
   aagaaggaac tagaatgagg g 21
<210> 802
   <211> 21
   <212> DNA
   <213> Artificial Sequence
<220>
   <223> Targeting sequence
<400> 802
   aaggaactag aatgagggcc c 21
<210> 803
   <211> 21
   <212> DNA
   <213> Artificial Sequence
<220>
   <223> Targeting sequence
<400> 803
   aactagaatg agggcccctg a 21
<210> 804
   <211> 21
   <212> DNA
   <213> Artificial Sequence
<220>
   <223> Targeting sequence
<400> 804
   aatgagggcc cctgattata c 21
<210> 805
   <211> 21
   <212> DNA
   <213> Artificial Sequence
<220>
   <223> Targeting sequence
<400> 805
   aaatgtacca gaccatgctg g 21
<210> 806
   <211> 21
   <212> DNA
   <213> Artificial Sequence
<220>
   <223> Targeting sequence
<400> 806
   aatgtaccag accatgctgg a 21
<210> 807
   <211> 21
   <212> DNA
   <213> Artificial Sequence
<220>
   <223> Targeting sequence
<400> 807
   aacatttggg aaatctcttg c 21
<210> 808
   <211> 21
   <212> DNA
   <213> Artificial Sequence
<220>
   <223> Targeting sequence
<400> 808
   aaatctcttg caagctaatg c 21
<210> 809
   <211> 21
   <212> DNA
   <213> Artificial Sequence
<220>
   <223> Targeting sequence
<400> 809
   aatctcttgc aagctaatgc t 21
<210> 810
   <211> 21
   <212> DNA
   <213> Artificial Sequence
<220>
   <223> Targeting sequence
<400> 810
   aagctaatgc tcagcaggat g 21
<210> 811
   <211> 21
   <212> DNA
   <213> Artificial Sequence
<220>
   <223> Targeting sequence
<400> 811
   aatgctcagc aggatggcaa a 21
<210> 812
   <211> 21
   <212> DNA
   <213> Artificial Sequence
<220>
   <223> Targeting sequence
<400> 812
   aaagactaca ttgttcttcc g 21
<210> 813
   <211> 21
   <212> DNA
   <213> Artificial Sequence
<220>
   <223> Targeting sequence
<400> 813
   aagactacat tgttcttccg a 21
<210> 814
   <211> 21
   <212> DNA
   <213> Artificial Sequence
<220>
   <223> Targeting sequence
<400> 814
   aagaggattc tggactctct c 21
<210> 815
   <211> 21
   <212> DNA
   <213> Artificial Sequence
<220>
   <223> Targeting sequence
<400> 815
   aagtatgtga ccccaaattc c 21
<210> 816
   <211> 21
   <212> DNA
   <213> Artificial Sequence
<220>
   <223> Targeting sequence
<400> 816
   aaattccatt atgacaacac a 21
<210> 817
   <211> 21
   <212> DNA
   <213> Artificial Sequence
<220>
   <223> Targeting sequence
<400> 817
   aattccatta tgacaacaca g 21
<210> 818
   <211> 21
   <212> DNA
   <213> Artificial Sequence
<220>
   <223> Targeting sequence
<400> 818
   aacacagcag gaatcagtca g 21
<210> 819
   <211> 21
   <212> DNA
   <213> Artificial Sequence
<220>
   <223> Targeting sequence
<400> 819
   aatcagtcag tatctgcaga a 21
<210> 820
   <211> 21
   <212> DNA
   <213> Artificial Sequence
<220>
   <223> Targeting sequence
<400> 820
   aacagtaagc gaaagagccg g 21
<210> 821
   <211> 21
   <212> DNA
   <213> Artificial Sequence
<220>
   <223> Targeting sequence
<400> 821
   aagcgaaaga gccggcctgt g 21
<210> 822
   <211> 21
   <212> DNA
   <213> Artificial Sequence
<220>
   <223> Targeting sequence
<400> 822
   aaagagccgg cctgtgagtg t 21
<210> 823
   <211> 21
   <212> DNA
   <213> Artificial Sequence
<220>
   <223> Targeting sequence
<400> 823
   aagagccggc ctgtgagtgt a 21
<210> 824
   <211> 21
   <212> DNA
   <213> Artificial Sequence
<220>
   <223> Targeting sequence
<400> 824
   aaaaacattt gaagatatcc c 21
<210> 825
   <211> 21
   <212> DNA
   <213> Artificial Sequence
<220>
   <223> Targeting sequence
<400> 825
   aaaacatttg aagatatccc g 21
<210> 826
   <211> 21
   <212> DNA
   <213> Artificial Sequence
<220>
   <223> Targeting sequence
<400> 826
   aaacatttga agatatcccg t 21
<210> 827
   <211> 21
   <212> DNA
   <213> Artificial Sequence
<220>
   <223> Targeting sequence
<400> 827
   aacatttgaa gatatcccgt t 21
<210> 828
   <211> 21
   <212> DNA
   <213> Artificial Sequence
<220>
   <223> Targeting sequence
<400> 828
   aagatatccc gttagaagaa c 21
<210> 829
   <211> 21
   <212> DNA
   <213> Artificial Sequence
<220>
   <223> Targeting sequence
<400> 829
   aagaaccaga agtaaaagta a 21
<210> 830
   <211> 21
   <212> DNA
   <213> Artificial Sequence
<220>
   <223> Targeting sequence
<400> 830
   aaccagaagt aaaagtaatc c 21
<210> 831
   <211> 21
   <212> DNA
   <213> Artificial Sequence
<220>
   <223> Targeting sequence
<400> 831
   aagtaaaagt aatcccagat g 21
<210> 832
   <211> 21
   <212> DNA
   <213> Artificial Sequence
<220>
   <223> Targeting sequence
<400> 832
   aaaagtaatc ccagatgaca a 21
<210> 833
   <211> 21
   <212> DNA
   <213> Artificial Sequence
<220>
   <223> Targeting sequence
<400> 833
   aaagtaatcc cagatgacaa c 21
<210> 834
   <211> 21
   <212> DNA
   <213> Artificial Sequence
<220>
   <223> Targeting sequence
<400> 834
   aagtaatccc agatgacaac c 21
<210> 835
   <211> 21
   <212> DNA
   <213> Artificial Sequence
<220>
   <223> Targeting sequence
<400> 835
   aatcccagat gacaaccaga c 21
<210> 836
   <211> 21
   <212> DNA
   <213> Artificial Sequence
<220>
   <223> Targeting sequence
<400> 836
   aaccagacgg acagtggtat g 21
<210> 837
   <211> 21
   <212> DNA
   <213> Artificial Sequence
<220>
   <223> Targeting sequence
<400> 837
   aagagctgaa aactttggaa g 21
<210> 838
   <211> 21
   <212> DNA
   <213> Artificial Sequence
<220>
   <223> Targeting sequence
<400> 838
   aaaactttgg aagacagaac c 21
<210> 839
   <211> 21
   <212> DNA
   <213> Artificial Sequence
<220>
   <223> Targeting sequence
<400> 839
   aaactttgga agacagaacc a 21
<210> 840
   <211> 21
   <212> DNA
   <213> Artificial Sequence
<220>
   <223> Targeting sequence
<400> 840
   aactttggaa gacagaacca a 21
<210> 841
   <211> 21
   <212> DNA
   <213> Artificial Sequence
<220>
   <223> Targeting sequence
<400> 841
   aagacagaac caaattatct c 21
<210> 842
   <211> 21
   <212> DNA
   <213> Artificial Sequence
<220>
   <223> Targeting sequence
<400> 842
   aaccaaatta tctccatctt t 21
<210> 843
   <211> 21
   <212> DNA
   <213> Artificial Sequence
<220>
   <223> Targeting sequence
<400> 843
   aaattatctc catcttttgg t 21
<210> 844
   <211> 21
   <212> DNA
   <213> Artificial Sequence
<220>
   <223> Targeting sequence
<400> 844
   aattatctcc atcttttggt g 21
<210> 845
   <211> 21
   <212> DNA
   <213> Artificial Sequence
<220>
   <223> Targeting sequence
<400> 845
   aatggtgccc agcaaaagca g 21
<210> 846
   <211> 21
   <212> DNA
   <213> Artificial Sequence
<220>
   <223> Targeting sequence
<400> 846
   aaaagcaggg agtctgtggc a 21
<210> 847
   <211> 21
   <212> DNA
   <213> Artificial Sequence
<220>
   <223> Targeting sequence
<400> 847
   aaagcaggga gtctgtggca t 21
<210> 848
   <211> 21
   <212> DNA
   <213> Artificial Sequence
<220>
   <223> Targeting sequence
<400> 848
   aagcagggag tctgtggcat c 21
<210> 849
   <211> 21
   <212> DNA
   <213> Artificial Sequence
<220>
   <223> Targeting sequence
<400> 849
   aaggctcaaa ccagacaagc g 21
<210> 850
   <211> 21
   <212> DNA
   <213> Artificial Sequence
<220>
   <223> Targeting sequence
<400> 850
   aaaccagaca agcggctacc a 21
<210> 851
   <211> 21
   <212> DNA
   <213> Artificial Sequence
<220>
   <223> Targeting sequence
<400> 851
   aaccagacaa gcggctacca g 21
<210> 852
   <211> 21
   <212> DNA
   <213> Artificial Sequence
<220>
   <223> Targeting sequence
<400> 852
   aagcggctac cagtccggat a 21
<210> 853
   <211> 21
   <212> DNA
   <213> Artificial Sequence
<220>
   <223> Targeting sequence
<400> 853
   aagcagaact tttaaagctg a 21
<210> 854
   <211> 21
   <212> DNA
   <213> Artificial Sequence
<220>
   <223> Targeting sequence
<400> 854
   gacatactta caattaaggc t 21
<210> 855
   <211> 21
   <212> DNA
   <213> Artificial Sequence
<220>
   <223> Targeting sequence
<400> 855
   gatttgttcc tgatggtaac a 21
<210> 856
   <211> 21
   <212> DNA
   <213> Artificial Sequence
<220>
   <223> Targeting sequence
<400> 856
   ttttgagcac cttaactata g 21
<210> 857
   <211> 21
   <212> DNA
   <213> Artificial Sequence
<220>
   <223> Targeting sequence
<400> 857
   gagccatgtg gtctctctgg t 21
<210> 858
   <211> 21
   <212> DNA
   <213> Artificial Sequence
<220>
   <223> Targeting sequence
<400> 858
   ggtgatctcc ttccacgtga c 21
<210> 859
   <211> 21
   <212> DNA
   <213> Artificial Sequence
<220>
   <223> Targeting sequence
<400> 859
   gtggtcaggc agctcacagt c 21
<210> 860
   <211> 21
   <212> DNA
   <213> Artificial Sequence
<220>
   <223> Targeting sequence
<400> 860
   cggtgattgc catgttcttc t 21
<210> 861
   <211> 21
   <212> DNA
   <213> Artificial Sequence
<220>
   <223> Targeting sequence
<400> 861
   ttctaggtgc ctgtaccaag c 21
<210> 862
   <211> 21
   <212> DNA
   <213> Artificial Sequence
<220>
   <223> Targeting sequence
<400> 862
   tgtatccaca gggacctggc g 21
<210> 863
   <211> 21
   <212> DNA
   <213> Artificial Sequence
<220>
   <223> Targeting sequence
<400> 863
   taccagacca tgctggactg c 21
<210> 864
   <211> 21
   <212> DNA
   <213> Artificial Sequence
<220>
   <223> Targeting sequence
<400> 864
   ttatctccat cttttggtgg a 21